(19) Europäisches Patentamt
European Patent Office
Office européen des brevets

(11) EP 1 670 790 B1

(12) # EUROPEAN PATENT SPECIFICATION

(45) Date of publication and mention of the grant of the patent:
**20.08.2008 Bulletin 2008/34**

(21) Application number: **04768675.3**

(22) Date of filing: **24.09.2004**

(51) Int Cl.:
***C07D 409/12*** (2006.01) ***C07D 413/12*** (2006.01)
***C07D 403/12*** (2006.01) ***C07D 235/08*** (2006.01)
***C07D 405/06*** (2006.01) ***A61K 31/4184*** (2006.01)
***A61P 25/00*** (2006.01)

(86) International application number:
**PCT/GB2004/004132**

(87) International publication number:
**WO 2005/030762 (07.04.2005 Gazette 2005/14)**

(54) **BENZIMIDAZOLE DERIVATIVES, COMPOSITIONS CONTAINING THEM, PREPARATION THEREOF AND USES THEREOF**

BENZIMIDAZOLDERIVATE, ZUSAMMENSETZUNGEN, DIE DIESE ENTHALTEN, ZUBEREITUNG DAVON UND DEREN VERWENDUNGEN

DERIVES DE BENZIMIDAZOLE, COMPOSITIONS CONTENANT CES DERIVES, PREPARATION ET UTILISATIONS

(84) Designated Contracting States:
**AT BE BG CH CY CZ DE DK EE ES FI FR GB GR HU IE IT LI LU MC NL PL PT RO SE SI SK TR**

(30) Priority: **26.09.2003 SE 0302572**

(43) Date of publication of application:
**21.06.2006 Bulletin 2006/25**

(73) Proprietor: **AstraZeneca AB**
**151 85 Södertälje (SE)**

(72) Inventors:
- **LIU, Ziping,**
**AstraZeneca R & D Montréal**
**St Laurent, Québec H4S 1Z9 (CA)**
- **MILBURN, Claire,**
**AstraZeneca R & D Montréal**
**St Laurent, Québec H4S 1Z9 (CA)**
- **PAG , Daniel,**
**AstraZeneca R & D Montréal**
**St Laurent, Québec H4S 1Z9 (CA)**
- **WALPOLE, Christopher,**
**AstraZeneca R & D Montréal**
**St Laurent, Québec H4S 1Z9 (CA)**
- **YANG, Hua,**
**AstraZeneca R & D Montréal**
**St Laurent, Québec H4S 1Z9 (CA)**

(74) Representative: **Wahlström, Stig Christer Gunnar**
**AstraZeneca**
**Global Intellectual Property**
**S-151 85 Södertälje (SE)**

(56) References cited:
**EP-A- 0 597 304** **WO-A-02/085866**

- **DATABASE BEILSTEIN BEILSTEIN INSTITUTE FOR ORGANIC CHEMISTRY, FRANKFURT-MAIN, DE; XP002307925 & KOSHCHIENKO ET AL.: KHIM. GETEROTSIKL. SOEDIN., vol. 7, 1971, page 1132,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 670 790 B1

**Description**

## BACKGROUND OF THE INVENTION

1. Field of the invention

The invention is related to therapeutic compounds, pharmaceutical compositions containing these compounds, manufacturing processes thereof and uses thereof. Particularly, the present invention is related to compounds that may be effective in treating pain, multiple sclerosis, Parkinson's disease, cancer, Huntington's chorea, Alzheimer's disease, anxiety disorders, gastrointestinal disorders and/or cardiovascular disorders.

2. Discussion of Relevant Technology

Pain management has been studied for many years. It is known that cannabinoid receptor (e.g., $CB_1$ receptor, $CB_2$ receptor) ligands including agonists, antagonists and inverse agonists produce relief of pain in a variety of animal models by interacting with $CB_1$ and/or $CB_2$ receptors. Generally, $CB_1$ receptors are located predominately in the central nervous system, whereas $CB_2$ receptors are located primarily in the periphery and are primarily restricted to the cells and tissues derived from the immune system.

While $CB_1$ receptor agonists, such as $\Delta^9$-tetrahydrocannabinol ($\Delta^9$-THC) and anadamide, are useful in anti-nociception models in animals, they tend to exert undesired CNS side-effects, e.g., psychoactive side effects, the abuse potential, drug dependence and tolerance, etc. These undesired side effects are known to be mediated by the $CB_1$ receptors located in CNS. There are lines of evidence, however, suggesting that $CB_1$ agonists acting at peripheral sites or with limited CNS exposure can manage pain in humans or animals with much improved overall in vivo profile.

Therefore, there is a need for new $CB_1$ receptor ligands such as agonists that may be useful in managing pain or treating other related symptoms or diseases with reduced or minimal undesirable CNS side-effects.

## DESCRIPTION OF THE EMBODIMENTS

The present invention provides $CB_1$ receptor ligands which may be useful in treating pain and/or other related symptoms or diseases.

Unless specified otherwise within this specification, the nomenclature used in this specification generally follows the examples and rules stated in Nomenclature of Organic Chemistry, Sections A, B, C, D, E, F, and H, Pergamon Press, Oxford, 1979, which is incorporated by references herein for its exemplary chemical structure names and rules on naming chemical structures.

"$CB_1$/$CB_2$ receptors" means $CB_1$ and/or $CB_2$ receptors.

The term "$C_{m-n}$" or "$C_{m-n}$ group" used alone or as a prefix, refers to any group having m to n carbon atoms.

The term "hydrocarbon" used alone or as a suffix or prefix, refers to any structure comprising only carbon and hydrogen atoms up to 14 carbon atoms. The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms. Unless otherwise specified, "alkyl" general includes both saturated alkyl and unsaturated alkyl.

The term "alkylene" used alone or as a suffix or prefix, refers to divalent straight or branched chain hydrocarbon radicals comprising 1 to about 12 carbon atoms, which serves to links two structures together.

The term "alkenyl" used alone or as a suffix or prefix, refers to a monovalent straight or branched chain hydrocarbon radical having at least one carbon-carbon double bond and comprising at least 2 up to about 12 carbon atoms.

The term "alkynyl" used alone or as a suffix or prefix, refers to a monovalent straight or branched chain hydrocarbon radical having at least one carbon-carbon triple bond and comprising at least 2 up to about 12 carbon atoms.

The term "cycloalkyl," used alone or as a suffix or prefix, refers to a monovalent ring-containing hydrocarbon radical comprising at least 3 up to about 12 carbon atoms. "Cycloalkyl" includes both monocyclic and multicyclic hydrocarbon structures. Multicyclic hydrocarbon structure includes non-fused, fused and bridged rings.

The term "cycloalkenyl" used alone or as a suffix or prefix, refers to a monovalent ring-containing hydrocarbon radical having at least one carbon-carbon double bond and comprising at least 3 up to about 12 carbon atoms. "Cycloalkenyl" includes both monocyclic and multicyclic hydrocarbon structures. Multicyclic hydrocarbon structure includes non-fused, fused and bridged rings.

The term "cycloalkynyl" used alone or as a suffix or prefix, refers to a monovalent ring-containing hydrocarbon radical having at least one carbon-carbon triple bond and comprising about 7 up to about 12 carbon atoms. "Cycloalkynyl" includes both monocyclic and multicyclic hydrocarbon structures. Multicyclic hydrocarbon structure includes non-fused,

fused and bridged rings.

The term "aryl" used alone or as a suffix or prefix, refers to a hydrocarbon radical having one or more polyunsaturated carbon rings having aromatic character, (*e.g.,* 4n + 2 delocalized electrons) and comprising 5 up to about 14 carbon atoms, wherein the radical is located on a carbon of the aromatic ring.

The term "non-aromatic group" or "non-aromatic" used alone, as a suffix or prefix, refers to a chemical group or radical that does not contain a ring having aromatic character (*e.g.,* 4n + 2 delocalized electrons).

The term "arylene" used alone or as suffix or prefix, refers to a divalent hydrocarbon radical having one or more polyunsaturated carbon rings having aromatic character, (*e.g.,* 4n + 2 delocalized electrons) and comprising 5 up to about 14 carbon atoms, which serves to link two structures together.

The term "heterocycle" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s). Heterocycle may be saturated or unsaturated, containing one or more double bonds, and heterocycle may contain more than one ring. When a heterocycle contains more than one ring, the rings may be fused or unfused. Fused rings generally refer to at least two rings share two atoms therebetween. Heterocycle may have aromatic character or may not have aromatic character.

The term "heteroalkyl" used alone or as a suffix or prefix, refers to a radical formed as a result of replacing one or more carbon atom of an alkyl with one or more heteroatoms selected from N, O, P and S.

The term "heteroaromatic" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s), wherein the ring-containing structure or molecule has an aromatic character (*e.g.,* 4n + 2 delocalized electrons).

The term "heterocyclic group," "heterocyclic moiety," "heterocyclic," or "heterocyclo" used alone or as a suffix or prefix, refers to a radical derived from a heterocycle by removing one or more hydrogens therefrom.

The term "heterocyclyl" used alone or as a suffix or prefix, refers a radical derived from a heterocycle by removing one hydrogen from a carbon of a ring of the heterocycle.

The term "heterocyclylene" used alone or as a suffix or prefix, refers to a divalent radical derived from a heterocycle by removing two hydrogens therefrom, which serves to links two structures together.

The term "heteroaryl" used alone or as a suffix or prefix, refers to a heterocyclyl having aromatic character, wherein the radical of the heterocyclyl is located on a carbon of an aromatic ring of the heterocyclyl. A heteroaryl may contain both aromatic and non-aromatic rings therein. These rings may be fused or otherwised linked together.

The term "heterocylcoalkyl" used alone or as a suffix or prefix, refers to a heterocyclyl that does not have aromatic character.

The term "heteroarylene" used alone or as a suffix or prefix, refers to a heterocyclylene having aromatic character.

The term "heterocycloalkylene" used alone or as a suffix or prefix, refers to a heterocyclylene that does not have aromatic character.

The term "six-membered" used as a prefix refers to a group having a ring that contains six ring atoms.

The term "five-membered" used as a prefix refers to a group having a ring that contains five ring atoms.

A five-membered ring heteroaryl is a heteroaryl with a ring having five ring atoms wherein 1, 2 or 3 ring atoms are independently selected from N, O and S.

Exemplary five-membered ring heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4- oxadiazolyl.

A six-membered ring heteroaryl is a heteroaryl with a ring having six ring atoms wherein 1, 2 or 3 ring atoms are independently selected from N, O and S.

Exemplary six-membered ring heteroaryls are pyridyl, pyrazinyl, pyrimidinyl, triazinyl and pyridazinyl.

The term "substituted" used as a prefix refers to a structure, molecule or group, wherein one or more hydrogens are replaced with one or more $C_{1-12}$hydrocarbon groups, or one or more chemical groups containing one or more heteroatoms selected from N, O, S, F, Cl, Br, I, and P. Exemplary chemical groups containing one or more heteroatoms include heterocyclyl, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$ -S(=O)R, - CN, -OH, -C(=O)OR, $-C(=O)NR_2$, -NRC(=O)R, oxo (=O), imino (=NR), thio (=S), and oximino (=N-OR), wherein each "R" is a $C_{1-12}$hydrocarbyl. For example, substituted phenyl may refer to nitrophenyl, pyridylphenyl, methoxyphenyl, chlorophenyl, aminophenyl, etc., wherein the nitro, pyridyl, methoxy, chloro, and amino groups may replace any suitable hydrogen on the phenyl ring.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

The term "optionally substituted" refers to both groups, structures, or molecules that are substituted and those that are not substituted.

Heterocycle includes, for example, monocyclic heterocycles such as: aziridine, oxirane, thiirane, azetidine, oxetane, thietane, pyrrolidine, pyrroline, imidazolidine, pyrazolidine, pyrazoline, dioxolane, sulfolane 2,3-dihydrofuran, 2,5-dihydrofuran tetrahydrofuran, thiophane, piperidine, 1,2,3,6-tetrahydro-pyridine, piperazine, morpholine, thiomorpholine, pyran, thiopyran, 2,3-dihydropyran, tetrahydropyran, 1,4-dihydropyridine, 1,4-dioxane, 1,3-dioxane, dioxane, homopiperidine, 2,3,4,7-tetrahydro-1*H*-azepine homopiperazine, 1,3-dioxepane, 4,7-dihydro-1,3-dioxepin, and hexamethylene oxide.

In addition, heterocycle includes aromatic heterocycles, for example, pyridine, pyrazine, pyrimidine, pyridazine, thiophene, furan, furazan, pyrrole, imidazole, thiazole, oxazole, pyrazole, isothiazole, isoxazole, 1,2,3-triazole, tetrazole, 1,2,3-thiadiazole, 1,2,3-oxadiazole, 1,2,4-triazole, 1,2,4-thiadiazole, 1,2,4-oxadiazole, 1,3,4-triazole, 1,3,4-thiadiazole, and 1,3,4- oxadiazole.

Additionally, heterocycle encompass polycyclic heterocycles, for example, indole, indoline, isoindoline, quinoline, tetrahydroquinoline, isoquinoline, tetrahydroisoquinoline, 1,4-benzodioxan, coumarin, dihydrocoumarin, benzofuran, 2,3-dihydrobenzofuran, isobenzofuran, chromene, chroman, isochroman, xanthene, phenoxathiin, thianthrene, indolizine, isoindole, indazole, purine, phthalazine, naphthyridine, quinoxaline, quinazoline, cinnoline, pteridine, phenanthridine, perimidine, phenanthroline, phenazine, phenothiazine, phenoxazine, 1,2-benzisoxazole, benzothiophene, benzoxazole, benzthiazole, benzimidazole, benztriazole, thioxanthine, carbazole, carboline, acridine, pyrolizidine, and quinolizidine.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidine, diazabicyclo[2.2.1]heptane and 7-oxabicyclo[2.2.1]heptane.

Heterocyclyl includes, for example, monocyclic heterocyclyls, such as: aziridinyl, oxiranyl, thiiranyl, azetidinyl, oxetanyl, thietanyl, pyrrolidinyl, pyrrolinyl, imidazolidinyl, pyrazolidinyl, pyrazolinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydropyridinyl, piperazinyl, morpholinyl, thiomorpholinyl, pyranyl, thiopyranyl, 2,3-dihydropyranyl, tetrahydropyranyl, 1,4-dihydropyridinyl, 1,4-dioxanyl, 1,3-dioxanyl, dioxanyl, homopiperidinyl, 2,3,4,7-tetrahydro-1*H*-azepinyl, homopiperazinyl, 1,3-dioxepanyl, 4,7-dihydro-1,3-dioxepinyl, and hexamethylene oxidyl. In addition, heterocyclyl includes aromatic heterocyclyls or heteroaryl, for example, pyridinyl, pyrazinyl, pyrimidinyl, pyridazinyl, thienyl, furyl, furazanyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4 oxadiazolyl.

Additionally, heterocyclyl encompasses polycyclic heterocyclyls (including both aromatic or non-aromatic), for example, indolyl, indolinyl, isoindolinyl, quinolinyl, tetrahydroquinolinyl, isoquinolinyl, tetrahydroisoquinolinyl, 1,4-benzodioxanyl, coumarinyl, dihydrocoumarinyl, benzofuranyl, 2,3-dihydrobenzofuranyl, isobenzofuranyl, chromenyl, chromanyl, isochromanyl, xanthenyl, phenoxathiinyl, thianthrenyl, indolizinyl, isoindolyl, indazolyl, purinyl, phthalazinyl, naphthyridinyl, quinoxalinyl, quinazolinyl, cinnolinyl, pteridinyl, phenanthridinyl, perimidinyl, phenanthrolinyl, phenazinyl, phenothiazinyl, phenoxazinyl, 1,2-benzisoxazolyl, benzothiophenyl, benzoxazolyl, benzthiazolyl, benzimidazolyl, benztriazolyl, thioxanthinyl, carbazolyl, carbolinyl, acridinyl, pyrolizidinyl, and quinolizidinyl.

In addition to the polycyclic heterocyclyls described above, heterocyclyl includes polycyclic heterocyclyls wherein the ring fusion between two or more rings includes more than one bond common to both rings and more than two atoms common to both rings. Examples of such bridged heterocyclyls include quinuclidinyl, diazabicyclo[2.2.1]heptyl; and 7-oxabicyclo[2.2.1]heptyl.

The term "alkoxy" used alone or as a suffix or prefix, refers to radicals of the general formula -O-R, wherein -R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "aryloxy" used alone or as a suffix or prefix, refers to radicals of the general formula -O-Ar, wherein -Ar is an aryl.

The term "heteroaryloxy" used alone or as a suffix or prefix, refers to radicals of the general formula -O-Ar', wherein -Ar' is a heteroaryl.

The term "amine" or "amino" used alone or as a suffix or prefix, refers to radicals of the general formula -NRR', wherein R and R' are independently selected from hydrogen or a hydrocarbon radical.

"Acyl" used alone, as a prefix or suffix, means -C(=O)-R, wherein -R is an optionally substituted hydrocarbyl, hydrogen, amino or alkoxy. Acyl groups include, for example, acetyl, propionyl, benzoyl, phenyl acetyl, carboethoxy, and dimethylcarbamoyl.

Halogen includes fluorine, chlorine, bromine and iodine.

"Halogenated," used as a prefix of a group, means one or more hydrogens on the group is replaced with one or more halogens.

"RT" or "rt" means room temperature.

A first ring group being "fused" with a second ring group means the first ring and the second ring share at least two atoms therebetween.

"Link," "linked," or "linking," unless otherwise specified, means covalently linked or bonded.

When a first group, structure, or atom is "directly connected" to a second group, structure or atom, at least one atom of the first group, structure or atom forms a chemical bond with at least one atom of the second group, structure or atom.

"Saturated carbon" means a carbon atom in a structure, molecule or group wherein all the bonds connected to this carbon atom are single bond. In other words, there is no double or triple bonds connected to this carbon atom and this carbon atom generally adopts an *sp*[3] atomic orbital hybridization.

"Unsaturated carbon" means a carbon atom in a structure, molecule or group wherein at least one bond connected to this carbon atom is not a single bond. In other words, there is at least one double or triple bond connected to this carbon atom and this carbon atom generally adopts a *sp* or *sp*[2] atomic orbital hybridization.

In one aspect, an embodiment of the invention provides a compound of Formula I, a pharmaceutically acceptable salt thereof, diastereomers, enantiomers, or mixtures thereof:

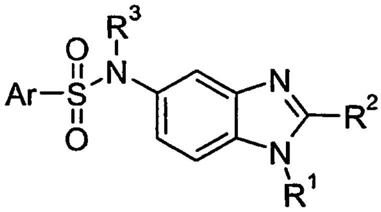

**I**

wherein

$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkcyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy and amino;

$R^2$ is selected from $C_{1-10}$alkyl, $C_{2-10}$alkenyl, $C_{3-10}$cyloalkyl, $C_{3-10}$cycloalkyl-$C_{1-4}$aklyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, ethoxy, methyl, ethyl, hydroxy, and amino;

$R^3$ is selected from -H, $C_{1-6}$alkyl, $C_{2-6}$alkenyl, $C_{3-6}$cycloalkyl, and $C_{3-6}$cycloalkyl-$C_{1-4}$alkyl; and

Ar is selected from $C_{6-10}$aryl and $C_{3-6}$heteroaryl, wherein said $C_{6-10}$aryl and $C_{3-6}$heteroaryl are optionally substituted with one or more groups selected from $C_{1-3}$alkyl, $C_{1-6}$alkoxy, $C_{1-6}$alkylaminocarbonyl and halogen.

In another embodiment, the compounds of the present invention are those of Formula I, wherein

$R^1$ is selected from $C_{1-6}$alkyl, $C_{2-6}$alkenyl, $C_{3-6}$cyloalkyl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloakyl-$C_{1-4}$alkyl used in defining $R^1$ is optionally substituted by one or more groups selected from halogen, methoxy, ethoxy, methyl, hydroxy and amino;

$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$cyloalkenyl-$C_{1-4}$alkyl, wherein said $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, methoxy, ethoxy and hydroxy;

$R^3$ is selected from -H and $C_{1-3}$alkyl; and

Ar is selected from phenyl and $C_{3-6}$heteroaryl, wherein said phenyl and $C_{3-6}$heteroaryl are optionally substituted with one or more groups selected from methyl, methoxy, fluoro, chloro, bromo and iodo.

In a further embodiment, the compounds of the present invention are those of Formula I,

wherein $R^1$ is selected from cyclopentyl-methyl, cyclohexyl-methyl, cyclobutyl-methyl, cyclopropyl-methyl, 4,4-difluorocyclohexanemethyl, tetrahydropyranyl-methyl, tetrahydrofuranyl-methyl, morpholinyl-methyl, piperdinylethyl, N-methyl-piperdinyl-methyl and piperdinyl-methyl;

$R^2$ is selected from t-butyl, n-butyl, 2-methyl-2-butyl, isopentyl, 2-methoxy-2-propyl, 2-hydroxy-propyl, 1-methyl-propyl, 1,1-dimethyl-propyl, 1,1-dimethyl-3-buten-1-yl, trifluoromethyl, 1,1-difluoroethyl, 2,2,2-trifluoroethyl, ethyl, and 2-propyl;

$R^3$ is selected from -H and methyl; and

Ar is selected from phenyl, pyridyl, pyrimidyl, thiazolyl, thienyl, isoxazolyl, imidazolyl, and pyrazolyl, wherein said phenyl, pyridyl, pyrimidyl, thiazolyl, thienyl, isoxazolyl, imidazolyl, and pyrazolyl are optionally substituted with one or more groups selected from methyl, methoxy, fluoro and chloro.

In an even further embodiment, the compounds of the present invention are those of Formula I, wherein

$R^1$ is cyclohexyl-methyl, tetrahydropyranyl-methyl and 4,4-difluorocyclohexanemethyl,;

$R^2$ is t-butyl and 1,1-difluoroethyl;

$R^3$ is selected from -H and methyl; and

Ar is selected from phenyl, pyridyl, thiazolyl, thienyl, isoxazolyl, imidazolyl, and pyrazolyl, wherein said phenyl, pyridyl, thiazolyl, thienyl, isoxazolyl, imidazolyl, and pyrazolyl are optionally substituted with one or more methyl and fluoro groups.

It will be understood that when compounds of the present invention contain one or more chiral centers, the compounds of the invention may exist in, and be isolated as, enantiomeric or diastereomeric forms, or as a racemic mixture. The present invention includes any possible enantiomers, diastereomers, racemates or mixtures thereof, of a compound of Formula I. The optically active forms of the compound of the invention may be prepared, for example, by chiral chromatographic separation of a racemate, by synthesis from optically active starting materials or by asymmetric synthesis based on the procedures described thereafter.

It will also be appreciated that certain compounds of the present invention may exist as geometrical isomers, for example E and Z isomers of alkenes. The present invention includes any geometrical isomer of a compound of Formula I. It will further be understood that the present invention encompasses tautomers of the compounds of the Formula I.

It will also be understood that certain compounds of the present invention may exist in solvated, for example hydrated, as well as unsolvated forms. It will further be understood that the present invention encompasses all such solvated forms of the compounds of the Formula I.

Within the scope of the invention are also salts of the compounds of the Formula I. Generally, pharmaceutically acceptable salts of compounds of the present invention may be obtained using standard procedures well known in the art, for example by reacting a sufficiently basic compound, for example an alkyl amine with a suitable acid, for example, HCl or acetic acid, to afford a physiologically acceptable anion. It may also be possible to make a corresponding alkali metal (such as sodium, potassium, or lithium) or an alkaline earth metal (such as a calcium) salt by treating a compound of the present invention having a suitably acidic proton, such as a carboxylic acid or a phenol with one equivalent of an alkali metal or alkaline earth metal hydroxide or alkoxide (such as the ethoxide or methoxide), or a suitably basic organic amine (such as choline or meglumine) in an aqueous medium, followed by conventional purification techniques.

In one embodiment, the compound of Formula I above may be converted to a pharmaceutically acceptable salt or solvate thereof, particularly, an acid addition salt such as a hydrochloride, hydrobromide, phosphate, acetate, fumarate, maleate, tartrate, citrate, methanesulphonate or *p*-toluenesulphonate.

We have now found that the compounds of the invention have activity as pharmaceuticals, in particular as modulators or ligands such as agonists, partial agonists, inverse agonist or antagonists of $CB_1$ receptors. More particularly, the compounds of the invention exhibit selective activity as agonist of the $CB_1$ receptors and are useful in therapy, especially for relief of various pain conditions such as chronic pain, neuropathic pain, acute pain, cancer pain, pain caused by rheumatoid arthritis, migraine, visceral pain etc. This list should however not be interpreted as exhaustive. Additionally, compounds of the present invention are useful in other disease states in which dysfunction of $CB_1$ receptors is present or implicated. Furthermore, the compounds of the invention may be used to treat cancer, multiple sclerosis, Parkinson's disease, cancer, Huntington's chorea, Alzheimer's disease, anxiety disorders, gastrointestinal disorders and cardiovascular disorders.

Compounds of the invention are useful as immunomodulators, especially for autoimmune diseases, such as arthritis, for skin grafts, organ transplants and similar surgical needs, for collagen diseases, various allergies, for use as anti-tumour agents and anti viral agents.

Compounds of the invention are useful in disease states where degeneration or dysfunction of cannabinoid receptors is present or implicated in that paradigm. This may involve the use of isotopically labelled versions of the compounds of the invention in diagnostic techniques and imaging applications such as positron emission tomography (PET).

Compounds of the invention are useful for the treatment of diarrhoea, depression, anxiety and stress-related disorders such as post-traumatic stress disorders, panic disorder, generalized anxiety disorder, social phobia, and obsessive compulsive disorder, urinary incontinence, premature ejaculation, various mental illnesses, cough, lung oedema, various gastro-intestinal disorders, e.g. constipation, functional gastrointestinal disorders such as Irritable Bowel Syndrome and Functional Dyspepsia, Parkinson's disease and other motor disorders, traumatic brain injury, stroke, cardioprotection following miocardial infarction, spinal injury and drug addiction, including the treatment of alcohol, nicotine, opioid and other drug abuse and for disorders of the sympathetic nervous system for example hypertension.

Compounds of the invention are useful as an analgesic agent for use during general anaesthesia and monitored anaesthesia care. Combinations of agents with different properties are often used to achieve a balance of effects needed

to maintain the anaesthetic state (e.g. amnesia, analgesia, muscle relaxation and sedation). Included in this combination are inhaled anaesthetics, hypnotics, anxiolytics, neuromuscular blockers and opioids.

Also within the scope of the invention is the use of any of the compounds according to the Formula I above, for the manufacture of a medicament for the treatment of any of the conditions discussed above.

Thus, the invention provides a compound of Formula I, or pharmaceutically acceptable salt or solvate thereof, as hereinbefore defined for use in therapy.

In a further aspect, the present invention provides the use of a compound of Formula I, or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The term "therapeutic" and "therapeutically" should be contrued accordingly. The term "therapy" within the context of the present invention further encompasses to administer an effective amount of a compound of the present invention, to mitigate either a pre-existing disease state, acute or chronic, or a recurring condition. This definition also encompasses prophylactic therapies for prevention of recurring conditions and continued therapy for chronic disorders.

The compounds of the present invention are useful in therapy, especially for the therapy of various pain conditions including, but not limited to: acute pain, chronic pain, neuropathic pain, back pain, cancer pain, and visceral pain.

In use for therapy in a warm-blooded animal such as a human, the compound of the invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

In one embodiment of the invention, the route of administration may be oral, intravenous or intramuscular.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of this invention, inert, pharmaceutically acceptable carriers can be either solid and liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or table disintegrating agents; it can also be an encapsulating material.

In powders, the carrier is a finely divided solid, which is in a mixture with the finely divided compound of the invention, or the active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture in then poured into convenient sized moulds and allowed to cool and solidify.

Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term composition is also intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form compositions include solutions, suspensions, and emulsions. For example, sterile water or water propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will preferably include from 0.05% to 99%w (per cent by weight), more preferably from 0.10 to 50%w, of the compound of the invention, all percentages by weight being based on total composition.

A therapeutically effective amount for the practice of the present invention may be determined, by the use of known criteria including the age, weight and response of the individual patient, and interpreted within the context of the disease which is being treated or which is being prevented, by one of ordinary skills in the art.

Within the scope of the invention is the use of any compound of Formula I as defined above for the manufacture of a medicament.

Also within the scope of the invention is the use of any compound of Formula I for the manufacture of a

medicament for the therapy of pain.

Additionally provided is the use of any compound according to Formula I for the manufacture of a medicament for the therapy of various pain conditions including, but not limited to: acute pain, chronic pain, neuropathic pain, back pain, cancer pain, and visceral pain.

Additionally, there is provided a pharmaceutical composition comprising a compound of Formula I, or a pharmaceutically acceptable salt thereof, in association with a pharmaceutically acceptable carrier.

Particularly, there is provided a pharmaceutical composition comprising a compound of Formula I, or a pharmaceutically acceptable salt thereof, in association with a pharmaceutically acceptable carrier for therapy, more particularly for therapy of pain.

Further, there is provided a pharmaceutical composition comprising a compound of Formula I, or a pharmaceutically acceptable salt thereof, in association with a pharmaceutically acceptable carrier use in any of the conditions discussed above.

In a further aspect, the present invention provides a method of preparing the compounds of the present invention.

In one embodiment, the invention provides a process for preparing a compound of Formula I,

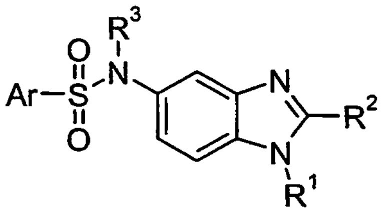

**I**

comprising:

reacting a compound of Formula II,

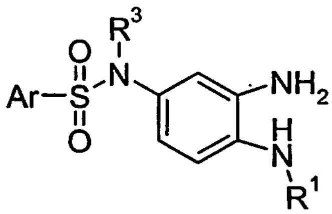

**II**

with a compound of $R^2COX$, in the presence of a base, such as an alkylamine, and optionally a coupling reagent, such as HATU, EDC;

wherein

X is selected from Cl, Br, F and OH;

$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cyloalkyl, $C_{4-8}$cycloalkenyl, and $C_{3-6}$heterocycloalkyl, wherein said $C_{1-10}$alkyl, $C_{2-10}$akenyl, $C_3$-$_{10}$cycloalk-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cyloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy and amino;

$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloakenyl-$C_{1-4}$alkyl, wherein said $C_{1-10}$alkyl, $C_{2-10}$alkenyl, $C_3$-$_{10}$cycloakyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, ethoxy, methyl, ethyl, hydroxy, and amino;

$R^3$ is selected from -H, $C_{1-6}$alkyl, $C_{2-6}$alkenyl, $C_{3-6}$cycloalkyl, and $C_{3-6}$cycloalkyl-$C_{1-4}$alkyl; and

Ar is selected from $C_{6-10}$aryl and $C_3$-$_6$heteroaryl, wherein said $C_{6-10}$aryl and $C_3$-$_6$heteroaryl are optionally substituted with one or more groups selected from $C_{1-3}$alkyl, $C_{1-6}$alkoxy, $C_{1-6}$alkylaminocarbonyl and halogen.

Compounds of the present invention may also be prepared according to the synthetic routes as depicted in Schemes 1-3.

**Scheme 1**

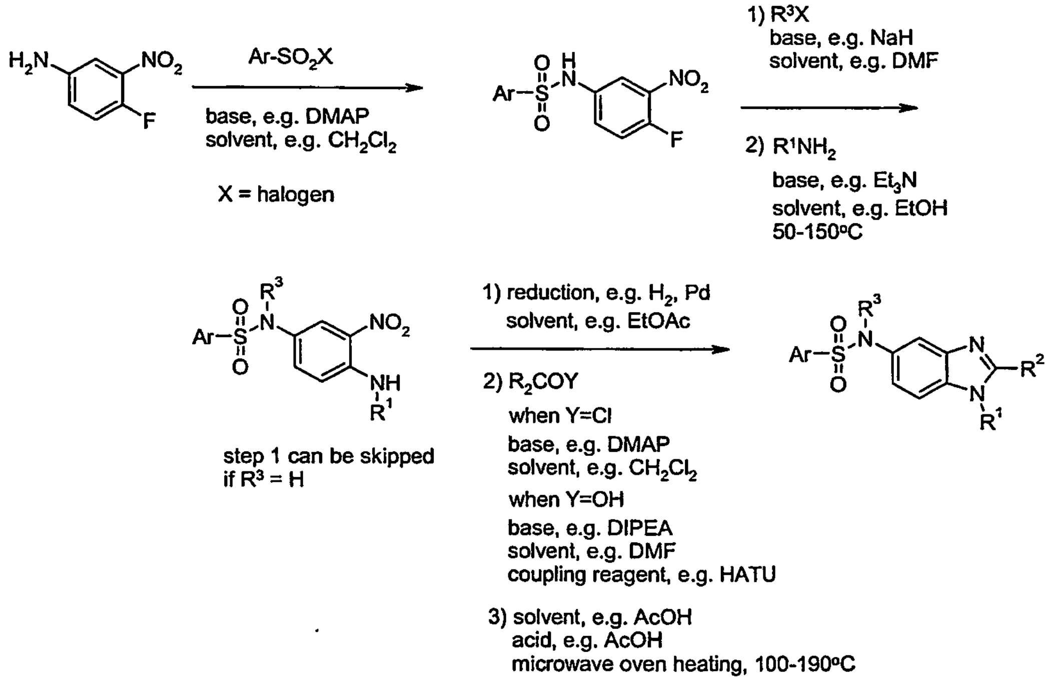


Ar, $R^1$, $R^2$ and $R^3$ are as defined in the specifications.

**Scheme 2**

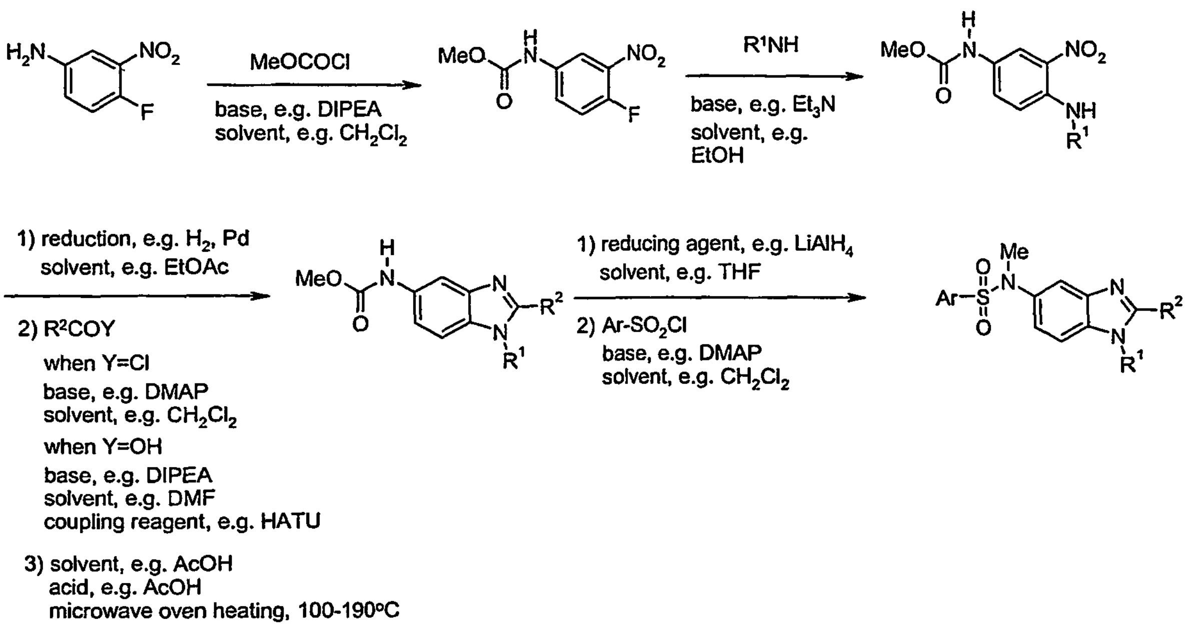


n, Ar, $R^1$, $R^2$ and $R^3$ are as defined in the specifications.

**Scheme 3**

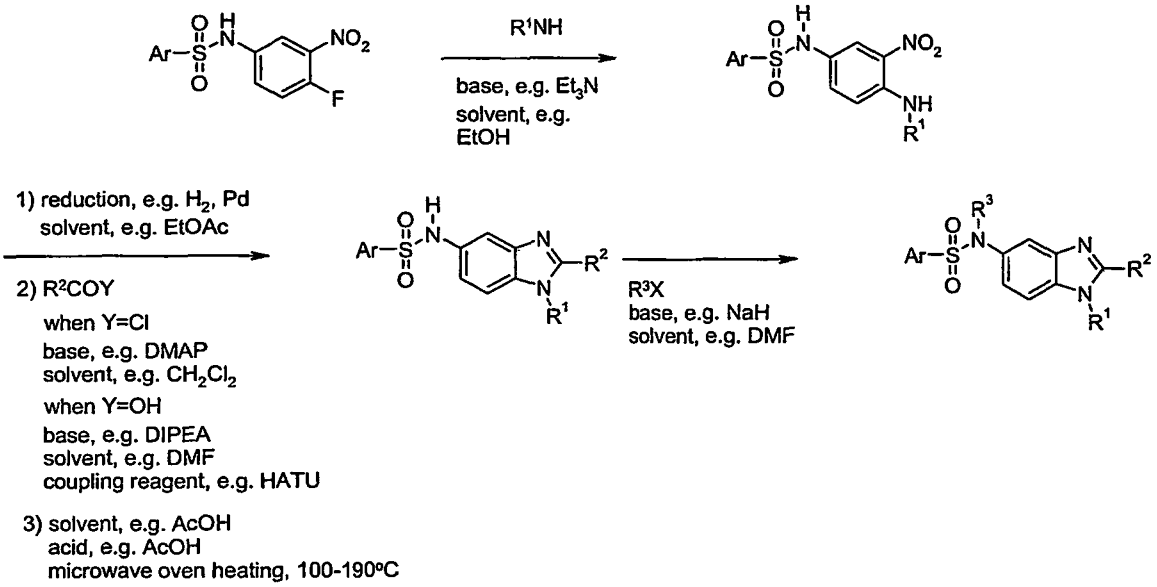


Ar, $R^1$, $R^2$ and $R^3$ are as defined in the specifications.

**Scheme 4**

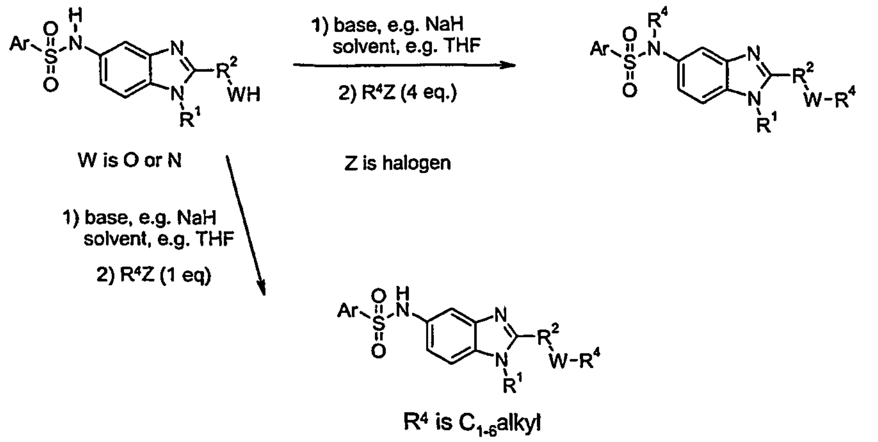


$R^1$, $R^2$, and Ar are as defined in the specifications.

## Biological Evaluation

### $hCB_1$ and $hCB_2$ receptor binding

Human $CB_1$ receptor from Receptor Biology ($hCB_1$) or human $CB_2$ receptor from BioSignal ($hCB_2$) membranes are thawed at 37 °C, passed 3 times through a 25-gauge blunt-end needle, diluted in the cannabinoid binding buffer (50 mM Tris, 2.5 mM EDTA, 5 mM $MgCl_2$, and 0.5 mg/mL BSA fatty acid free, pH 7.4) and aliquots containing the appropriate amount of protein are distributed in 96-well plates. The $IC_{50}$ of the compounds of the invention at $hCB_1$ and $hCB_2$ are evaluated from 10-point dose-response curves done with $^3H$-CP55,940 at 20000 to 25000 dpm per well (0.17-0.21 nM) in a final volume of 300 μl. The total and non-specific binding are determined in the absence and presence of 0.2 μM of HU210 respectively. The plates are vortexed and incubated for 60 minutes at room temperature, filtered through

Unifilters GF/B (presoaked in 0.1% polyethyleneimine) with the Tomtec or Packard harvester using 3 mL of wash buffer (50 mM Tris, 5 mM $MgCl_2$, 0.5 mg BSA pH 7.0). The filters are dried for 1 hour at 55 °C. The radioactivity (cpm) is counted in a TopCount (Packard) after adding 65 µl/well of MS-20 scintillation liquid.

$hCB_1$ and $hCB_2$ GTPγS binding

Human $CB_1$ receptor from Receptor Biology ($hCB_1$) or human $CB_2$ receptor membranes (BioSignal) are thawed at 37 °C, passed 3 times through a 25-gauge blunt-end needle and diluted in the GTPγS binding buffer (50 mM Hepes, 20 mM NaOH, 100 mM NaCl, 1 mM EDTA, 5 mM $MgCl_2$, pH 7.4,0.1% BSA). The $EC_{50}$ and $E_{max}$ of the compounds of the invention are evaluated from 10-point dose-response curves done in 300µl with the appropriate amount of membrane protein and 100000-130000 dpm of $GTPg^{35}S$ per well (0.11 -0.14 nM). The basal and maximal stimulated binding is determined in absence and presence of 1 µM ($hCB_2$) or 10 µM ($hCB_1$) Win 55,212-2 respectively. The membranes are pre-incubated for 5 minutes with 56.25 µM (hCB2) or 112.5 µM ($hCB_1$) GDP prior to distribution in plates (15 µM ($hCB_2$) or 30 µM ($hCB_1$) GDP final). The plates are vortexed and incubated for 60 minutes at room temperature, filtered on Unifilters GF/B (presoaked in water) with the Tomtec or Packard harvester using 3 ml of wash buffer (50 mM Tris, 5 mM $MgCl_2$, 50 mM NaCl, pH 7.0). The filters are dried for 1 hour at 55°C. The radioactivity (cpm) is counted in a TopCount (Packard) after adding 65 µl/well of MS-20 scintillation liquid. Antagonist reversal studies are done in the same way except that (a) an agonist dose-response curve is done in the presence of a constant concentration of antagonist, or (b) an antagonist dose-response curve is done in the presence of a constant concentration of agonist.

Based on the above assays, the dissociation constant (Ki) for a particular compound of the invention towards a particular receptor is determined using the following equation:

$$Ki = IC_{50}/(1+[rad]/Kd),$$

Wherein $IC_{50}$ is the concentration of the compound of the invention at which 50% displacement has been observed;
[rad] is a standard or reference radioactive ligand concentration at that moment; and
Kd is the dissociation constant of the radioactive ligand towards the particular receptor.

Using the above-mentioned assays, the Ki towards human $CB_1$ receptors for most compounds of the invention is measured to be in the range of 1.7-5000 nM. The Ki towards human $CB_2$ receptors for most compounds of the invention is measured to be in the range of about 0.5-22.2 nM. The $EC_{50}$ towards human $CB_1$ receptors for most compounds of the invention is measured to be in the range of about 4.7-10000 nM. The $E_{max}$ towards human $CB_1$ receptors for most compounds of the invention is measured to be in the range of about 22.3-130%.

In one embodiment, the Ki towards human $CB_1$ receptors for most compounds of the invention is measured to be in the range of 1.7-50 nM. The Ki towards human $CB_2$ receptors for most compounds of the invention is measured to be in the range of about 0.5-15 nM. The $EC_{50}$ towards human $CB_1$ receptors for most compounds of the invention is measured to be in the range of about 4.7-100 nM. The $E_{max}$ towards human $CB_1$ receptors for most compounds of the invention is measured to be in the range of about 60-113%.

## EXAMPLES

The invention will further be described in more detail by the following Examples which describe methods whereby compounds of the present invention may be prepared, purified, analyzed and biologically tested, and which are not to be construed as limiting the invention.

### Example 1

***N*-[2-*tert*-Butyl-1-(cyclohexylmethyl)-1*H*-benzimidazol-5-yl]thiophene-2-sulfonamide**

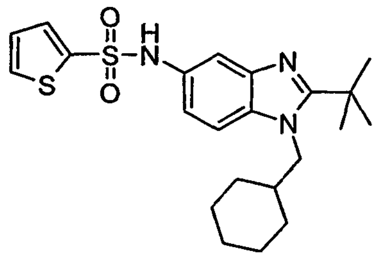

**Step A. *N*-[2-*tert*-Butyl-1-(cyclohexylmethyl)-1*H*-benzimidazol-5-yl]thiophene-2-sulfonamide**

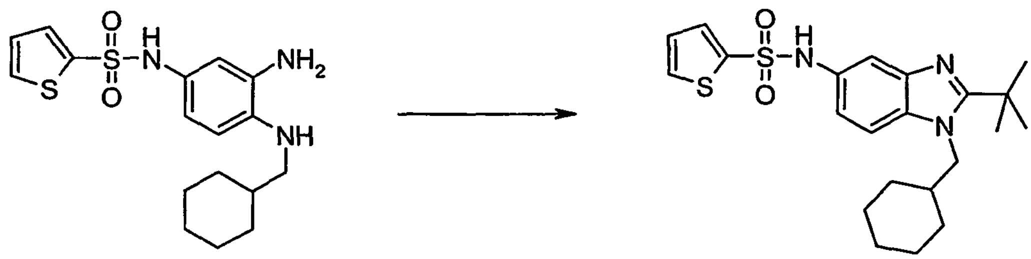

*N*-{3-Amino-4-[(cyclohexylmethyl)amino]phenyl}thiophene-2-sulfonamide (55 mg, 0.150 mmol) (for preparation, see the following steps B, C and D) was dissolved in 3 mL of 1,2-dichloroethane containing TEA (0.030 mL, 0.225 mmol). Trimethylacetyl chloride (0.020 mL, 0.165 mmol) was added dropwise and the solution was stirred at rt for 1h. Glacial AcOH (1 mL) and a few drops of concentrated HCl were added and the solution was stirred at 80°C overnight. The solvent was evaporated. The crude product was dissolved in EtOAc and washed with 2M NaOH aqueous solution, brine and dried over anhydrous $MgSO_4$. The product was purified by reversed-phase HPLC using 20-80% $CH_3CN/H_2O$ and then lyophilized affording the title compound as the corresponding TFA salt. Yield: 10 mg (15%); $^1H$ NMR (400 MHz, METHANOL-$D_4$): δ 1.19 (m, 5 H), 1.57 (m, 1 H), 1.59 (m, 1 H), 1.61 (s, 9 H), 1.66 (m, 1 H), 1.73 (m, 2 H), 2.05 (m, 1 H), 4.37 (d, J=7.62 Hz, 2 H), 7.02 (dd, J=4.98, 3.81 Hz, 1 H), 7.25 (dd, J=8.98, 2.15 Hz, 1 H), 7.52 (dd, J=3.71, 1.37 Hz, 1 H), 7.66 (d, J=2.15 Hz, 1 H), 7.69 (dd, J=5.08, 1.37 Hz, 1 H), 7.76 (d, J=8.98 Hz, 1 H); MS (ESI) $(M+H)^+$ 432.1; Anal. Calcd for $C_{22}H_{29}N_3O_2S_2$ + 1.4 TFA + 0.6 $H_2O$: C, 49.48; H, 5.29; N, 6.98. Found: C, 49.47; H, 5.36; N, 6.83.

**Step B. *N*-(4-Fluoro-3-nitrophenyl)thiophene-2-sulfonamide**

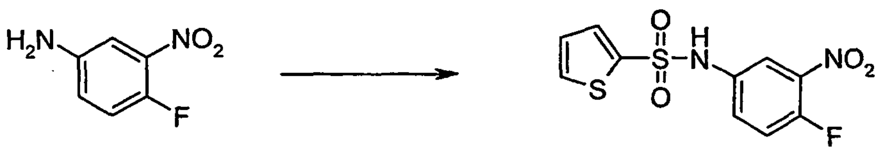

4-Fluoro-3-nitroaniline (1.00 g, 6.41 mmol) and 2-thiophenesulfonyl chloride (1.75 g, 9.62 mmol) were stirred in dichloromethane (150 mL) containing DMAP (1.17g, 9.62 mmol) at rt for 24 h. The solution was washed with 5% $KHSO_4$ aqueous solution, saturated $NaHCO_3$ aqueous solution, brine and dried over anhydrous $MgSO_4$. The crude product was purified by flash chromatography with dichloromethane as eluent on silica gel to afford the title product. Yield: 425 mg (22%); $^1H$ NMR (400 MHz, CHLOROFORM-D): δ 6.90 (m, 1H), 7.08 (dd, J= 5.08, 3.91 Hz, 1H), 7.26 (t, J = 10.35 Hz, 1H), 7.49 (m, 1H), 7.56 (dd, J = 3.71,1.37 Hz, 1H), 7.63 (dd, J = 5.08, 1.37 Hz, 1H), 7.78 (dd, J = 6.35, 2.83Hz, 1H).

**Step C. *N*-{4-[(Cyclohenylmethyl)amino]-3-nitrophenyl}thiophene-2-sulfonamide**

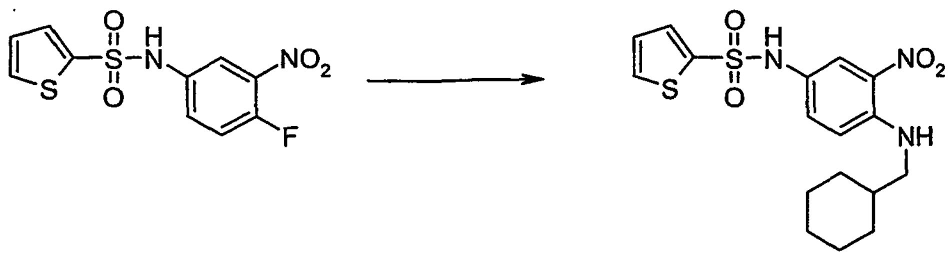

*N*-(4-Fluoro-3-nitrophenyl)thiophene-2-sulfonamide (73 mg, 0.241 mmol) and cyclohexylmethyl amine (0.040 mL, 0.289 mmol) were stirred in 3 mL of EtOH containing TEA (0.050 mL, 0.361 mmol) at 75°C for 6h. The solvent was evaporated. The crude product was dissolved in EtOAc and washed with 5% $KHSO_4$ solution, saturated $NaHCO_3$ solution, brine and dried over anhydrous $MgSO_4$. The crude product was purified by flash chromatography using 2:1 / hexanes:EtOAc on silica gel. Yield: 60 mg (63%); $^1$H NMR (400 MHz, CHLOROFORM-D): δ 1.03 (m, 2H), 1.26 (m, 3H), 1.61 (m, 2H), 1.76 (m, 2H), 1.82 (m, 4H), 3.13 (dd, J = 6.64, 5.47Hz, 2H), 6.46 (m, 1H), 6.82 (d, J = 9.18Hz, 1H), 7.05 (dd, J = 4.98, 3.81Hz, 1H), 7.40 (dd, J = 9.18, 2.54Hz, 1H), 7.47 (dd, J = 3.71, 1.37Hz, 1H), 7.59 (dd, J = 5.08, 1.37Hz, 1H), 7.73 (d, J = 2.73Hz, 1H), 8.17 (m, 1H).

**Step D. *N*-{3-Amino4-[(cyclohexylmethyl)amino]phenyl}thiophene-2-sulfonamide**

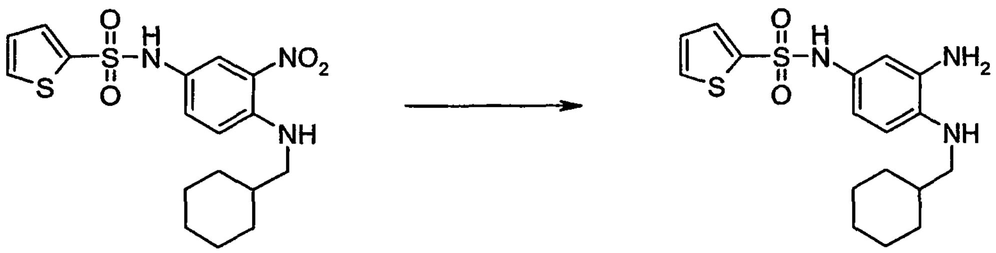

*N*-{4-[(Cyclohexylmethyl)amino]-3-nitrophenyl}thiophene-2-sulfonamide (60 mg, 0.152 mmol) was dissolved in 5 mL of DMF under nitrogen. Tin (II) chloride dihydrate (170 mg, 0.760 mmol) was added and the solution stirred at rt for 6h. More tin(II) chloride dihydrate (170 mg, 0.760 mmol) was added and the solution stirred at rt overnight. The reaction mixture was quenched by addition of saturated $NaHCO_3$ solution at 0°C. The solution was then extracted (2X) with EtOAc and washed with brine and dried over anhydrous $MgSO_4$. The product was used directly for Step A without further purification. Yield: 55 mg (99%); MS (ESI) $(M+H)^+$ 366.14.

**Example 2**

***N*-[2-*tert*-Butyl-1-(cyclohexylmethyl)-1*H*-benzimidazol-5-yl]-*N*-methylthiophene-2-sulfonamide**

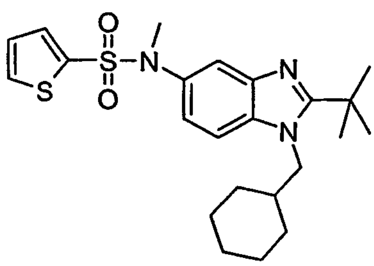

**Step A. *N*-[2-*tert*-Butyl-1-(cyclohexylmethyl)-1*H*-benzimidazol-5-yl]-*N*-methylthiophene-2-sulfonamide**

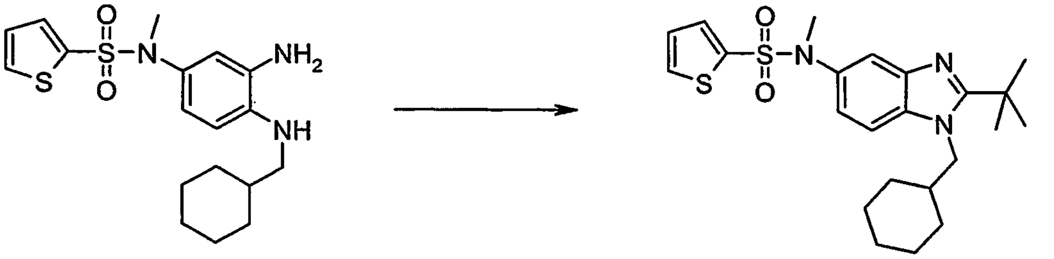

Following the procedure for Step A in Example 1, using *N*-{3-amino-4-[(cyclohexylmethyl) amino]phenyl} -*N*-methylthiophene-2-sulfonamide (115 mg, 0.303 mmol) (for preparation, see the following steps B, C and D) and trimethylacetyl chloride (0.041 mL, 0.333 mmol) in 3mL of DCE. The product was purified by reversed-phase HPLC using 20-80% $CH_3CN/H_2O$ and then lyophilized affording the title compound as the corresponding TFA salt. Yield: 65 mg (38%); $^1H$ NMR (400 MHz, METHANOL-$D_4$): δ1.22 (m, 5 H), 1.61 (m, 2 H), 1.64 (s, 9 H), 1.67 (m, 1 H), 1.74 (m, 2 H), 2.08 (m, 1 H), 3.29 (s, 3 H), 4.43 (d, J=7.62 Hz, 2 H), 7.15 (dd, J=5.08, 3.71 Hz, 1 H), 7.33 (dd, J=8.98, 1.95 Hz, 1 H), 7.40 (dd, J=3.71, 1.37 Hz, 1 H), 7.55 (d, J=1.56 Hz, 1 H), 7.81 (dd, J=5.08, 1.37 Hz, 1 H), 7.85 (d, J=8.98 Hz, 1 H); MS (ESI) $(M+H)^+$ 446.1; Anal. Calcd for $C_{23}H_{31}N_3O_2S_2$ + 1.5 TFA + 0.1 $H_2O$: C, 50.49; H, 5.33; N, 6.79. Found: C, 50.55; H, 5.39; N, 6.76.

**Step B. *N*-(4-Fluoro-3-nitrophenyl)-*N*-methylthiophene-2-sulfonamide**

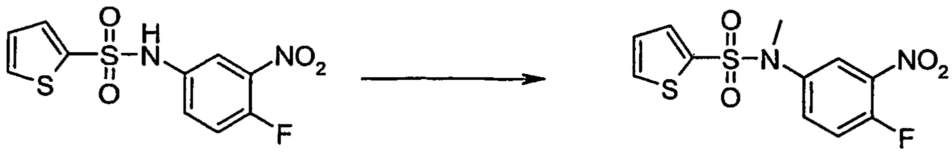

A solution of *N*-(4-fluoro-3-nitrophenyl)thiophene-2-sulfonamide (100 mg, 0.331 mmol) in MDF (1 mL) was added to a cold (0°C) stirring DMF solution (2 mL) of NaH (60% dispersion in oil) (20 mg, 0.496 mmol) under nitrogen. The solution was stirred at 0°C for 20 min. Methyl iodide (0.060 mL, 0.993 mmol) was added dropwise and the solution stirred at rt for 3h. The reaction mixture was quenched at 0°C by the slow addition of saturated $NH_4Cl$ solution. The solvent was evaporated *in vacuo.* The crude product was dissolved in EtOAc and washed with saturated $NaHCO_3$ aqueous solution, brine and dried over anhydrous $MgSO_4$. The product was purified by flash chromatography with dichloromethane as eluent on silica gel to afford the title product. Yield: 78 mg (74%); $^1H$ NMR (400 MHz, CHLOROFORM-D): δ 3.22 (s, 3H), 7.10 (dd, J = 4.98, 3.81Hz, 1H), 7.26 (dd, J= 3.91, 1.37Hz, 1H), 7.56 (m, 1H), 7.63 (dd, J = 5.08, 1.37Hz, 1H), 7.69 (dd, J = 6.44, 2.73Hz, 1H).

**Step C. *N*-{4-[(Cyclohexylmethyl)amino]-3-nitrophenyl}-*N*-methylthiophene-2-sulfonamide**

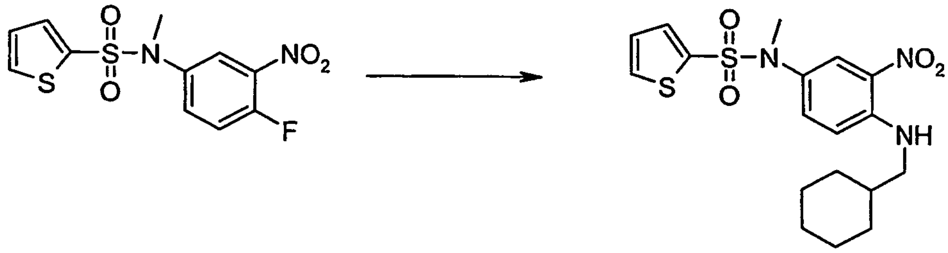

Following the procedure for Step C in Example 1, using *N*-(4-Fluoro-3-nitrophenyl)-*N*-methylthiophene-2-sulfonamide (3) (100 mg, 0.316 mmol), methylcyclohexylamine (0.050 mL, 0.379 mmol) and TEA (0.050mL, 0.474mmol) in 3mL of EtOH. The product was used directly for the next step without any column chromatography purification. Yield: 130 mg (99%); $^1$H NMR (400 MHz, CHLOROFORM-D): δ 0.98 (m, 1H), 1.04 (m, 1H), 1.20 (m, 1H), 1.66 (m, 2H), 1.75 (m, 2H), 1.81 (d, J = 12.50Hz, 2H), 3.12 (m, 2H), 3.16 (s, 3H), 6.80 (d, J = 9.37Hz, 1H), 7.09 (dd, J = 4.98, 3.81Hz, 1H), 7.37 (dd, J = 3.81, 1.27Hz, 1H), 7.42 (dd, J = 9.28, 2.64Hz, 1H), 7.59 (dd, J = 4.98, 1.27Hz, 1H), 7.65 (d, J = 2.54Hz, 1H), 8.20 (m, 1H).

**Step D. *N*-{3-Amino-4-[(cyclohexylmethyl)amino]phenyl}-*N*-methylthiophene-2-sulfonamide**

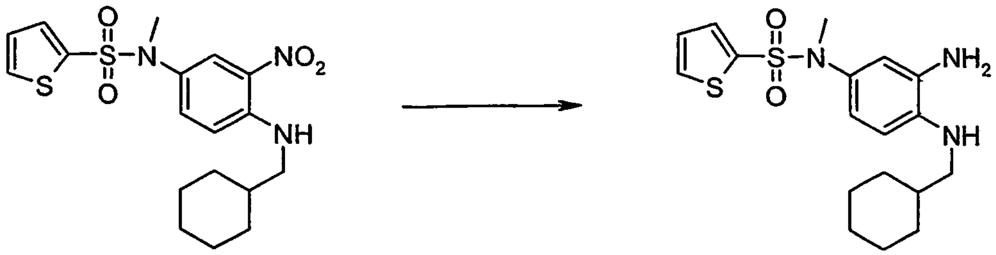

Following the procedure for Step D in Example 1, using *N*-{4-[(cylohexylmethyl)amino]-3-nitrophenyl}-*N*-methylthiophene-2-sulfonamide (125 mg, 0.305 mmol) and tin(II) chloride dihydrate (2X345 mg, 2X1.52 mmol). The product was used directly for Step A without further purification. Yield: 115 mg (99%); MS (ESI) $(M+H)^+$ 379.97.

**Example 3**

***N*-(1-Benzyl-2-*tert*-butyl-1*H*-benzimidazol-5-yl)-*N*-methylbenzenesulfonamide**

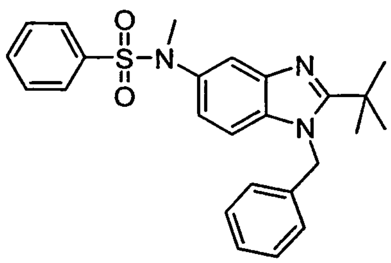

**Step A. *N*-(1-Benzyl-2-*tert*-butyl-1*H*-benzimidazol-5-yl)-*N*-methylbenzenesulfonamide**

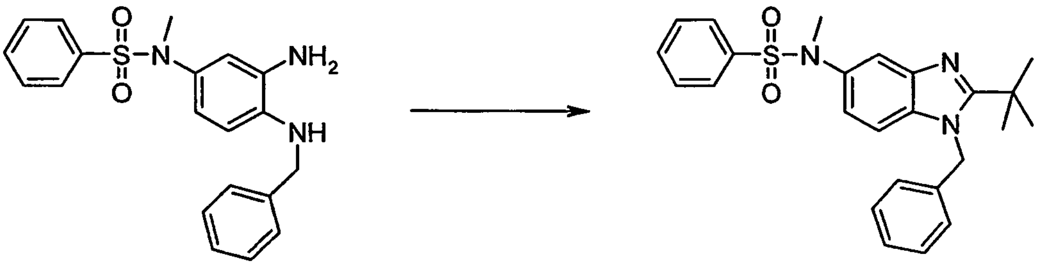

Following the procedure for Step A in Example 1, using *N*-{3-amino-4-[(cyclohexylmethyl)amino]phenyl} -*N*-methylbenzenesulfonamide (88mg, 0.239mmol), trimethylacetyl chloride (0.032mL, 0.262mmol) and DMAP (7.0mg, 0.060mmol) in 5 mL of dichloromethane. The product was purified by reversed-phase HPLC using 20-80% $CH_3CN/H_2O$ and then lyophilized affording the title compound as the corresponding TFA salt. Yield: 38 mg (29%); $^1H$ NMR (400 MHz, METHANOL-$D_4$): δ 1.62 (s, 9 H), 3.21 (s, 3 H), 5.93 (s, 2 H), 7.09 (m, 2 H), 7.12 (dd, J=8.98, 2.15 Hz, 1 H), 7.32 (m, 4 H), 7.48 (m, 4 H), 7.57 (dd, J=1.95, 0.59 Hz, 1 H), 7.62 (m, 1 H); MS (ESI) $(M+H)^+$ 434.1; Anal. Calcd for $C_{25}H_{27}N_3O_2S$ + 1.4 TFA + 0.2 $H_2O$: C, 55.95; H, 4.86; N, 7.04. Found: C, 55.90; H, 4.85; N, 7.06.

**Step B. *N*-(4-Fluoro-3-nitrophenyl)benzenesulfonamide**

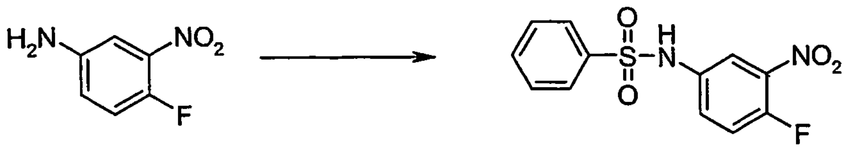

4-Fluoro-3-nitroaniline (2.00 g, 12.8 mmol) was dissolved in 50 mL of pyridine containing a catalytic amount of DMAP. Benzenesulfonyl chloride (1.96 mL, 15.36 mmol) was added and the solution was stirred at rt for 3h. The solvent was evaporated. The crude product was dissolved in EtOAc and washed with 5% $KHSO_4$ solution, saturated $NaHCO_3$ solution, brine and dried over anhydrous $MgSO_4$. The product was purified by flash chromatography using 2:1 / hexanes: EtOAc on silica gel. Yield: 3.40 g (90%); $^1H$ NMR (400 MHz, CHLOROFORM-D): δ 7.09 (m, 1H), 7.18 (dd, J= 9.96, 8.98 Hz, 1H), 7.40 (m, 1H), 7.48 (m, 2H), 7.58 (m, 1H), 7.71 (dd, J = 6.25, 2.73Hz, 1H), 7.76 (m, 2H).

**Step C. *N*-(4-Fluoro-3-nitrophenyl)-*N*-methylbenzenesulfonamide**

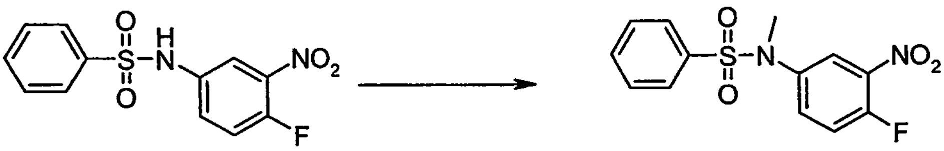

Following the procedure for Step B in Example 2, using *N*-(4-fluoro-3-nitrophenyl)benzenesulfonamide (1.00 g, 3.38 mmol), NaH (60% dispersion in oil) (160 mg, 4.06 mmol) and methyl iodide (0.315 mL, 5.07 mmol) in 25 mL of DMF. The product was purified by flash chromatography using dichloromethane as eluent on silica gel. Yield: 815 mg (78%); $^1H$ NMR (400 MHz, CHLOROFORM-D): δ 3.19 (s, 3H), 7.28 (m, 1H), 7.51 (m, 1H), 7.54 (m, 2H), 7.57 (m, 1H), 7.65 (m, 2H).

**Step D. *N*-[4-(Benzylamino)-3-nitrophenyl]-*N*-methylbenzenesulfonamide**

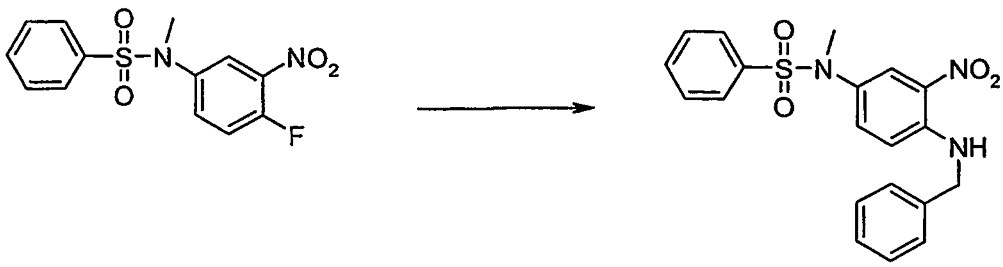

Following the procedure for Step C in Example 1, using *N*-(4-fluoro-3-nitrophenyl)-*N*-methylbenzene sulfonamide (71 mg, 0.229 mmol, benzylamine (0.030 mL, 0.275 mmol) and TEA (0.050mL, 0.344mmol)) in 3mL of EtOH. The product was used directly for the next step without further purification. Yield: 99 mg (99%); $^1$H NMR (400 MHz, CHLOROFORM-D): δ 3.12 (s, 3H), 4.56 (d, J = 5.47Hz, 2H), 6.81 (d, J = 9.18Hz, 1H), 7.34 (m, 3H), 7.40 (m, 3H), 7.49 (m, 2H), 7.57 (m, 1H), 7.60 (m, 1H), 7.64 (dd, J = 4.30, 1.95Hz, 1H), 8.47 (m, 1H).

**Step E. *N*-{3-Amino-4-[(cyclohexylmethyl)amino]phenyl}-*N*-methylbenzenesulfonamide**

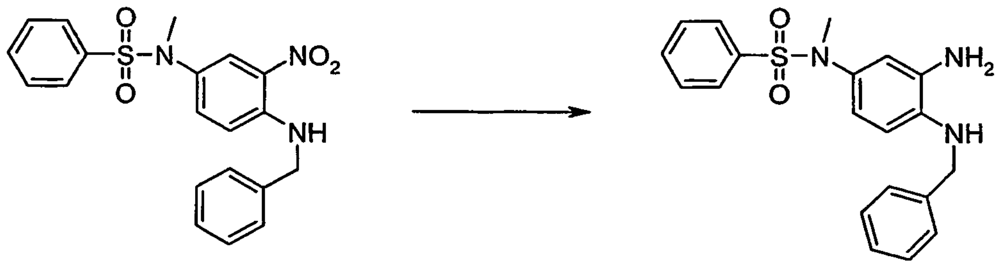

*N*-[4-(Benzylamino)-3-nitrophenyl]-*N*-methylbenzenesulfonamide (95 mg, 0.239 mmol) was dissolved in 15 mL of EtOAc containing a catalytic amount of 10% Pd/C. The solution was shaken in a Parr hydrogenation apparatus under $H_2$ atmosphere (40 psi) at rt for 4h. The solution was filtered through Celite and the solvent was evaporated. The product was used directly for Step A without further purification. Yield: 88 mg (99%); MS (ESI) $(M+H)^+$ 367.97.

**Example 4**

***N*-[2-*tert*-Butyl-1-(cyclohexylmethyl)-1*H*-benzimidazol-5-yl]-*N*,3,5-trimethylisoxazole-4-sulfonamide**

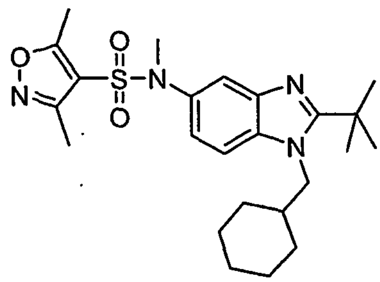

**Step A. *N*-[*2-tert*-Butyl-1-(cyclohexylmethyl)-1*H*-benzimidazol-5-yl]-*N*,3,5-trimethylisoxazole-4-sulfonamide**

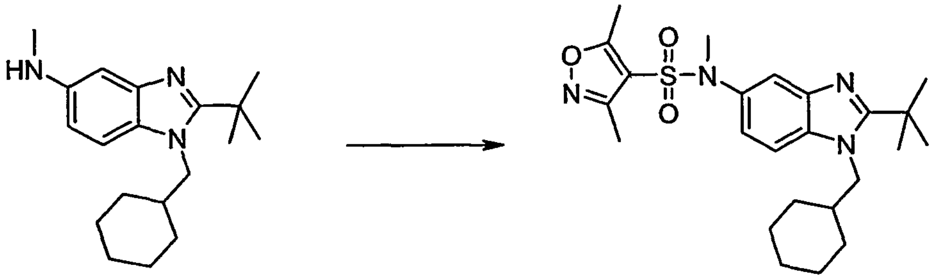

2-*tert*-Butyl-1-(cyclohexylmethyl)-*N*-methyl-1*H*-benzimidazol-5-amine (35 mg, 0.117 mmol) (for preparation, see the following steps B, C, D, E and F) and 3,5-dimethylisoxazole-4-sulfonyl chloride (0.030 mg, 0.140 mmol) were stirred in 3 mL of dichloromethane containing a catalytic amount of DMAP overnight at rt. The solvent was evaporated. The product was purified by reversed-phase HPLC using 20-80% $CH_3CN/H_2O$ and then lyophilized affording the title compound as the corresponding TFA salt. Yield: 49 mg (73%); $^1$HNMR (400 MHz, METHANOL-$D_4$): δ 1.21 (m, 5 H), 1.58 (m, 2 H), 1.65 (s, 10 H), 1.74 (m, 2 H), 1.88 (s, 3 H), 2.08 (m, 1 H), 2.33 (s, 3 H), 3.31 (s, 3 H), 4.46 (d, J=7.81 Hz, 2 H), 7.47 (dd, J=8.98, 1.95 Hz, 1 H), 7.68 (d, J=1.76 Hz, 1 H), 7.91 (d, J=8.98 Hz, 1 H); MS (ESI) $(M+H)^+$ 459.2; Anal. Calcd for $C_{24}H_{34}N_4O_3S$ + 1.6 TFA + 0.2 $H_2O$: C, 50.68; H, 5.63; N, 8.69. Found: C, 50.70; H, 5.65; N, 8.81.

**Step B. Methyl (4-fluoro-3-nitrophenyl)carbamate**

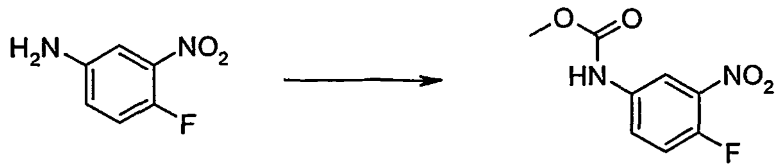

Methyl chloroformate (13.2 mL, 170.2 mmol) was added dropwise to a cold (0°C) dichloromethane (200 mL) solution of 4-fluoro-3-nitro aniline (24.15 g, 154.7 mmol) and DIPEA (35 mL, 201 mmol). The reaction mixture was stirred at rt overnight. The solution was then diluted with 200 mL of dichloromethane and washed with 2M HCl, brine and dried over anhydrous $MgSO_4$. The solvent was concentrated and the product was directly used for next step without further purification. Yield: 35.5 g (99%); $^1$H NMR (400 MHz, CHLOROFORM-D): δ 3.81 (s, 3 H), 7.02 (s, 1 H), 7.23 (m, 1 H), 7.72 (d, *J*=8.59 Hz, 1 H), 8.17 (dd, *J*=6.35, 2.64 Hz, 1 H).

**Step C. Methyl {4-[(cyclohexylmethyl)amino]-3-nitrophenyl}carbamate**

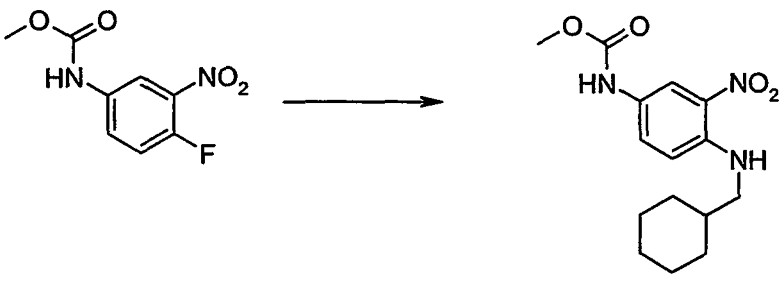

Methyl (4-fluoro-3-nitrophenyl)carbamate (1.00 g, 4.67 mmol) and cyclohexylmethyl amine (0.730 mL, 5.60 mmol) were stirred in EtOH (20 mL) containing TEA (1.0 mL, 7.00 mmol) at 75°C for 24h. The solvent was concentrated. The residue was dissolved in EtOAc and washed with 5% $KHSO_4$ solution, saturated $NaHCO_3$ solution, brine and dried over anhydrous $MgSO_4$. The crude product was purified by flash chromatography using 4:1/hex:EtOAc on silica gel. Yield: 1.05 g

(73%); $^1$H NMR (400 MHz, CHLOROFORM-D): δ1.04 (ddd, J = 24.02, 12.11, 2.93Hz, 2H), 1.25 (m, 3H), 1.69 (m, 2H), 1.76 (m, 1H), 1.79 (m, 1H), 1.83 (m, 1H), 1.86 (m, 1H), 3.14 (dd, J = 6.44, 5.66Hz, 2H), 3.78 (s, 3H), 6.46 (m, 1H), 6.84 (d, J = 9.37 Hz, 1H), 7.63 (m, 1H), 8.05 (d, J = 2.54 Hz, 1H), 8.09 (m, 1H).

**Step D. Methyl {3-amino-4-[(cyclohexylmethyl)amino]phenyl}carbamate**

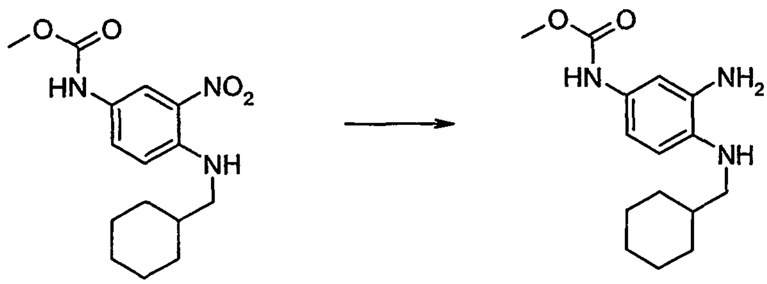

Methyl {4-[(cyclohexylmethyl)amino]-3-nitrophenyl}carbamate (1.05 g, 3.42 mmol) was dissolved in 30 mL of EtOAc containing a catalytic amount of 10% Pd/C. The solution was shaken in a Parr hydrogenation apparatus under $H_2$ atmosphere (40 psi) at rt overnight. The solution was filtered through Celite and the solvent was evaporated. The product was directly used for the next step without further purification. Yield: 950 mg (99%). MS (ESI) $(M+H)^+$ 277.9.

**Step E. Methyl [2-*tert*-butyl-1-(cyclohexylmethyl)-1*H*-benzimidazol-5-yl]carbamate**

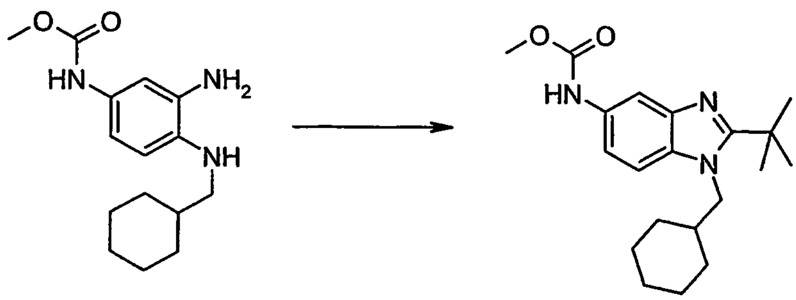

Methyl {3-amino-4-[(cyclohexylmethyl)amino]phenyl}carbamate (950 mg, 3.43 mmol) and DMAP (100 mg, 0.858 mmol) were dissolved in 25 mL of dichloromethane. Trimethylacetyl chloride (0.460 mL, 3.77 mmol) was added dropwise and the solution was stirred at rt for 1h. The solvent was concentrated. The residue was divided in two portions and each of them was dissolved in 3 mL of glacial AcOH in a sealed tube. The solutions were heated at 150°C using a Personal Chemistry Smith Synthesizer microwave instrument for three intervals of 30 min (3 X 30 min). The contents of the two tubes were combined and the solvent was evaporated. The residue was dissolved in EtOAc and washed with saturated $NaHCO_3$ solution, brine and dried over anhydrous $MgSO_4$. The crude product was purified by flash chromatography using 3:1/dichloromethane:diethyl ether. Yield: 656 mg (56%); $^1$H NMR (400 MHz, CHLOROFORM-D): δ 1.08 (m, 2H), 1.18 (m, 3H), 1.54 (s, 9H), 1.65 (m, 1H), 1.69 (m, 2H), 1.73 (dd, J = 5.96, 3.22 Hz, 2H), 2.02 (m, 1H), 3.78 (s, 3H), 4.10 (d, J = 7.42 Hz, 2H), 6.64 (m, 1H), 7.25 (d, J = 8.79 Hz, 1H), 7.39 (m, 1H), 7.59 (d, J = 1.76 Hz, 1H).

**Step F. 2-*tert*-Butyl-1-(cyclohexylmethyl)-*N*-methyl-1*H*-benzimidazol-5-amine**

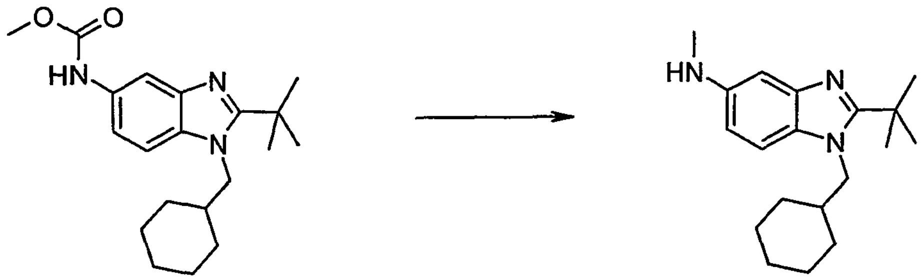

Methyl [2-*tert*-butyl-1-(cyclohexylmethyl)-1*H*-benzimidazol-5-yl]carbamate (650 mg, 1.89 mmol) was dissolved in 20 mL of HF at 0°C under nitrogen. 1M HCl/ether (2.65 mL, 2.65 mmol) was added dropwise and the solution was stirred at 0°C for 15min. $LiA1H_4$ (360 mg, 9.45 mmol) was then slowly added and the solution was stirred at rt overnight. The reaction mixture was quenched at 0°C by addition of MeOH (5 mL) followed by water (10 mL). The solution was diluted with EtOAc and washed with saturated $NaHCO_3$ solution, brine and dried over anhydrous $MgSO_4$. The solvent was evaporated and the product was used directly for Step A without further purification. Yield: 544 mg (96%). $^1$H NMR (400 MHz, CHLOROFORM-D): δ 1.08 (s, 2 H), 1.17 (m, 3 H), 1.54 (s, 9 H), 1.64 (m, 2 H), 1.67 (m, 2 H), 1.72 (m, 2 H), 2.02 (m, 1 H), 2.87 (s, 3 H), 4.06 (d, *J*=7.62 Hz, 2 H), 6.60 (dd, *J*=8.69, 2.25 Hz, 1 H), 7.00 (d, *J*=1.76 Hz, 1 H), 7.12 (d, *J*=8.59 Hz, 1 H).

**Example 5**

***N*-[2-*tert*-Butyl-1-(cyclohexylmethyl)-1*H*-benzimidazol-5-yl]-*N*,1,2-trimethyl-1*H***-imidazole-4-sulfonamide

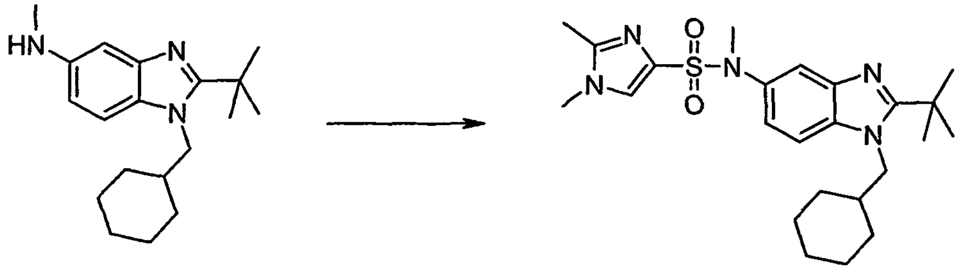

Following the procedure for Step A in Example 4, using 2-*tert*-butyl-1-(cyclohexylmethyl)-*N*-methyl-1*H*-benzimidazol-5-amine (36 mg, 0.120 mmol), 1,2-dimethyl-1*H*-imidazole-4-sulfonyl chloride (30 mg, 0.144 mmol) and DMAP (catalytic) in 3 mL of dichloromethane. The product was purified by reversed-phase HPLC using 20-80% $CH_3CN/H_2O$ and then lyophilized affording the title compound as the corresponding TFA salt. Yield: 47 mg (69%); $^1$H NMR (400 MHz, METH-ANOL-$D_4$): δ 1.22 (m, 5 H), 1.62 (m, 2 H), 1.64 (s, 9 H), 1.67 (m, 1 H), 1.75 (m, 2 H), 2.09 (m, 1 H), 2.34 (s, 3 H), 3.28 (s, 3 H), 3.61 (s, 3 H), 4.43 (d, J=7.62 Hz, 2 H), 7.43 (dd, J=8.98, 2.15 Hz, 1 H), 7.51 (s, 1 H), 7.66 (d, J=1.95 Hz, 1 H), 7.82 (d, J=9.18 Hz, 1 H); MS (ESI) $(M+H)^+$ 458.2; Anal. Calcd for $C_{24}H_{35}N_5O_2S$ + 1.7 TFA + 0.1 $H_2O$: C, 50.38; H, 5.69; N, 10.72. Found: C, 50.39; H, 5.73; N, 10.73.

**Example 6**

***N*-[2-*tert*-Butyl-1-(cyclohexylmethyl)-1*H*-benzimidazol-5-yl]-*N*,1,3,5-tetramethyl-1*H*-pyrazole-4-sulfonamide**

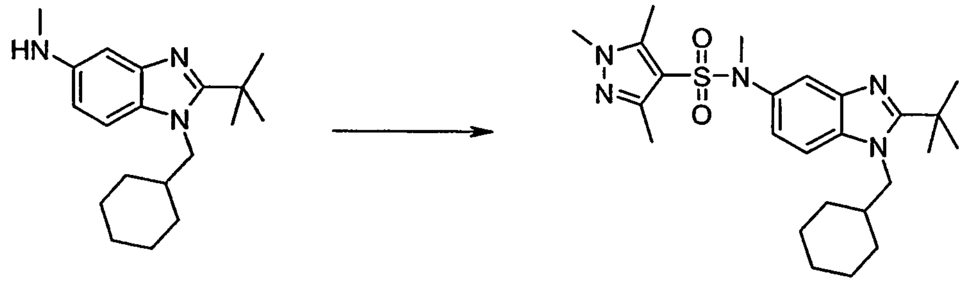

Following the procedure for Step A in Example 4, using *2-tert*-butyl-1-(cyclohexylmethyl)-*N*-methyl-1*H*-benzimidazol-5-amine (36 mg, 0.120 mmol), 1,3,5-trimethyl-1*H*-pyrazole-4-sulfonyl chloride (30 mg, 0.144 mmol) and DMAP (catalytic) in 3mL of dichloromethane. The product was purified by reversed-phase HPLC using 20-80% $CH_3CN/H_2O$ and then lyophilized affording the title compound as the corresponding TFA salt. Yield: 49 mg (70%); $^1$H NMR (400 MHz, METHANOL-$D_4$): δ 1.20 (m, 5 H), 1.56 (m, 2 H), 1.65 (s, 9 H), 1.67 (m, 1 H), 1.74 (m, 2 H), 1.80 (s, 3 H), 2.08 (m, 1 H), 2.16 (s, 3 H), 3.21 (s, 3 H), 3.69 (s, 3 H), 4.44 (d, J=7.81 Hz, 2 H), 7.38 (dd, J=9.08, 2.05 Hz, 1 H), 7.62 (d, J=1.56 Hz, 1 H), 7.84 (d, J=8.98 Hz, 1 H); MS (ESI) (N4+H)+ 472.2; Anal. Calcd for $C_{25}H_{37}N_5O_2S$ + 1.2 TFA + 0.4 $H_2O$: C, 53.45; H, 6.38; N, 11.37. Found: C, 53.50; H, 6.38; N, 11.29.

**Example 7**

***N*-[2-*tert*-butyl-1-(cyclohexylmethyl)-1*H*-benzimidazol-5-yl]benzene sulfonamide**

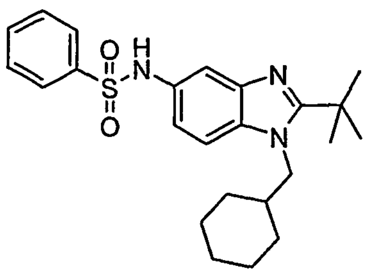

**Step A. *N*-[2-*tert*-butyl-1-(cyclohexylmethyl)-1*H*-benzimidazol-5-yl]benzene sulfonamide**

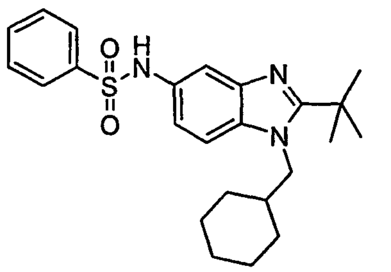

DMAP (44.0 mg, 0.36 mmol) and then trimethylacetyl chloride (199.0 mg, 1.65 mmol) at 0 °C was added into a solution of *N*-{3-amino-4-[(cyclohexylmethyl)amino]phenyl}benzene sulfonamide (540.1 mg, 1.5 mmol) (for preparation, see the following steps B, C and D) in dichloromethane (40 mL). The mixture was stirred for 5 h at room temperature. After evaporation of the solvent, the residue was dissolved in 1,2-dichloroethane (5x5 mL) in five Teflon-capped test tubes. The vessels were irradiated by microwave for 2h at 170 °C. The combined reaction mixture diluted with EtOAc (100

mL), washed with 2N NaOH(10 mL), sat. NaCl (10 mL) and dried over $Na_2SO_4$. After filtration and evaporation, the residue was purified by MPLC (hex/EtOAc 1:1 on silica gel) to give 568.2 mg (89%) of a white solid as the title compound. Part of the product was converted to TFA salt. $^1$HNMR (400 MHz, $CD_3OD$): δ 1.18 (m, 5 H), 1.55 (m, 2 H), 1.59 (s, 9 H), 1.65 (m, 1 H), 1.71 (m, 2 H), 2.03 (m, 1 H), 4.34 (d, J=7.42 Hz, 2 H), 7.20 (d, J=9.18 Hz, 1 H), 7.44 (m, 2 H), 7.53 (m, 1 H), 7.58 (s, 1 H), 7.70 (d, J=8.79 Hz, 1 H), 7.76 (d, J=7.42 Hz, 2 H). MS (ESI) $(M+H)^+$ = 426.1. Anal. Calcd for $C_{24}H_{31}N_3O_2S$+1.10 TFA+0.10 $H_2O$: C, 56.92; H, 5.89; N, 7.60. Found: C, 56.95; H, 5.92; N, 7.56.

**Step B. *N*-(4-fluoro-3-nitrophenyl)benzenesulfonamide**

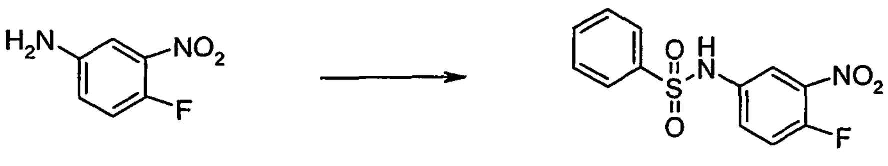

Benzenesulfonyl chloride (5.61 mL, 7.74 g, 44 mmol) was added into a solution of 4-fluoro-3-nitro-aniline (6.24 g, 40 mmol) in pyridine (40 mL) at 0 °C. The reaction mixture was stirred for two days at room temperature. Upon evaporation of the solvent, the residue was dissolved in EtOAc (500 mL), washed with water (50 mL), 2N HCl (2x50 mL), water (50 mL), sat. sodium bicarbonate aqueous solution (2x50 mL), sat. sodium chloride aqueous solution (2x50 mL) and dried over sodium sulphate. After filtration and concentration, 12.1 g (100%) of a brown solid was obtained as the title compound. $^1$HNMR (400 MHz, $CD_3Cl$): δ 7.20 (m, 2 H), 7.44 (m, 1 H), 7.52 (m, 2 H), 7.61 (m, 1 H), 7.75 (dd, *J*=6.25, 2.73 Hz, 1 H), 7.81 (m, 2 H).

**Step C: *N*-{4-[(cyclohexylmethyl)amino]-3-nitrophenyl}benzenesulfonamide**

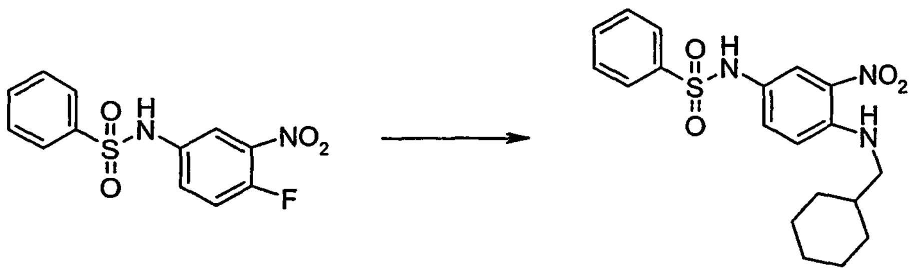

Cyclohexylmethylamine (3.48 mL, 3.03 g, 26.7 mmol) was added to a mixture of *N*-(4-fluoro-3-nitrophenyl)benzenesulfonamide (3.60 g, 12.1 mmol) in 60 mL of EtOH-$H_2O$ (1:1 V/V) at room temperature. The reaction mixture was heated for 48 h at 60 °C, and allowed to cool to room temperature and concentrated to a small volume, and then extracted with EtOAc. The crude product was purified by MPLC using Hex/EtOAc (4:1) on silica gel to give 3.75 g (79%) of an orang-red solid as the title compound. $^1$HNMR (400 MHz, $CD_3Cl$): δ 1.05 (m, 2 H), 1.24 (m, 3 H), 1.72 (m, 6 H), 3.12 (dd, *J*=6.64, 5.47 Hz, 2 H), 6.23 (s, 1 H), 6.79 (d, *J*=9.18 Hz, 1 H), 7.36 (dd, *J*=9.08, 2.64 Hz, 1 H), 7.48 (m, 2 H), 7.58 (m, 1 H), 7.63 (d, *J*=2.54 Hz, 1 H), 7.72 (m, 2 H), 8.14 (s, 1 H).

**Step D: *N*-{3-amino-4-[(cyclohexylmethyl)amino]phenyl}benzenesulfonamide**

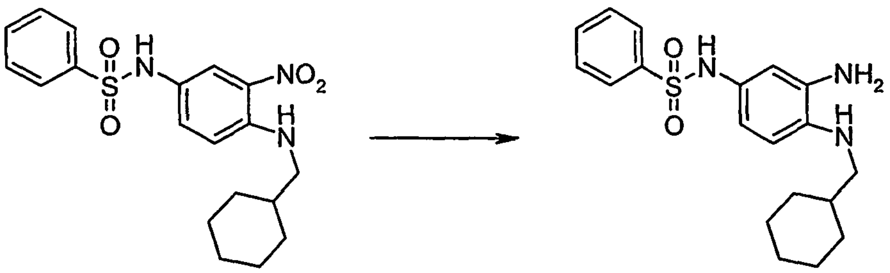

*N*-{4-[(cylohexylmethyl)amino]-3-nitrophenyl}benzenesulfonamide (3.75 g, 9.63 mmol) was hydrogenated in ethyl acetate (200 mL) catalyzed by 10% Pd/C (0.5 g) at 30-40 psi $H_2$ in Parr shaker for 20 h at room temperature. After filtration through Celite and concentration, 4.0 g (100%) of a light yellow solid was obtained as the title compound, which was used for Step A without further purification. $^1$HNMR (400 MHz, $CD_3Cl$): δ 0.88 (m, 1 H), 0.99 (m, 2 H), 1.23 (m, 3 H), 1.56 (m, 1 H), 1.75 (m, 4 H), 2.86 (d, *J*=6.64 Hz, 2 H), 3.33 (s broad, 3 H), 6.30 (s broad, 1 H), 6.33 (dd, *J=8.30,* 2.44 Hz, 1 H), 6.41 (m, 1 H), 6.56 (d, *J*=234 Hz, 1 H), 7.41 (m, 2 H) 7.52 (m, 1 H), 7.70 (m, 2 H). MS (ESI) $(M-H)^+$: 359.89.

**Example 8**

***N*-[1-(cyclohexylmethyl)-2-ethyl-1*H*-benzimidazol-5-yl]benzenesulfonamide**

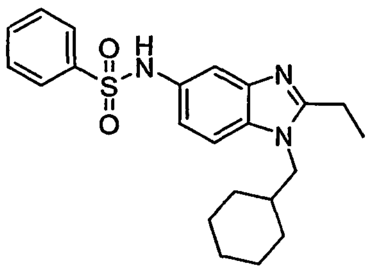

DMAP (15.0 mg, 0.12 mmol) and then propionyll chloride (50.9 mg, 0.55 mmol) was added into a solution of *N*-{3-amino-4-[(cyclohexylmethyl)amino]phenyl}benzene sulfonamide (180.3 mg, 0.5 mmol) in dichloromethane (15 mL) at 0 °C. The mixture was stirred for 5 h at room temperature. After evaporation of the solvent, the residue was dissolved in acetic acid (10 mL) and then heated for 20 h at 80°C. Upon concentration, the residue diluted with EtOAc (100 mL), washed with 2N NaOH(10 mL), sat. NaCl (10 mL) and dried over $Na_2SO_4$. The crude product was purified by MPLC (hexane/EtOAc 1:9 on silica gel) to give 157.7 mg (79%) of a white solid as the title compound. Part of the product was converted to TFA salt. $^1$HNMR (400 MHz, $CD_3OD$): δ 1.12 (m, 2 H), 1.21 (m, 3 H), 1.48 (t, J=7.62 Hz, 3 H), 1.60 (m, 2 H), 1.68 (m, 1 H), 1.73 (m, 2 H), 1.90 (m, 1 H), 3.18 (q, J=7.49 Hz, 2 H), 4.19 (d, J=7.62 Hz, 2 H), 7.24 (m, 1 H), 7.47 (m, 2 H), 7.56 (m, 2 H), 7.71 (d, J=8.98 Hz, 1 H), 7.79 (m, 2 H). MS (ESI) $(M+H)^+$= 398.1.

**Example 9**

***N*-[1-(cyclohexylmethyl)-2-isopropyl-1*H*-benzimidazol-5-yl]benzene sulfonamide**

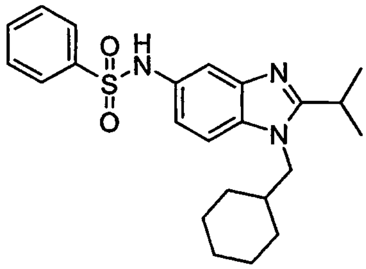

Following the procedure for Example 8, using *N*- {3-amino-4-[(cyclohexylmethyl)amino]phenyl}benzene sulfonamide (182.1 mg, 0.5 mmol), DMAP (15.0 mg, 0.12 mmol) and isobutyryl chloride (50.2 mg, 0.56 mmol) $CH_2Cl_2$ (15 mL). The crude product was purified by MPLC (Hex/EtOAc 1:1). Yield: 178.4 mg (86%). $^1$HNMR (400 MHz, $CD_3OD$): δ 1.17 (m, 5 H), 1.47 (d, J=6.83 Hz, 6 H), 1.60 (m, 2 H), 1.72 (m, 3 H), 1.88 (m, 1 H), 3.62 (m, 1 H), 4.25 (d, J=7.62 Hz, 2 H), 7.25 (dd, J=8.88, 2.05 Hz, 1 H), 7.48 (m, 2 H), 7.57 (m, 2 H), 7.73 (d, J=8.98 Hz, 1 H), 7.80 (m, 2 H). MS (ESI) $(M+H)^+$ = 412.1.

**Example 10**

***N*-[1-(cyclohexylmethyl)-2-(1-methylcyclopropyl)-1*H*-benzimidazol-5-yl]benzenesulfonamide**

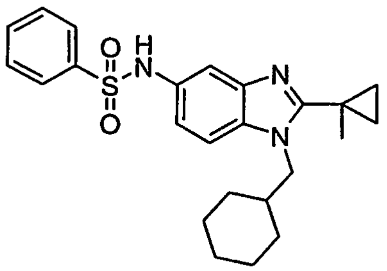

Diisopropylethylamine (104.7 mg, 0.81 mmol) was added into a solution of *N*-{3-amino-4-[(cyclohexylmethyl)amino] phenyl}benzene sulfonamide (195.6 mg, 0.543 mmol), and 1-methylcyclopropanecarboxylic acid (59.8 mg, 0.6 mmol) in DMF (5 mL) at 0 °C. Stirring for 20 min. HATU (246.4 mg, 0.65 mmol) was added. The reaction mixture was stirred for 24 h at room temperature, diluted with water (100 mL), and extracted with EtOAc (2x50 mL). The combined organic phases were washed with NaCl (20 mL) and dried with anhydrous sodium sulphate. After filtration and concentration, the residue was dissolved in acetic acid (10 mL) and heated for 20 h at 80 °C. Upon evaporation of the solvent, 93.7 mg of the acetate salt was lyophilized, and the rest was diluted with EtOAc (100 mL), washed with 2N NaOH(10 mL), sat. NaCl (2x10 mL) and dried over anhydrous sodium sulphate. After filtration and evaporation, 144.9 mg of the free amine as the title compound was obtained. Total yield: 99%. $^1$HNMR (400 MHz, $CD_3OD$): δ 0.89 (m, 2 H), 1.10 (m, 4 H), 1.21 (m, 3 H), 1.45 (s, 3 H), 1.55 (d, 2 H), 1.69 (m, 3H), 2.08 (m, 1 H), 4.13 (d, J=7.62 Hz, 2 H), 7.01 (dd, J=8.79, 1.95 Hz, 1 H,) 7.25 (d, J=1.95 Hz, 1 H), 7.35 (d, J=8.79 Hz, 1 H), 7.40 (t, J=7.62 Hz, 2 H), 7.50 (t, J=7.42 Hz, 1 H), 7.68 (d, J=7.42 Hz, 2 H). MS (ESI) $(M+H)^+$ = 424.1. Anal. Calcd for $C_{24}H_{29}N_3O_2S$+0.4 AcOH+0.10 $H_2O$: C, 65.92; H, 6.98; N, 9.45. Found: C, 66.01; H, 6.89; N, 9.09.

**Example 11**

***N*-[1-(cyclohexylmethyl)-2-(1,1-dimethylpropyl)-1*H*-benzimidazol-5-yl]-benzenesulfonamide**

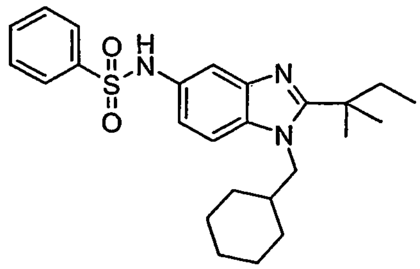

Following the procedure in Example 10, using *N*-{3-amino-4-[(cyclohexylmethyl)amino]phenyl}benzene sulfonamide (180.3 mg, 0.50 mmol), 2,2-dimethylbutyric acid (63.9 mg, 0.55 mmol), diisopropylethylamine (96.6 mg, 0.75 mmol) and HATU (228.1 mg, 0.60 mmol) in DMF (5 mL) and then in acetic acid (10 mL), the crude product was purified by reversed HPLC using 30-80% $CH_3CN/H_2O$ to give 39.9 mg (14%) of a white solid as the title compound. $^1$HNMR (400 MHz, $CD_3OD$): δ 0.81 (t, J=7.52 Hz, 3 H), 1.20 (m, 5 H), 1.58 (m, 2 H), 1.62 (s, 6 H), 1.68 (m, 1 H), 1.75 (m, 2 H), 1.97 (q, J=7.62 Hz, 2 H), 2.03 (m, 1 H), 4.37 (d, J=7.62 Hz, 2 H), 7.23 (dd, J=8.98, 1.95 Hz, 1 H), 7.47 (m, 2 H), 7.56 (m, 1 H), 7.59 (m, 1 H), 7.73 (d, J=8.98 Hz, 1 H), 7.80 (m, 2 H). MS (ESI) $(M+H)^+$ = 440.2. Anal. Calcd for $C_{25}H_{33}N_3O_2S$+1.0 TFA+0.60 $H_2O$: C, 57.45; H, 6.29; N, 7.44. Found: C, 57.49; H, 6.39; N, 7.35.

**Example 12**

***N*-[1-(cyclohexylmethyl)-2-(1,1-dimethyl-3-butenyl)-1*H*-benzimidazol-5-yl]-benzenesulfonamide**

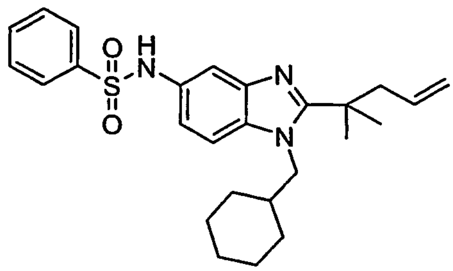

Following the procedure in Example 10, using *N*-{3-amino-4-[(cylohexylmethyl)amino]phenyl)benzene sulfonamide (180.3 mg, 0.50 mmol), 2,2-dimethyl-4-pentenoic acid (70.5 mg, 0.55 mmol), diisopropylethylamine (96.6 mg, 0.75 mmol) and HATU (228.1 mg, 0.60 mmol)) in DMF (5 mL) and then in acetic acid (10 mL), the crude product was purified by by reversed HPLC using 30-80% $CH_3CN/H_2O$ to give 38.2 mg (14%) of a white solid as the title compound. $^1$HNMR (400MHz, $CD_3OD$): δ 1.19 (m, 5 H), 1.59 (m, 2 H), 1.65 (s, 6 H),1.68 (m, 1 H,) 1.74 (m, 2 H), 2.04 (m, 1 H), 2.67 (d, J=7.42 Hz, 2 H), 4.40 (d, J=7.62 Hz, 2 H), 5.03 (s, 1 H), 5.07 (m, 1 H), 5.60 (m, 1 H), 7.23 (dd, J=8.98, 2.15 Hz, 1 H), 7.47 (m, 2 H), 7.56 (m, 1 H), 7.59 (d, J=1.56 Hz, 1 H), 7.74 (d, J=8.98 Hz, 1 H), 7.80 (m, 2 H). MS (ESI) $(M+H)^+$ = 452.2. Anal. Calcd for $C_{26}H_{33}N_3O_2S$+1.2 TFA: C, 57.97; H, 5.86; N, 7.14. Found: C, 58.00; H, 5.74; N, 7.06.

**Example 13**

***N*-[1-(cyclohexylmethyl)-2-(1,1-dimethylethyl)-1*H*-benzimidazol-5-yl)]-*N*-methylbenzenesulfonamide**

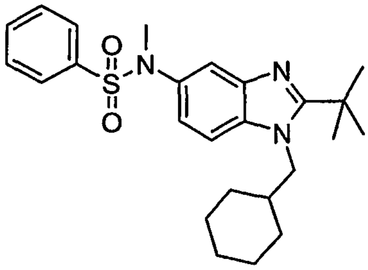

NaH (54.0 mg, 60%, 1.34 mmol) was added to a solution of of *N*-[2-*tert*-butyl-1-(cylohexymethyl)-1*H*-benzhnidazol-5-yl]benzene sulfonamide (258.5 mg, 0.607 mmol) in THF (15 mL) at 0 °C. After stirring for 1h, MeI (259.8 mg, 1.83 mmol) was added. The mixture was stirred overnight at room temperature, quenched with sat. $NaHCO_3$ (5 mL). The two phases were separated. The aqueous was extracted with EtOAc (3x20 ml). The combined organic phases were washed with $NaHCO_3$ (2x10 mL) and dried with $Na_2SO_4$. After concentration, the residue was purified by MPLC using hex/EtOAc (1: 1) on silica gel to give 216.5 mg (81%) of the title product, and converted to TFA salt as a white solid. $^1$HNMR (400 MHz, $CD_3OD$): δ 1.20 (m, 5 H), 1.60 (m, 2 H), 1.64 (s, 9 H), 1.67 (m, 1 H), 1.75 (m, 2 H), 2.07 (m, 1 H), 3.24 (s, 3 H), 4.42 (d, J=7.62 Hz, 2 H), 7.26 (dd, J=8.98,2.15 Hz, 1 H), 7.50 (m, 5 H), 7.65 (m, 1 H), 7.81 (d, J=9.18 Hz, 1 H). MS (ESI) $(M+H)^+$ = 440.2. Anal. Calcd for $C_{25}H_{33}N_3O_2S$+1.20 TFA: C, 57.09; H, 5.98; N, 7.29. Found: C, 57.07; H, 6.01; N, 7.25.

**Example 14**

***N*-[1-(cyclohexylmethyl)-2-ethyl-1*H*-benzimidazol-5-yl]-*N*-methyl-benzene sulfonamide**

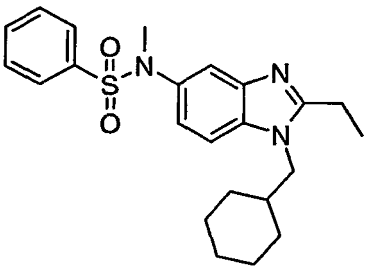

Following the procedure in Example 13, using *N*-[1-(cyclohexylmethyl)-2-ethyl-1*H*-benzimidazol-5-yl]benzenesulfonamide (91.3 mg, 0.23 mmol), sodium hydride (28.2 mg, 60%, 0.71 mmol) and iodomethane (97.9 mg, 0.69 mmol) in THF (10 mL). Yield: 69.7 mg (74%); white solid for TFA salt. $^1$HNMR (400 MHz, $CD_3OD$): δ 1.20 (m, 5 H), 1.51 (t, J=7.52 Hz, 3 H), 1.65 (m, 2 H), 1.70 (m, 1 H), 1.76 (m, 2 H), 1.95 (m, 1 H), 3.22 (q, J=7.62 Hz, 2 H), 3.27 (s, 3 H), 4.26 (d, J=7.62 Hz, 2 H), 7.29 (dd, J=8.88, 2.05 Hz, 1 H), 7.50 (d, J=1.95 Hz, 1 H), 7.54 (m, 4 H), 7.67 (m, 1 H), 7.80 (d, J=8.98 Hz, 1 H). MS (ESI) $(M+H)^+$ = 412.1

**Example 15**

***N*-[1-(cyclohexylmethyl)-2-isopropyl-1*H*-benzimidazol-5-yl]-*N*-methyl-benzene sulfonamide**

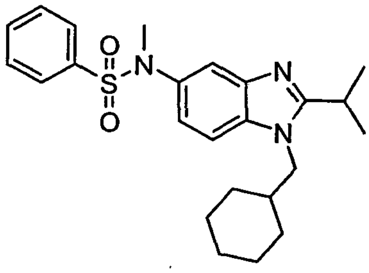

Following the procedure in Example 13, using *N*-[1-(cyclohexymethyl)-2-isoproyl-1*H*-benzimidazol-5-yl]benzenesulfonamide (81.8 mg, 0.199 mmol), sodium hydride (24.0 mg, 60%, 0.596 mmol) and iodomethane (84.7 mg, 0.597 mmol) in THF (15 mL). Yield: 80.1 mg (95%); The title compound was converted to white solid as a TFA salt. $^1$HNMR (400 MHz, $CD_3OD$): δ 1.20 (m, 5 H), 1.51 (d, J=6.83 Hz, 6 H), 1.64 (m, 2 H), 1.69 (m, 1 H), 1.76 (m, 2 H), 1.93 (m, 1 H), 3.27 (s, 3 H), 3.67 (m, 1 H), 4.31 (d, J=7.62 Hz, 2 H), 7.28 (m, 1 H), 7.54 (m, 5 H), 7.68 (m, 1 H), 7.81 (d, J=8.98 Hz, 1 H). MS (ESI) $(M+H)^+$ = 426.1.

**Example 16**

***N*-[1-(cyclohexylmethyl)-2-(1-methylcyclopropyl)-1*H*-benzimidazol-5-yl]-*N*-methyl-benzenesulfonamide**

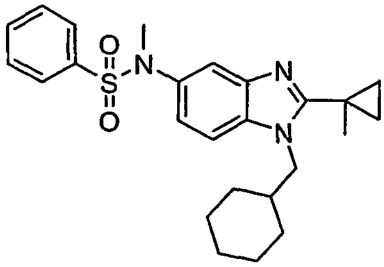

Following the procedure in Example 13, using *N*-[1-(cyclohexylmethyl)-2-(1-methylcyclopropyl)-1*H*-benzimidazol-5-yl] benzenesulfonamide (144.9 mg, 0.342 mmol), sodium hydride (30.0 mg, 60%, 0.752 mmol) and iodomethane (145.7 mg, 1.03 mmol) in THF (15 mL). Yield: 72.3 mg (48%); compound was converted to a white solid as a TFA salt. $^1$HNNR (400 MHz, $CD_3OD$): δ 1.19 (m, 4 H), 1.26 (m, 3 H), 1.34 (m, 2 H), 1.59 (s, 3 H), 1.71 (m, 5 H), 2.19 (m, 1 H), 3.26 (s, 3 H), 4.38 (d, J=7.81 Hz, 2 H), 7.29 (dd, J=9.08, 2.05 Hz, 1 H), 7.50 (m, 1 H), 7.52 (m, 1 H) 7.55 (m, 3 H), 7.67 (m, 1 H), 7.83 (d, J=8.98 Hz, 1 H). MS (ESI) $(M+H)^+$ = 438.2. Anal. Calcd for $C_{25}H_{31}N_3O_2S$+1.2 TFA+0.10 $H_2O$: C, 57.11; H, 5.67; N, 7.29. Found: C, 57.19; H, 5.74; N, 7.22.

**Example 17**

***N*-[2-(1,1-dimethylethyl)-1-[(tetrahydro-2*H*-pyran-4-yl)methyl]-1*H*-benzimidazol-5-yl]-benzenesulfonamide**

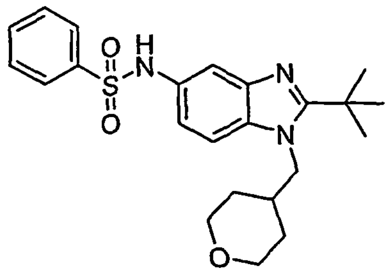

**Step A. *N*-[2-(1,1-dimethylethyl)-1-[(tetrahydro-2*H*-pyran-4-yl)methyl]-1*H*-benzimidazol-5-yl]-benzenesulfonamide**

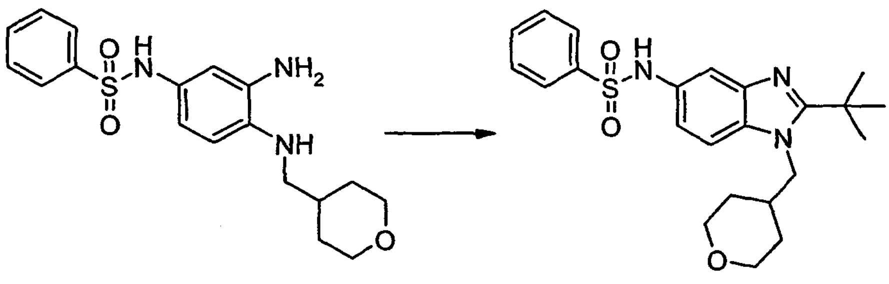

Catalytic DMAP in one portion and pivaloyl chloride (0.26 mL, 1.1 eq) were added dropwise sequentially to a stirring solution of *N*-[3-amino-4-[[(tetrahydro-2*H*-pyran-4-yl)methyl]amino] phenyl]-benzenesulfonamide (705.2 mg, 1.95 mmol) (for preparation, see Steps B, C and D) in $CH_2Cl_2$ (100 mL) at 0°C. The solution was stirred for 2 hours, then the solvent was concentrated, and the residue was re-dissolved in AcOH (2 mL). The resulting solution was heated at 150°C for 1800 s using microwave irradiation and the solvent was then concentrated. The residue was re-dissolved in EtOAc, washed with 1N NaOH (2X) and brine and dried over anhydrous $\mathbf{Na_2SO_4}$. Purification by MPLC using EtOAc followed by reversed-phase HPLC using 10-90% MeCN in $H_2O$ afforded the title compound as a colourless solid (16.3 mg, 2% yield); $^1$H NMR (400 MHz, $CD_3OD$) δ 1.46-1.55 (m, 4 H), 1.62 (s, 9 H), 2.25-2.35 (m, 1 H), 3.30-3.34 (m, 2 H), 3.88-3.92 (m, 2 H), 4.43 (d, *J*=7.42 Hz, 2 H), 7.22 (dd, *J*=9.08, 2.05 Hz, 1 H), 7.43-7.47 (m, 2 H), 7.52-7.56 (m, 1 H), 7.60 (d, *J*=2.05 Hz, 1 H), 7.75-7.80 (m, 3 H); MS (ESI) $(M+H)^+$ = 428.0.

**Step B. *N*-(4-fluoro-3-nitrophenyl)-benzenesulfonamide**

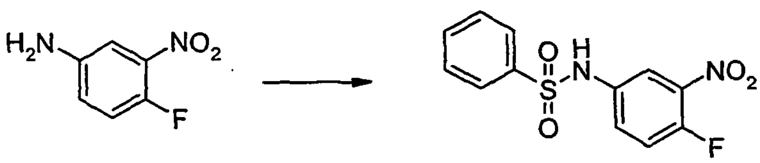

Catalytic DMAP in one portion and benzene sulfonyl chloride (9.82 mL, 1.2 eq) dropwise were added sequentially to a stirring solution of 4-fluoro-3-nitroaniline (10 g, 64.1 mmol) in pyridine (52 mL) at 0°C. The solution was stirred at 0°C for 30 minutes and then warmed gradually to room temperature. After 3 hours at room temperature, the solution was diluted with EtOAc (15 mL) and was washed with water until the organic layer contained no pyridine by TLC ($CH_2Cl_2$). The solution was dried with anhydrous $Na_2SO_4$. Purification by MPLC using $CH_2Cl_2$ afforded the title compound as a pale yellow solid (13.6 g, 71% yield); $^1$H NMR (400 MHz, $CDCl_3$): δ 7.21 (dd, *J*=10.15, 8.98 Hz, 1 H), 7.32-7.36 (brs, 1 H), 7.44 (m, 1 H), 7.51 1 (m, 2 H), 7.62 (m, 1 H), 7.77 (dd, *J*=6.25, 2.93 Hz, 1 H), 7.82 (m, 2 H).

**Step C. *N*-[3-nitro-4-[[(tetrahydro-2*H*-pyran-4-yl)methyl]amino]phenyl]-benzenesulfonamide**

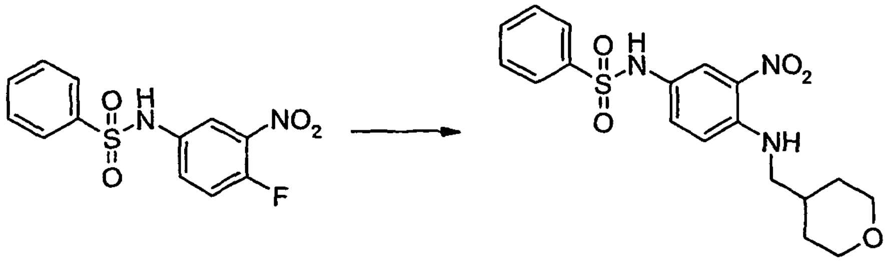

4-Tetrahydropyranmethylamine (468 mg, 1.2 eq) in EtOH (0.5 mL) was added to a stirring solution of *N*-(4-fluoro-3-nitrophenyl)- benzenesulfonamide (1.0 g, 3.38 mmol) and triethylamine (0.47 mL, 1 eq) in EtOH:$H_2O$ 4:1 (15 mL) at room temperature. The solution was heated at 60°C overnight, then cooled to room temperature. The cooled solution was poured into $H_2O$ and was extracted (3X) with EtOAc. The combined organic phases were washed with brine and dried over anhydrous $Na_2SO_4$. The crude product was purified by MPLC using 1:1 heptane:EtOAc to afford the title compound as a bright red solid (767.5 mg, 58%); $^1$H NMR (400 MHz, $CDCl_3$): δ 1.41-1.46 (m, 2 H), 1.65-1.75 (m, 2 H), 1.90-1.98 (m, 1 H), 3.18-3.21 (m, 2 H), 3.39-3.45 (m, 2 H), 4.00-4.04 (m, 2 H), 6.61 (s, 1 H), 6.80 (d, *J*=9.18 Hz, 1 H), 7.38 (dd, *J*=9.28, 2.64 Hz, 1 H), 7.46-7.50 (m, 2 H), 7.56-7.60 (m, 1 H), 7.69 (d, *J*=2.54 Hz, 1 H), 7.73-7.75 (m, 2 H), 8.10 (m, 1 H).

**Step D. *N*-[3-amino-4-[[(tetrahydro-2*H*-pyran-yl)methyl]amino]phenyl]-benzenesulfonamide**

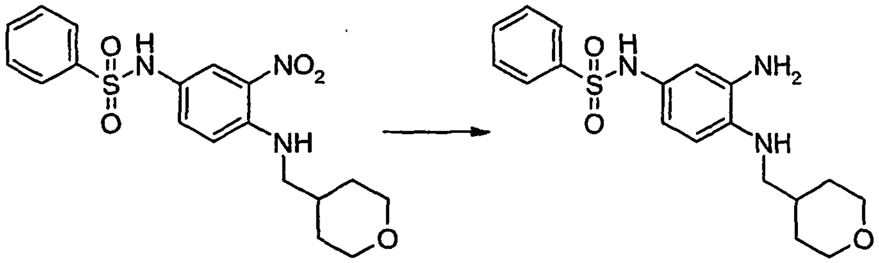

Following the procedure for Step E in Example 3, using *N*-[3-nitro-4-[[(tetrahydro-2*H*-pyran-4-yl)methyl]amino]phenyl]-benzenesulfonamide (767.5 mg, 1.96 mmol) and a catalytic amount of 10% Pd/C in EtOAc (50 mL). LC/MS analysis indicated that the compound was of sufficient purity (>95%) to be used directly for Step A. Yield: 705.2 mg, 100%; $^1$HNMR (400 MHz, $CDCl_3$) δ1.33-1.44 (m, 2 H), 1.61-1.73 (m, 2 H), 1.81-1.88 (m, 1 H), 2.95 (d, *J*=6.64 Hz, 2 H), 3.37-3.43 (m, 5 H), 3.99 (dd, *J*=10.84, 3.42 Hz, 2 H), 6.27-6.31 (br. s., 1 H), 6.34-6.36 (m, 1H), 6.42-6.44 (m, 1 H), 6.58 (d, *J*=2.34 Hz, 1 H), 7.40-7.44 (m, 2 H), 7.50-7.55 (m, 1 H), 7.70-7.74 (m, 1 H).

**Example 18**

***N*-[2-(1,1-dimethylethyl)-1-[(tetrahydro-2-furanyl)methyl]-1*H*-benzimidazol-5-yl]-benzenesulfonamide**

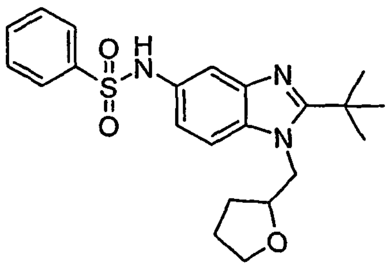

**Step A. *N*-[2-(1,1-dimethylethyl)-1-[(tetrahydro-2-furanyl)methyl]-1*H* benzimidazol-5-yl]-benzenesulfonamide**

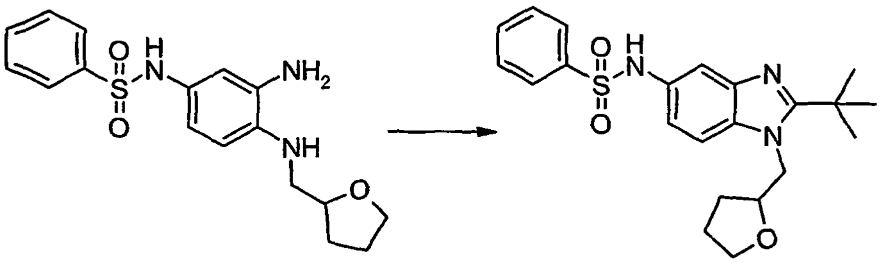

Following the procedure for Step A in Example 17, using *N*-[3-amino-4-[[(tetrahydro-2-furanyl)methyl]amino] phenyl]-benzenesulfonamide (597.2 mg, 1.72 mmol) (for preparation, see the following steps B and C), $CH_2Cl_2$ (30 mL), catalytic DMAP and pivaloyl chloride (0.23 mL, 1.1 eq), followed by AcOH (3 mL), the crude product was purified by MPLC using 4:1 EtOAc:hexanes, converted to the corresponding TFA salt and lyophilized. Yield of the title product as TFA salt: 182.3 mg (17%); $^1$H NMR (400 MHz, $CD_3OD$) δ 1.60 (s, 9 H), 1.76-1.79 (m, 1 H), 1.89-2.04 (m, 2 H), 2.18-2.26 (m, 1 H), 3.64-3.69 (m, 1 H), 3.84-3.90 (m, 1 H), 4.28-4.34 (m, 1 H), 4.53-4.59 (m, 1 H), 4.66-4.71 (m, 1 H), 7.21 (dd, *J*=8.98, 1.95 Hz, 1 H), 7.43-7.47 (m, 2 H), 7.52-7.56 (m, 1 H), 7.59 (d, *J*=1.95 Hz, 1 H), 7.75-7.79 (m, 3 H); MS (ESI) $(M+H)^+$= 414.0.

**Step B. *N*-[3-nitro-4-[[(tetrahydro-2-furanyl)methyl]amino]phenyl]-benzenesulfonamide**

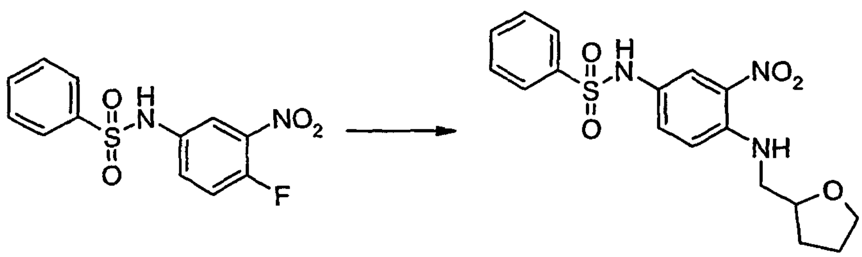

Following the procedure for Step C in Example 20, using *N*-(4-fluoro-3-nitrophenyl)-benzenesulfonamide (1.0 g, 3.38 mmol), tetrahydrofurylamine (0.42 mL, 1.2 eq), EtOH (12 mL) and $H_2O$ (3 mL), the crude product was purified by MPLC using 1:1 heptane:EtOAc to afford the title compound as a bright red solid (749.7 mg, 59%); $^1$H NMR (400 MHz, $CDCl_3$): δ 1.62-1.73 (m, 1 H), 1.94-2.13 (m, 3 H), 3.27-3.47 (m, 2 H), 3.80-3.98 (m, 2 H), 4.15-4.22 (m, 1 H), 6.51 (s, 1 H), 6.81 (d, *J*=9.18 Hz, 1 H), 7.35 (dd, *J*=8.98, 2.54 Hz, 1 H), 7.44-7.48 (m, 2 H), 7.55-7.59 (m, 1 H), 7.63 (d, *J*=2.73 Hz, 1 H), 7.69-7.72 (m, 2 H), 8.18-8.20 (m, 1 H).

**Step C. *N*-[3-amino-4-[[(tetrahydro-2-furanyl)methyl]amino]phenyl]-benzenesulfonamide**

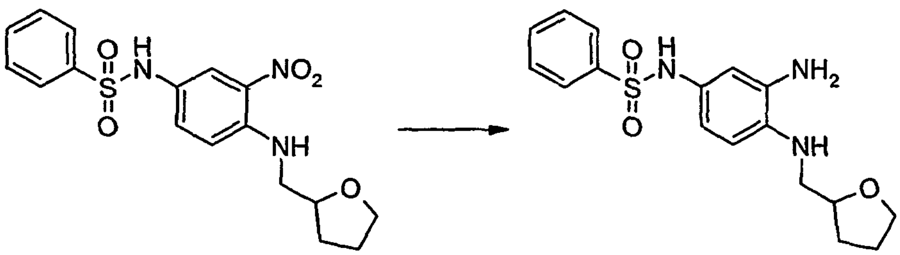

Following the procedure for Step E in Example 3, using *N*-[3-nitro-4-[[(tetrahydro-2-furanyl)methyl]amino] phenyl]-benzenesulfonamide (749.7 mg, 2 mmol) and a catalytic amount of 10% Pd/C in EtOAc (50 mL), LC/MS analysis indicated that the title compound was of sufficient purity (>95%) to be used directly for the next step. Yield: 597.2 mg, 86%;$^1$HNMR (400 MHz, $CDCl_3$): δ 1.61-1.70 (m, 2 H), 1.89-1.96 (m, 2 H), 2.01-2.09(m, 1 H), 2.97-3.02 (m,1 H), 3.12-3.16 (m, 1 H), 3.34-3.59 (m, 3 H), 3.74-3.90 (m, 2 H), 6.28 (s, 1 H), 6.33 (dd, *J*=8.20, 2.34 Hz, 1 H), 6.44 (d, *J*=8.40 Hz, 1 H), 6.53 (d, *J*=2.34 Hz, 1 H), 7.39-7.44 (m, 2 H), 7.50-7.54 (m, 1 H), 7.68-7.72 (m, 2 H).

**Example 19**

**N-[1-(cyclobutylmethyl)-2-(1,1-dimethylethyl)-1*H*-benzimidazol-5-yl]-benzenesulfonamide**

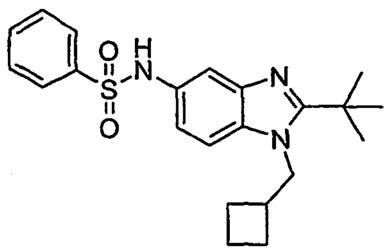

**Step A. *N*-[1-(cyclobutylmethyl)-2-(1,1-dimethylethyl)-1*H*-benzimidazol-5-yl]-benzenesulfonamide**

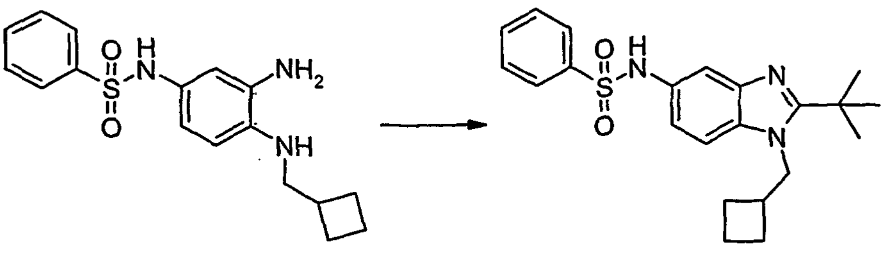

Following the procedure for Step A in Example 17, using *N*-[3-amino-4-[(cyclobutylmethyl)amino]phenyl]-benzenesulfonamide (763.8 mg, 2.30 mmol) (for preparation, see the following steps B and C), $CH_2Cl_2$ (40 mL), catalytic DMAP and pivaloyl chloride (0.31 mL, 1.1 eq), followed by AcOH (9 mL), the crude product was purified by MPLC using 1:1 hexanes:EtOAc, followed by reversed-phase HPLC using 20-65% MeCN in $H_2O$. The compound was lyophilized to afford the title compound as its TFA salt (226.8 mg, 19%);$^1$H NMR (400 MHz, $CD_3OD$): δ 1.60 (s, 9 H), 1.81-1.93 (m, 2 H), 2.01-2.07 (m, 4 H), 2.78-2.83 (m, 1 H), 4.55 (d, *J*=6.44 Hz, 2 H, 7.22 (dd, *J*=8.98, 1.95 Hz, 1 H), 7.43-7.47 (m, 2 H),

7.52-7.56 (m, 1 H), 7.59 (dd, *J*=1.95, 0.59 Hz, 1 H), 7.70 (dd, *J*=8.98, 0.59 Hz, 1 H), 7.76-7.79 (m, 2 H); MS (ESI) $(M+H)^+$ = 398.0.

**Step B. *N*-[4-[(cyclobutylmethyl)amino]-3-nitrophenyl]-benzenesulfonamide**

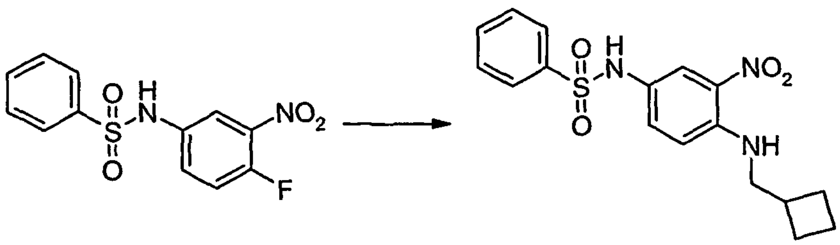

Following the procedure for Step C in Example 17, using *N*-(4-fluoro-3-nitrophenyl)-benzenesulfonamide (1.0 g, 3.38 mmol), cyclobutylmethylamine (345.7 mg, 1.2 eq), EtOH (12 mL) and $H_2O$ (5 mL) The crude product was purified by MPLC using 7:3 hexanes:EtOAc to afford the title compound as a bright orange solid (857.7 mg, 70%); $^1H$ NMR (400 MHz, $CDCl_3$): δ 1.73-1.78 (m, 2 H), 1.88-2.00 (m, 2 H), 2.13-2.21 (m, 2 H), 2.62-2.73 (m, 1 H), 3.27-3.30 (m, 2 H), 6.44 (s, 1 H), 6.78 (d, *J*=9.18 Hz, 1 H), 7.36 (dd, J=9.08, 2.44 Hz, 1 H), 7.45-7.49 (m, 2 H), 7.55-7.60 (m, 1H), 7.66 (d, *J*=2.73 Hz, 1 H) 7.72-7.74 (m, 2 H), 7.93-7.98 (br s, 1 H).

**Step C. *N*-[3-amino-4-[(cyclobutylmethyl)amino]phenyl]-benzenesulfonamide**

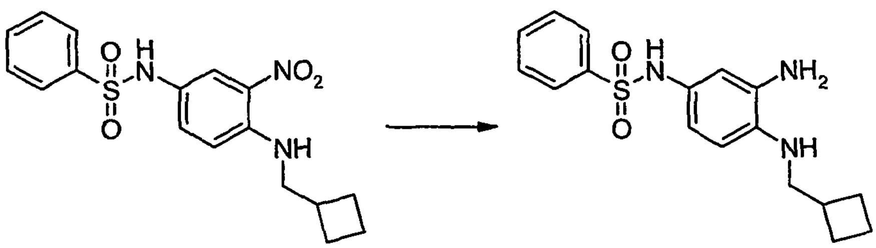

Following the procedure for Step E in Example 3, using *N*-[4-[(cyclobutylmethyl)amino]-3-nitrophenyl]-benzenesulfonamide (857.7 mg, 2.37 mmol) and a catalytic amount of 10% Pd/C in EtOAc (25 mL) and shaking for 48 h, LC/MS analysis indicated that the title compound was of sufficient purity (>95%) to be used directly for the next step. Yield: 763.8 mg, 97%;$^1$HNMR (400 MHz, $CDCl_3$): δ 1.68-1.78 (m, 2 H), 1.86-1.98 (m, 2 H), 2.08-2.16 (m, 2 H), 2.54-2.62 (m, 1 H), 3.04 (d, *J*=7.42 Hz, 2 H), 3.11-3.42 (brs, 2 H), 6.28-3.31 (br. s., 1 H), 6.33 (dd, *J*=8.40, 2.34 Hz, 1 H), 6.43 (d, *J*=8.40 Hz, 1 H), 6.56 (d, *J*=2.34 Hz, 1 H), 7.40-7.44 (m, 2 H), 7.50-7.54 (m, 1 H), 7.69-7.71 (m, 2 H).

**Example 20**

***N*-[1-(cyclopropylmethyl)-2-(1,1-dimethylethyl)-1*H*-benzimidazol-5-yl]-benzenesulfonamide**

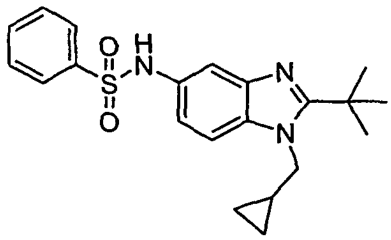

**Step A. *N*-[1-(cyclopropylmethyl)-2-(1,1-dimethylethyl)-1*H*-benzimidazol-5-yl]-benzenesulfonamide**

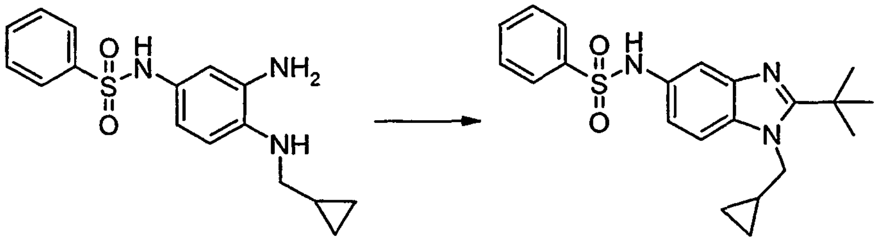

Following the procedure for Step A in Example 17, using *N*-[3-amino-4-[(cyclopropylmethyl)amino]phenyl]-benzenesulfonamide (736.4 mg, 2.32 mmol) (for preparation, see the following steps B and C), $CH_2Cl_2$ (50 mL), catalytic DMAP and pivaloyl chloride (0.31 mL, 1.1 eq), followed by AcOH (10 mL), the crude product was purified by reversed-phase HPLC using 15-45% MeCN in $H_2O$. The compound was lyophilized to afford the title compound as its TFA salt (50 mg, 4.2%); $^1H$ NMR (400 MHz, $CD_3OD$): δ 0.57-0.61 (m, 2 H), 0.65-0.70 (m, 2 H), 1.22-1.25 (m, 1 H), 1.62 (s, 9 H), 4.47 (d, *J*=6.44 Hz, 2 H), 7.23 (dd, *J*=9.08, 2.05 Hz, 1 H), 7.43-7.47 (m, 2 H), 7.52-7.55 (m, 1 H), 7.62 (d, *J=1.95* Hz, 1 H), 7.74 (d, *J*=8.98 Hz, 1 H), 7.73-7.78 (m, 2 H); MS (ESI) $(M+H)^+$ = 384.0.

**Step B. *N*-[4-[(cyclopropylmethyl)amino]-3-nitrophenyl]-benzenesulfonamide**

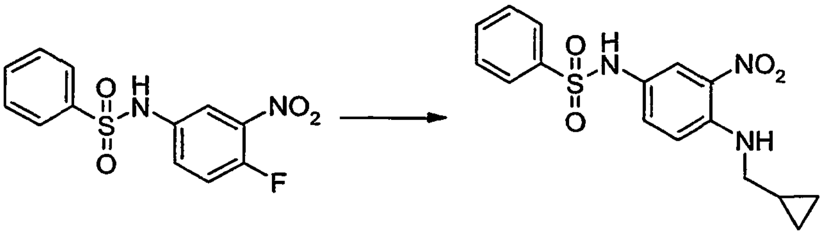

Following the procedure for Step C in Example 17, using *N*-(4-fluoro-3-nitrophenyl)-benzenesulfonamide (1.0 g, 3.38 mmol) and cyclopropylmethylamine (0.4 mL, 1.2 eq) in EtOH (12 mL) and $H_2O$ (5 mL). The crude product was purified by MPLC using 7:3 hexanes:EtOAc on silical gel to afford the title compound as a bright orange solid (828.3 mg, 71%); $^1H$ NMR (400 MHz, $CDCl_3$): δ 0.29-0.33 (m, 2 H), 0.63-0.67 (m, 2 H), 1.14-1.19 (m, 1 H), 3.13 (dd, *J*=7.03, 4.88 Hz, 2 H), 6.42 (s, 1 H), 6.76 (d, *J*=9.37 Hz, 1 H), 7.36 (dd, *J*=9.18, 2.54 Hz, 1 H), 7.45-7.49 (m, 2 H), 7.56-7.59 (m, 1 H), 7.67 (d, *J*=2.54 Hz, 1 H), 7.71-7.74 (m, 2 H), 8.07-8.13 (m, 1 H).

**Step C. *N*-[3-amino-4-[(cyclopropylmethyl)amino]phenyl]-benzenesulfonamide**

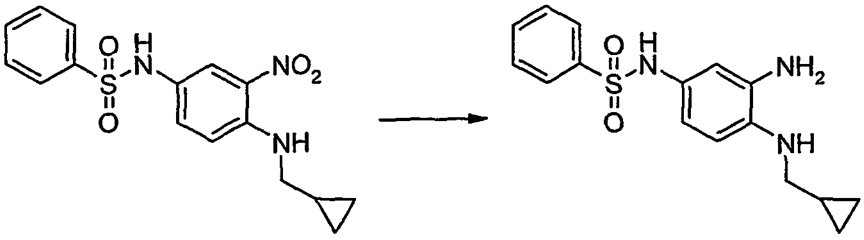

Following the procedure for Step E in Example 3, using *N*-[4-[(cyclopropylmethyl)amino]-3-nitrophenyl]-benzenesulfonamide (828.3 mg, 2.38 mmol), a catalytic amount of 10% Pd/C in EtOAc (30 mL). LC/MS analysis indicated that the title compound was of sufficient purity (>95%) to be used directly for the next step. Yield: 736.4 mg, 100%; $^1$H NMR (400 MHz, $CDCl_3$): δ 0.20-0.24 (m, 2 H), 0.53-0.58 (m, 2 H), 1.08-1.12 (m, 1 H), 2.87 (d, *J*=7.03 Hz, 2 H), 3.28-3.48 (br. s., 2 H), 6.27-6.31 (br. s., 1 H), 6.31-6.34 (m, 1 H), 6.39-6.41 (m, 1 H), 6.57 (d, *J*=2.34 Hz, 1 H), 7.40-7.44 (m, 2 H), 7.49-7.54 (m, 1 H), 7.69-7.71 (m, 2 H).

**Example 21**

***N*-[2-*tert*-Butyl-1-(tetrahydro-2*H*-pyran-4-ylmethyl)-1*H*-benzimidazol-5-yl]-*N*-methylbenzenesulfonamide**

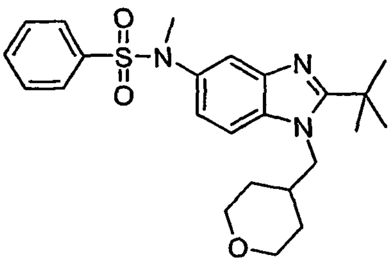

**Step A. *N*-[2-*tert*-Butyl-1-(tetrahydro-2*H*-pyran-4-ylmethyl)-1*H*-benzimidazol-5 yl]-*N*-methylbenzenesulfonamide**

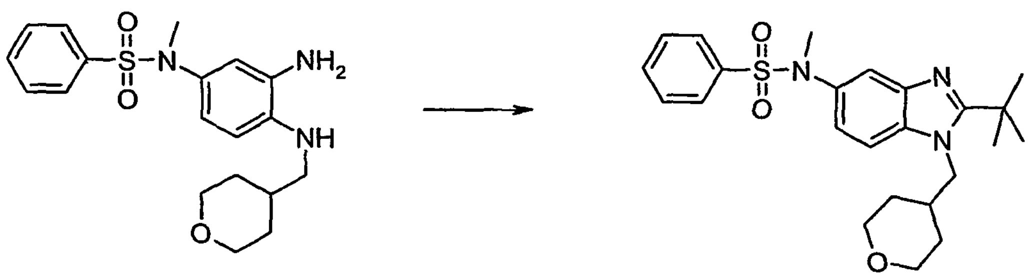

Following the procedure for Step E in Example 4, using *N*-{3-amino-4-[(tetrahydro-2*H*-pyran-4-ylmethyl)amino]phenyl}-*N*-methylbenzenesulfonamide (109 mg, 0.290 mmol) (for preparation, see the following steps B and C), trimethylacetyl chloride (0.039 mL, 0.319 mmol) and DMAP (7 mg, 0.058 mmol) in 3 mL of DCM. The second step was performed in 2 mL of glacial acetic acid. The final product was purified by reversed-phase HPLC using 20-80% $CH_3CN/H_2O$ and then lyophilized affording the title compound as the corresponding TFA salt. Yield: 60 mg (37%); $^1$H NMR (400 MHz, METHANOL-$D_4$): δ 1.55 (m, 2H), 1.60 (m, 2H), 1.68 (s, 9H), 2.37 (m, 1H), 3.27 (s, 3H), 3.36 (ddd, J =11.57, 2.64Hz, 2H), 3.93 (d, J=3.52Hz, 1H), 3.96 (m, 1H), 4.53 (d, J=7.42Hz, 2H), 7.31 (dd, J = 8.98, 1.95Hz, 1H), 7.54 (m, 5H), 7.68 (m, 1H), 7.89 (d, J = 8.98Hz, 1H); MS (ESI) $(M+H)^+$ 442.3.

**Step B. *N*-Methyl-*N*-{3-nitro-4-[(tetrahydro-2*H*-pyran-4-ylmethyl)amino]phenyl}benzenesulfonamide**

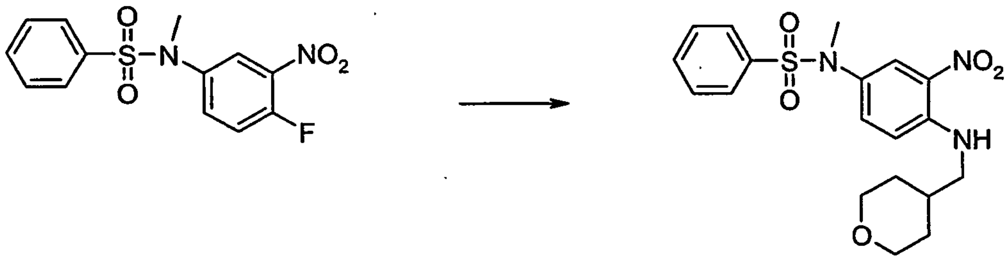

Following the procedure for Step C in Example 1, using *N*-(4-fluoro-3-nitrophenyl)-*N*-methylbenzene sulfonamide (100 mg, 0.322 mmol) (for preparation, see Example 3, Step B and C), 4-aminomethyltetrahydopyran (45 mg, 0.386 mmol) and TEA (0.070 mL, 0.483 mmol) in 3 mL of EtOH. The residue was dissolved in EtOAc and washed with 5% $KHSO_4$ solution, saturated $NaHCO_3$ solution, brine and dried over anhydrous $MgSO_4$. The crude product was purified by silica gel flash chromatography using a linear gradient of 30-50% EtOAc / hexanes. Yield: 123 mg (94%); $^1$H NMR (400 MHz, CHLOROFORM-D): δ 1.44 (m, 2H), 1.74 (m, 1H), 1.77 (m, 1H), 1.96 (m, 1H), 3.13 (s, 3H), 3.23 (dd, J = 6.74, 5.57Hz, 2H), 3.43 (ddd, J = 11.81, 2.15, 2H), 4.40 (dd, J = 11.13, 3.91Hz, 2H), 6.84 (d, J = 9.18Hz, 1H), 7.49 (m, 3H), 7.61 (m, 3H), 8.21 (m, 1H).

**Step C. *N*-{3-Amino-4-[(tetrahydro-2*H*-pyran-4-ylmethyl)amino]phenyl}-*N*-methylbenzenesulfonamide**

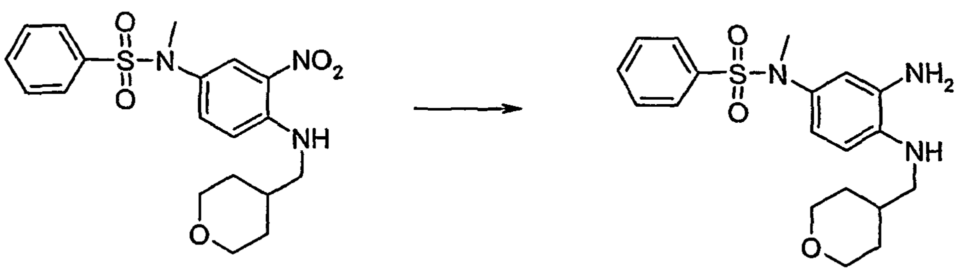

Following the procedure for Step E in Example 3, using *N*-methyl-*N*-{3-nitro-4-[(tetrahydro-2*H*-pyran-4-ylmethyl)amino] phenyl}benzenesulfonamide (118 mg, 0.291 mmol) and a catalytic amount of 10% Pd/C in 20 mL of EtOAc. Yield: 109 mg (99%); MS (ESI) $(M+H)^+$ 376.16.

**Example 22**

***N*-[*2-tert*-Butyl-1-(tetrahydro-2*H*-pyran-2-ylmethyl)-1*H*-benzinnidazol-5-yl]-*N* methylbenzenesulfonamide**

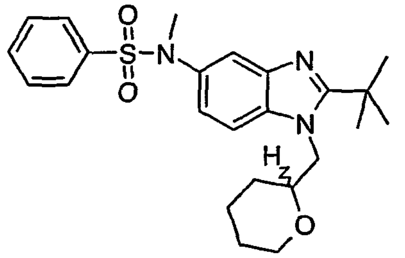

**Step A. *N*-[2-*tert*-Butyl-1-(tetrahydro-2*H*-pyran-2-ylmethyl)-1*H*-benzimidazol-5-yl] -*N*-methylbenzenesulfonamide**

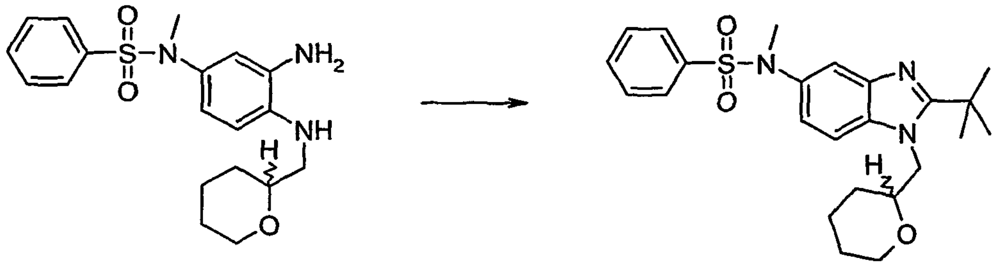

Following the procedure for Step E in Example 4, using *N*-{3-Amino-4-[(tetrahydro-2*H*-pyran-2-ylmethyl)amino]phenyl}-*N*-methylbenzenesulfonamide (47 mg, 0.125 mmol) (for preparation, see the following steps B and C), trimethylacetyl chloride (0.017 mL, 0.138 mmol) and DMAP (3 mg, 0.025 mmol) in 3 mL of DCM. The second step was performed in 2 mL of glacial acetic acid. The final product was purified by reversed-phase HPLC using 20-80% $CH_3CN/H_2O$ and then lyophilized affording the title compound as the corresponding TFA salt. Yield: 33 mg (48%); $^1$H NMR (400 MHz, METHANOL-$D_4$): δ 1.50 (m, 2H), 1.58 (m, 3H), 1.67 (s, 9H), 1.88 (m, 1H), 1.91 (m, 1H), 3.22 (ddd, J = 11.47, 2.64Hz, 1H), 3.27 (s, 3H), 3.85 (m, 2H), 4.65 (m, 2H), 7.29 (dd, J = 9.08, 2.05Hz, 1H), 7.51 (d, J = 1.56 Hz, 1H), 7.54 (m, 3H), 7.68 (m, 1H), 7.87 (d, J = 8.98Hz, 1H); MS (ESI) $(M+H)^+$ = 442.3. Anal. Calcd for $C_{24}H_{31}N_3O_3S$+1.5 TFA + 0.1 $H_2O$: C, 52.78; H, 5.36; N, 6.84. Found: C, 52.83; H, 5.37; N, 6.90.

**Step B. *N*-Methyl-*N*-{3-nitro-4-[(tetrahydro-2*H*-pyran-2-ylmethyl)amino]phenyl}benzenesulfonamide**

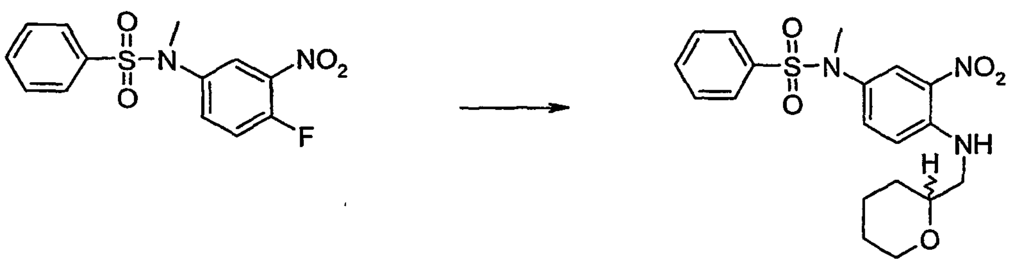

Following the procedure for Step C in Example 1, using *N*-(4-fluoro-3-nitrophenyl)-*N*-methylbenzene sulfonamide (50 mg, 0.161 mmol), 2-aminomethyltetrahydopyran hydrochloride (30 mg, 0.193 mmol) and TEA (0.056 mL, 0.403 mmol) in 3 mL of EtOH. The residue was dissolved in EtOAc and washed with 5% $KHSO_4$ solution, saturated $NaHCO_3$ solution, brine and dried over anhydrous $MgSO_4$. The crude product was purified by silica gel flash chromatography using first DCM and then EtOAc as eluent. Yield: 58 mg (89%); $^1$H NMR (400 MHz, CHLOROFORM-D): δ 1.46 (m, 1H), 1.55 (d, J = 7.03Hz, 2H), 1.60 (m, 2H), 1.67 (m, 1H),1.91 (m, 1H), 3.13 (s, 3H), 3.29 (m, 1H), 3.37 (m, 1H), 3.50 (ddd, J =11.33, 2.93Hz, 1H), 3.62 (m, 1H), 4.06 (m, 1H), 6.83 (d, J = 9.37Hz, 1H), 7.46 (dd, J = 9.37, 2.54Hz, 1H), 7.50 (m, 2H), 7.57

(m, 2H), 7.61 (m, 1H), 8.33 (m, 1H).

**Step C. *N*-{3-Amino-4-[(tetrahydro-2*H*-pyran-2-ylmethyl)amino]phenyl}-*N*-methylbenzenesulfonamide**

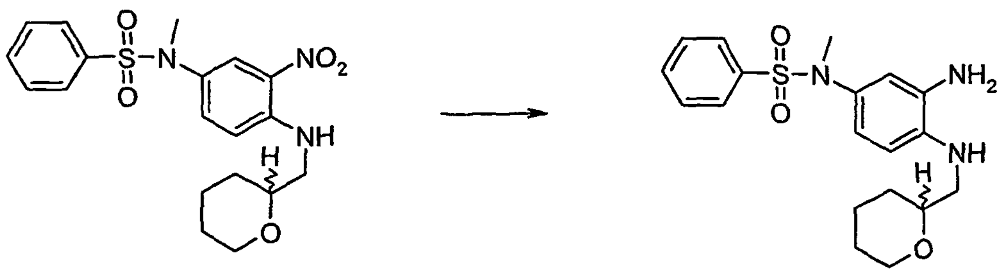

Following the procedure for Step E in Example 3, using *N*-methyl-*N*-{3-nitro-4-[(tetrahydro-2*H*-pyran-2-ylmethyl)amino] phenyl}benzenesulfonamide (55 mg, 0.136 mmol) and a catalytic amount of 10% Pd/C in 15 mL of EtOAc. Yield: 47 mg (92%); MS (ESI) $(M+H)^+$ 376.17.

**Example 23**

***N*-[1-(cyclohexylmethyl)-2-(1-hydroxy-1-methylethyl)-1*H*-benzimidazol-5-yl]-benzenesulfonamide**

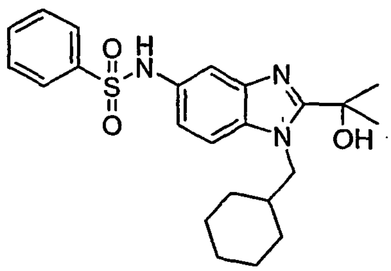

Diisopropylethylamine (291.4 mg, 2.25 mmol) was added into a solution of *N*-{3-amino-4-[(cyclohexylmethyl)amino] phenyl}benzene sulfonamide (540.1 mg,1.50 mmol) (for preparation, see Steps B, C and D in Example 7) and 2-hydroxy isobutyric acid (171.8 mg, 1.65 mmol) in DMF (15 mL) at 0 °C. Stirring for 20 min., HATU (684.4 mg, 1.80 mmol) was added. The reaction mixture was stirred for 24 h at room temperature, diluted with water (100 mL), and extracted with EtOAc (2x50 mL). The combined organic phases were washed with NaCl (20 mL) and dried with anhydrous sodium sulphate. After filtration and concentration, the residue was dissolved in acetic acid (5 mL) in a sealed tube. The solutions were heated at 140°C using a Personal Chemistry Smith Synthesizer microwave instrument for 30 min. Upon evaporation of the solvent, the residue was diluted with EtOAc (100 mL), washed with 2N NaOH(10 mL), sat. NaCl (2x10 mL) and dried over anhydrous sodium sulphate. After filtration and evaporation, the residue was purified by MPLC (EtOAc on silica gel) to give 364.6 mg (57%) of a white solid as the title compound. Part of the product was converted to TFA salt. $^1H$ NMR (400 MHz, $CD_3OD$): δ1.17 (m, 5 H), 1.56 (m, 2 H), 1.70 (m, 3 H), 1.76 (s, 6 H), 2.09 (m, 1 H), 4.48 (d, J=7.62 Hz, 2 H), 7.23 (m, 1 H), 7.47 (m, 2 H), 7.56 (m, 2 H), 7.72 (d, J=8.98 Hz, 1 H), 7.79 (m, 2 H); MS (ESI) $(M+H)^+$ = 428.0; Anal. Calcd for $C_{23}H_{29}N_3O_3S$+1.2TFA: C, 54.05; H, 5.39; N, 7.45. Found: C, 54.09; H, 5.50; N, 7.42.

**Example 24**

***N*-[1-(cyclohexylmethyl)-2-(1-methoxy-1-methylethyl)-1*H*-benzimidazol-5-yl]-*N*-methyl-benzenesulfonamide**

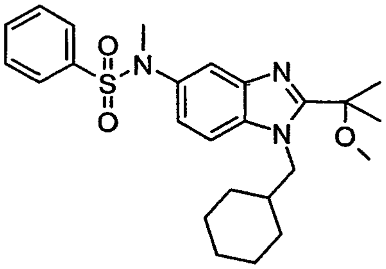

Sodium hydride (31.2 mg, 0.78 mmol) was added into a solution of *N*-[1-(cyclohexylmethyl)-2-(1-hydroxy-1-methylethyl)-1*H*-benzimidazol-5-yl]-benzenesulfonamide (111.0 mg, 0.26 mmol) (for preparation, see Example 23) in THF (10 mL) at 0 °C. Stirring for 30 min., methyl iodide (145.9 mg, 1.03 mmol) was added. The reaction mixture was stirred for 24 h at room temperature, quenched with saturated $NaHCO_3$ (2 mL), and extracted with EtOAc (2x50 mL). The combined organic phases were washed with NaCl (20 mL) and dried with anhydrous sodium sulphate. After filtration and concentration, the residue was purified by MPLC (Hex/EtOAc (1:1) on silica gel) to give 110.3mg (93%) of colorless syrup as the title compound, which was converted to TFA salt as a white solid. $^1H$ NMR (400 MHz, $CD_3OD$): δ 1.21 (m, 5 H), 1.62 (m, 2 H), 1.70 (m, 1 H), 1.76 (m, 2 H), 1.79 (s, 6 H), 2.13 (m, 1 H), 3.27 (s, 3 H), 3.31 (s, 3 H), 4.46 (d, J=7.62 Hz, 2 H), 7.24 (m, 1 H), 7.47 (m, 1 H), 7.55 (m, 4 H), 7.67 (m, 1 H), 7.76 (m, 1 H) ; MS (ESI) $(M+H)^+$ = 456.0; Anal. Calcd for $C_{25}H_{33}N_3O_3S$+0.8TFA+0.6$H_2O$: C, 57.29; H, 6.33; N, 7.54. Found: C, 57.34; H, 6.31; N, 7.33.

**Example 25**

***N*-[1-(cyclohexylmethyl)-2-(1-methoxy-1-methylethyl)-1*H*-benzimidazol-5-yl]-benzenesulfonamide**

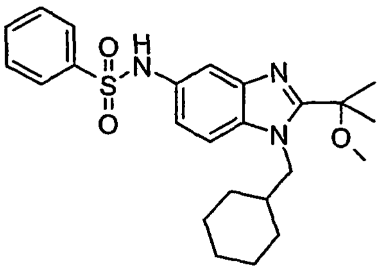

Following the same procedure in Example 24, using sodium hydride (25.0 mg, 0.63 mmol), *N*-[1-(cyclohexylmethyl)-2-(1-hydroxy-1-methylethyl)-1*H*-benzimidazol-5-yl]-benzenesulfonamide (89.3 mg, 0.21 mmol) (for preparation, see Example 23) and methyl iodide (27.4 mg, 0.19 mmol) in THF (10 mL). The desired title compound was purified by MPLC (Hex/EtOAc (1:1) on silica gel) to give 34.8 mg (38%) of colorless syrup, which was converted to TFA salt as a white solid. $^1H$ NMR (400 MHz, $CD_3OD$): δ 1.18 (m, 5 H), 1.59 (m, 2 H), 1.68 (m, 1 H), 1.74 (m, 2 H), 1.78 (s, 6 H), 2.09 (m, 1 H), 3.30 (s, 3 H), 4.42 (d, J=7.42 Hz, 2 H), 7.23 (dd, J=9.08, 2.05 Hz, 1 H), 7.47 (m, 2 H), 7.55 (m, 1 H), 7.58 (d, J=2.15 Hz, 1H), 7.72 (d, J=9.18 Hz, 1 H), 7.80 (m, 2 H); MS (ESI) $(M+H)^+$ = 442.0; Anal. Calcd for $C_{24}H_{31}N_3O_3S$+0.9TFA+0.5$H_2O$: C, 56.01; H, 5.99; N, 7.60. Found: C, 55.97; H, 6.00; N, 7.47.

**Example 26**

***N*-[2-*tert*-butyl-1-(cyclohexylmethyl)-1*H*-benzimidazol-5-yl]-*N*,1-dimethyl-1*H*-imidazole-4-sulfonamide**

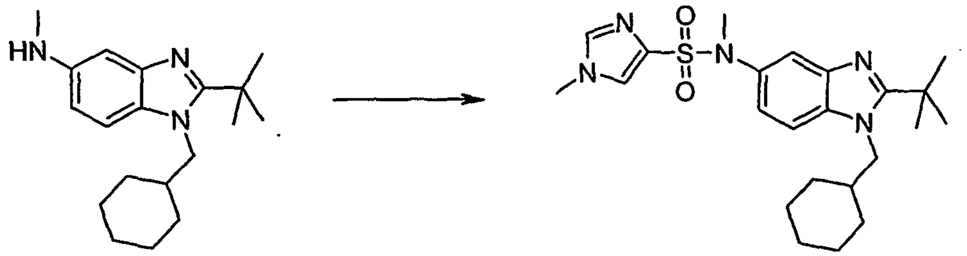

Following the same procedure of Step A in Example 4 using 2-*tert*-butyl-1-(cyclohexylmethyl)-*N*-methyl-1*H*-benzimidazol-5-amine (40 mg, 0.134 mmol), 1-methyl-1H-imidazole-4-sulphonyl chloride (31 mg, 0.174 mmol) in 3 mL of DCM containing a catalytic amount of DMAP. The solvent was evaporated and the product was purified by reversed-phase HPLC using 20-80% $CH_3CN/H_2O$ and lyophilized affording the title compound as the corresponding TFA salt. Yield: 62 mg (82%). $^1H$ NMR (400 MHz, METHANOL-$D_4$): δ 1.22 (m, 5 H), 1.62 (s, 2 H), 1.64 (s, 9 H), 1.67 (s, 1 H), 1.75 (m, 2 H), 2.08 (m, 1 H), 3.30 (s, 3 H), 3.73 (s, 3 H), 4.43 (d, J=7.62 Hz, 2 H), 7.44 (dd, J=8.98, 2.15 Hz, 1 H), 7.60 (d, J=1.37 Hz, 1 H), 7.65 (d, J=1.56 Hz, 1 H), 7.74 (d, J=0.98 Hz, 1 H), 7.82 (d, J=8.59 Hz, 1 H); MS (ESI) $(M+H)^+$ 444.0; Anal. Calcd for $C_{23}H_{33}N_5O_2S$ + 2.0 TFA + 0.1 $H_2O$: C, 48.15; H, 5.27; N, 10.40. Found: C, 48.06; H, 5.21; N, 10.55.

**Example 27**

***N*-(5-{[[2-*tert*-Butyl-1-(cyclohexylmethyl)-1*H*-benzimidazol-5-yl](methyl)amino]sulfonyl}-4-methyl-1,3-thiazol-2-yl)acetamide**

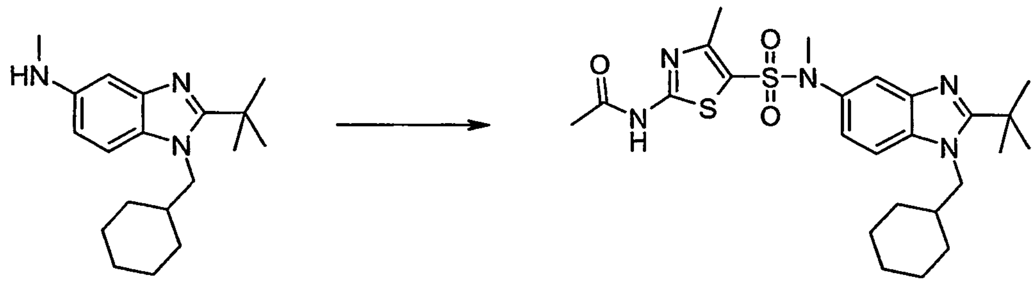

Following the same procedure of Step A in Example 4 using 2-*tert*-butyl-1-(cyclohexyhnethyl)-*N*-methyl-1*H*-benzimidazol-5-amine (40 mg, 0.134 mmol), 2-acetamido-4-methyl-5-thiazolesulfonyl chloride (44 mg, 0.174 mmol) in 3 mL of DCM containing a catalytic amount of DMAP. The solvent was evaporated and the product was purified by reversed-phase HPLC using 20-80% $CH_3CN/H_2O$ and lyophilized affording the title compound as the corresponding TFA salt. Yield: 51 mg (60%). $^1H$ NMR (400 MHz, METHANOL-$D_4$): δ 1.21 (m, 6 H), 1.59 (m, 2 H), 1.65 (m, 11 H), 2.08 (m, 4 H), 2.16 (s, 3 H), 3.32 (s, 3 H), 4.44 (d, J=7.81 Hz, 2 H), 7.41 (dd, J=9.08, 2.05 Hz, 1 H), 7.66 (d, J=1.76 Hz, 1 H), 7.86 (d, J=8.98 Hz, 1 H); MS (ESI) $(M+H)^+$ 518.0; Anal. Calcd for $C_{25}H_{35}N_5O_3S_2$ + 1.6 TFA + 0.4 $H_2O$: C, 47.88; H, 5.33; N, 9.90. Found: C, 47.88; H, 5.28; N, 10.02.

**Example 28**

***N*-[2-*tert*-Butyl-1-(cyclohexylmethyl)-1*H*-benzimidazol-5-yl]-*N*-methylpyridine-3-sulfonamide**

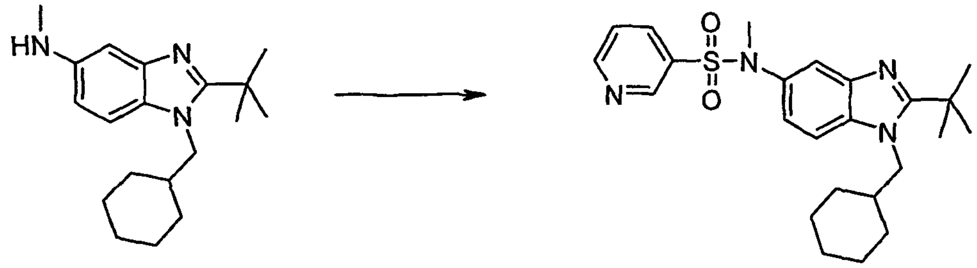

Following the same procedure of Step A in Example 4 using 2-*tert*-butyl-1-(cyclohexylmethyl)-*N*-methyl-1*H*-benzimidazol-5-amine (65 mg, 0.217 mmol), 3-pyridinesulfonyl chloride hydrochloride (70 mg, 0.325 mmol) in 3 mL of DCM containing a catalytic amount of DMAP. The solvent was evaporated and the product was purified by reversed-phase HPLC using 20-80% $CH_3CN/H_2O$ and lyophilized affording the title compound as the corresponding TFA salt. Yield: 81 mg (67%). $^1H$ NMR (400 MHz, METHANOL-$D_4$): δ 1.23 (m, 5 H), 1.62 (m, 2 H), 1.66 (s, 9 H), 1.69 (m, 1 H), 1.76 (m, 2 H), 2.09 (m, 1 H), 3.31 (s, 3 H), 4.45 (d, J=7.62 Hz, 2 H), 7.32 (dd, J=8.98, 2.15 Hz, 1 H), 7.58 (d, J=1.56 Hz, 1 H), 7.61 (dd, J=8.01, 4.88 Hz, 1 H), 7.87 (d, J=8.98 Hz, 1 H), 8.02 (dt, J=8.15, 1.88 Hz, 1 H), 8.59 (s, 1 H), 8.81 (s, 1 H); MS (ESI) $(M+H)^+$ 441.0; Anal. Calcd for $C_{24}H_{32}N_4O_2S$ + 2.0 TEA + 0.5 $H_2O$: C, 49.63; H, 5.21; N, 8.27. Found: C, 49.69; H, 5.20; N, 8.29.

**Example 29**

***N*-[2-*tert*-Butyl-1-(cyclohexylmethyl)-1*H*-benzimidazol-5-yl]-*N*,1,2-trimethyl-1*H* imidazole-5-sulfonamide**

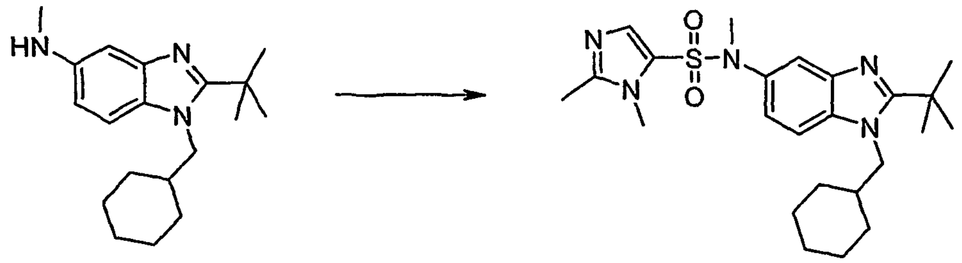

Following the same procedure of Step A in Example 4 using 2-*tert*-butyl-1-(cyclohexylmethyl)-*N*-methyl-1*H*-benzimidazol-5-amine (40 mg, 0.134 mmol), 1,2-dimethylimidazole-5-sulphonyl chloride (39 mg, 0.201 mmol) in 3 mL of DCM containing a catalytic amount of DMAP. The solvent was evaporated and the product was purified by reversed-phase HPLC using 20-80% $CH_3CN/H_2O$ and lyophilized affording the title compound as the corresponding TFA salt. Yield: 65 mg (85%). $^1H$ NMR (400 MHz, METHANOL-$D_4$): δ 1.25 (m, 5 H), 1.64 (m, 2 H), 1.69 (s, 10 H), 1.78 (m, 2 H), 2.11 (m, 1 H), 2.59 (s, 3 H), 3.40 (s, 3 H), 3.49 (s, 3 H), 4.48 (d, J=7.62 Hz, 2 H), 7.53 (dd, J=8.98, 1.95 Hz, 1 H), 7.78 (d, J=1.95 Hz, 1 H), 7.92 (s, 1 H), 7.95 (d, J=8.98 Hz, 1 H); MS (ESI) $(M+H)^+$ 458.3; Anal. Calcd for $C_{24}H_{35}N_5O_2S$ + 3.0 TFA + 0.9 $H_2O$: C, 44.16; H, 4.92; N, 8.58. Found: C, 44.24; H, 5.00; N, 8.43.

**Example 30**

***N*-[2-*tert*-Butyl-1-(tetrahydro-2*H*-pyran-4-ylmethyl)-1*H*-benzimidazol-5-yl]-*N*,1,2-trimethyl-1*H*-imidazole-5-sulfonamide**

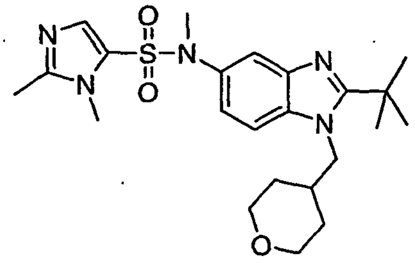

**Step A: *N*-[*2-tert*-Butyl-1-(tetrahydro-2*H*-pyran-4-ylmethyl)-1*H*-benzimidazol-5-yl]-*N*,1,2-trimethyl-1*H*-imidazole-5-sulfonamide**

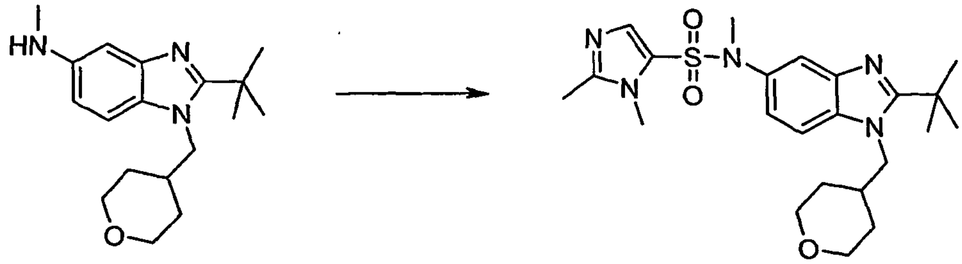

2-tert-Butyl-N-methyl-1-(tetrahydro-2H-pyran-4-ylmethyl)-1H-benzimidazol-5-amine (see following Steps B, C, D and E for preparation) (42 mg, 0.139 mmol) and 1,2-dimethylimidazole-5-sulphonyl chloride (33 mg, 0.167 mmol) were stirred in 3 mL of DCM containing a catalytic amount of DMAP overnight at rt. The solvent was evaporated and the product was purified by reversed-phase HPLC using 10-70% $CH_3CN/H_2O$ and lyophilized affording the title compound as the corresponding TFA salt. Yield: 64 mg (80%). $^1H$ NMR (400 MHz, METHANOL-$D_4$): δ 1.50 - 1.56 (m, 2 H), 1.57 - 1.64 (m, 2 H), 1.68 (s, 9 H), 2.32 - 2.41 (m, 1 H), 2.58 (s, 3 H), 3.33 (dt, J= 11.42, 2.34 Hz, 2 H), 3.38 (s, 3 H), 3.49 (s, 3 H), 3.90 - 3.93 (m, 1 H), 3.95 (d, J=2.54 Hz, 1 H), 4.54 (d, J=7.62 Hz, 2 H), 7.52 (dd, J=8.98, 2.15 Hz, 1 H), 7.76 (d, J=1.56 Hz, 1 H), 7.89 (s, 1 H), 7.96 (d, J=8.98 Hz, 1 H); MS (ESI) $(M+H)^+$ 460.0; Anal. Calcd for $C_{23}H_{33}N_5O_3S$ + 2.1 TFA + 2.3 $H_2O$: C, 44.12; H, 5.40; N, 9.72. Found: C, 43.89; H, 5.02; N, 10.12.

**Step B: Methyl {3-nitro-4-[(tetrahydro-2H-pyran-4-ylmethyl)amino]phenyl}carbamate**

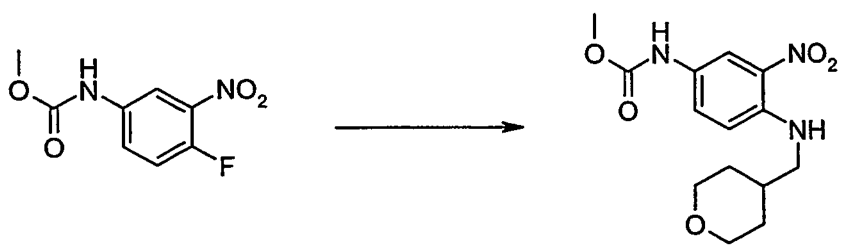

Methyl (4-fluoro-3-nitrophenyl)carbamate (2.0g, 9.32 mmol) and 4-aminomethyl tetrahydropyran (1.28g, 11.2 mmol) were stirred in 50 mL of EtOH containing TEA (2.0 mL, 14.0 mmol) at 75°C for 48h. The solvent was evaporated. The residue was dissolved in EtOAc and washed with aqueous 5% $KHSO_4$, saturated aqueous $NaHCO_3$ solution, brine and dried over anhydrous $MgSO_4$. The crude product was purified by silica gel flash chromatography using 1:1 / hexanes : EtOAc as eluent. Yield: 2.53g (88%). $^1H$ NMR (400 MHz, CHLOROFORM-D): δ 1.42 (ddd, *J*=25.24, 12.06, 4.49 Hz, 2 H), 1.73 (d, *J*=1.76 Hz, 1 H), 1.76 (d, *J*=1.95 Hz, 1 H), 1.88 - 2.01 (m, 1 H), 3.22 (dd, *J*=6.74, 5.57 Hz, 2 H), 3.42 (td, *J*=1.86, 2.05 Hz, 2 H), 3.78 (s, 3 H), 4.01 (d, *J*=4.30 Hz, 1 H), 4.04 (d, *J*=3.51 Hz, 1 H), 6.48 (br.s, 1 H), 6.85 (d, *J*=9.37

Hz, 1 H), 7.65 (br.s, 1 H), 8.03 - 8.09 (m, 2 H).

**Step C: Methyl {3-amino-4-[(tetrahydro-2*H*-pyran-4-ylmethyl)amino]phenyl}carbamate**

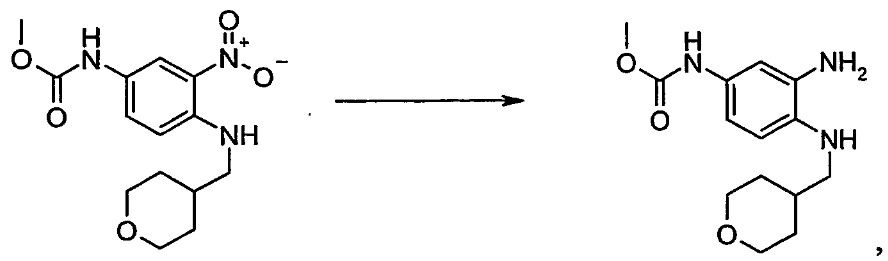

Methyl {3-nitro-4-[(tetrahydro-2H-pyran-4-ylmethyl)amino]phenyl} carbamate (2.53 g, 8.18 mmol) was dissolved in 50 mL of EtOAc containing a catalytic amount of 10% Pd/C. The solution was shaken under $H_2$ atmosphere (40 psi) using a Parr hydrogenation apparatus overnight at rt. The solution was filtered through celite and the solvent was evaporated. Yield: 2.29 g (99%). $^1$H NMR (400 MHz, CHLOROFORM-D): δ 1.40 (ddd, *J*=25.09, 12.01, 4.49 Hz, 2 H), 1.70 - 1.74 (m, 1 H), 1.74 - 1.77 (m, 1 H), 1.81 - 1.92 (m, 1 H), 2.99 (d, *J*=6.64 Hz, 2 H), 3.34 (br.s, 2 H), 3.41 (dt, *J*=11.81, 2.15 Hz, 2 H), 3.74 (s, 3 H), 3.99 (d, *J*=3.51 Hz, 1 H), 4.02 (d, *J*=3.51 Hz, 1 H), 6.38 (br.s, 1 H), 6.55 - 6.60 (m, 1H), 6.62 - 6.68 (m, 1 H), 6.95 (br.s, 1 H).

**Step D: Methyl [2-tert-butyl-1-(tetrahydro-2H-pyran-4-ylmethyl)-1H-benzimidazol-5-yl]carbamate**

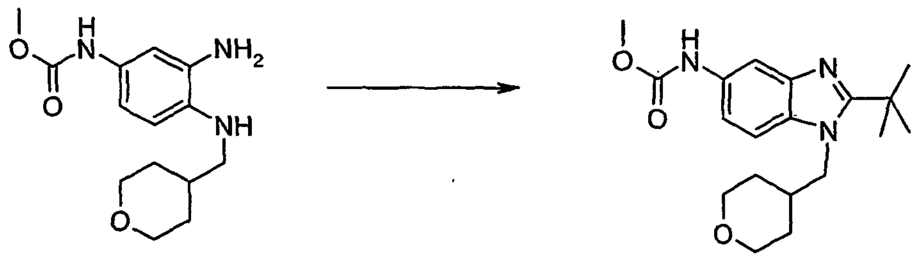

Methyl {3-amino-4-[(tetrahydro-2*H*-pyran-4-ylmethyl)amino]phenyl}carbamate (2.29 g, 8.20 mmol) and DMAP (0.20 g, 1.64 mmol) were dissolved in 75 mL of DCM. Trimethylacetyl chloride (1.10 mL, 9.02 mmol) was added dropwise and the solution was stirred at rt for 2h. The solution was washed with aqueous $NaHCO_3$ solution, brine and dried over anhydrous $MgSO_4$. The residue was dissolved in 25 mL of AcOH and was heated at 125°C for 1h using a Personal Chemistry microwaves apparatus. The solvent was evaporated. The residue was dissolved in EtOAc and washed with aqueous $NaHCO_3$ solution, brine and dried over anhydrous $MgSO_4$. The crude product was purified by silica gel flash chromatography using 4:3 / hexanes : acetone as eluent. Yield: 1.81 g (64%). $^1$H NMR (400 MHz, CHLOROFORM-D): δ 1.48 - 1.54 (m, 4 H) 1.56 (s, 9 H) 2.23 - 2.35 (m, 1H) 3.27 - 3.35 (m, 2 H) 3.78 (s, 3 H) 3.96 (t, *J*=2.93 Hz, 1 H) 3.99 (t, *J*=3.03 Hz, 1 H) 4.18 (d, J=7.42 Hz, 2 H) 6.63 (br.s, 1 H) 7.24 - 7.28 (m, 1 H) 7.41 (br.s, 1 H) 7.61 (d, *J*=1.95 Hz, 1 H).

**Step E: 2-tert-Butyl-N-methyl-1-(tetrahydro-2H-pyran-4-ylmethyl)-1H-benzimidazol-5-amine**

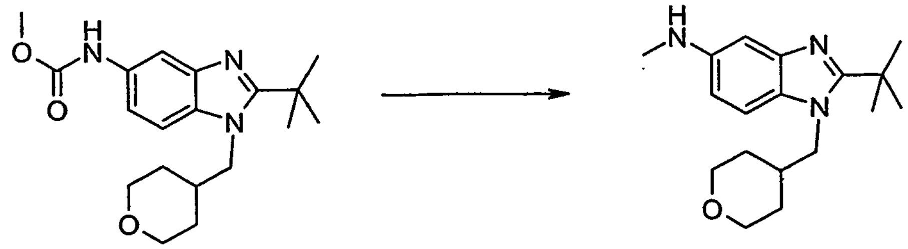

Methyl [2-tert-butyl-1-(tetrahydro-2H-pyran-4-ylmethyl)-1H-benzimidazol-5-yl]carbamate (1.80 g, 5.21 mmol) was dissolved in 75 mL of THF at 0°C. 1M HCl/ether (7.3 mL, 7.29 mmol) was added dropwise and the solution was stirred at 0°C for 15 min. $LiAlH_4$ (988 mg, 26.1 mmol) was added slowly and the solution was stirred at rt overnight. The reaction was quenched at 0°C by the addition of MeOH (5 mL) followed by water (10 mL) and the solution was left to stir at rt for 30 min. Anhydrous $Na_2SO_4$ (10 g) was added and the solution was stirred at rt for another 30 min. The solution was filtered and the solvent was evaporated. The residue was dissolved in EtOAc and washed with aqueous $NaHCO_3$ solution, brine and dried over anhydrous $MgSO_4$. The solvent was evaporated. Yield: 1.54 g (98%). $^1H$ NMR (400 MHz, CHLOROFORM-D): δ 1.49 - 1.53 (m, 4 H), 1.53 - 1.57 (m, 9 H), 2.22 - 2.32 (m, 1 H), 2.87 (s, 3 H), 3.26 - 3.35 (m, 2 H), 3.95 (t, *J*=3.03 Hz, 1 H), 3.97 - 4.00 (m, 1 H), 4.13 (d, *J*=7.42 Hz, 2 H), 6.61 (dd, *J*=8.59, 2.15 Hz, 1 H), 6.99 (d, *J*=1.95 Hz, 1 H),7.11 (d, *J*=8.59 Hz, 1 H).

**Example 31**

**Ethyl 4-{[[2-*tert*-butyl-1-(tetrahydro-2*H*-pyran-4-ylmethyl)-1*H*-benzimidazol-5-yl](methyl)amino]sulfonyl}-3,5-dimethyl-1*H*-pyrrole-2-carboxylate**

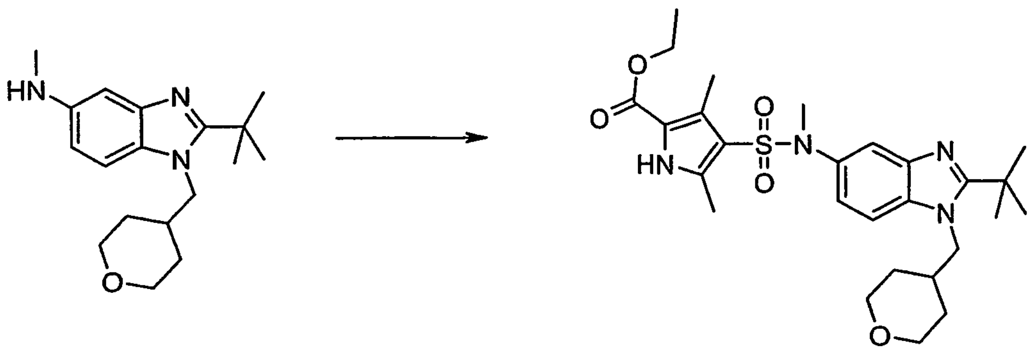

Following the same procedure as in Step A of Example 30 using 2-tert-butyl-N-methyl-1-(tetrahydro-2H-pyran-4-ylmethyl)-1H-benzimidazol-5-amine (42 mg, 0.139 mmol), ethyl 4-(chlorosulfonyl)-3,5-dimethyl-1H-pyrrole-2-carboxylate (44 mg, 0.167 mmol) in 3 mL of DCM containing a catalytic amount of DMAP. The product was purified by reversed-phase HPLC using 10-70% $CH_3CN/H_2O$ and lyophilized affording the title compound as the corresponding TFA salt. Yield: 61 mg (68%). $^1H$ NMR (400 MHz, METHANOL-$D_4$): δ 1.33 (t, J=7.13 Hz, 3 H), 1.45 - 1.53 (m, 2 H), 1.53 - 1.65 (m, 2 H), 1.68 (s, 9 H), 2.04 (s, 3 H), 2.16 (s, 3 H), 2.32 - 2.41 (m, 1 H), 3.24 (s, 3 H), 3.35 (td, J=11.77, 2.05 Hz, 2 H), 3.93 (d, J=3.51 Hz, 1 H), 3.96 (d, J=3.51 Hz, 1 H), 4.28 (q, J=7.09 Hz, 2 H), 4.54 (d, J=7.62 Hz, 2 H), 7.42 (dd, J=9.08, 2.05 Hz, 1 H), 7.65 (d, J=1.56 Hz, 1 H), 7.91 (d, J=8.98 Hz, 1 H); MS (ESI) $(M+H)^+$ 531.2; Anal. Calcd for $C_{27}H_{38}N_4O_5S$ + 1.4 TFA + 0.6 $H_2O$: C, 51.05; H, 5.84; N, 7.99. Found: C, 51.07; H, 5.91; N, 7.88.

**Example 32**

***N*-[2-*tert*-Butyl-1-(tetrahydro-2*H*-pyran-4-ylmethyl)-1*H*-benzimidazol-5-yl]-4-(hydroxymethyl)-*N*-methylbenzenesulfonamide**

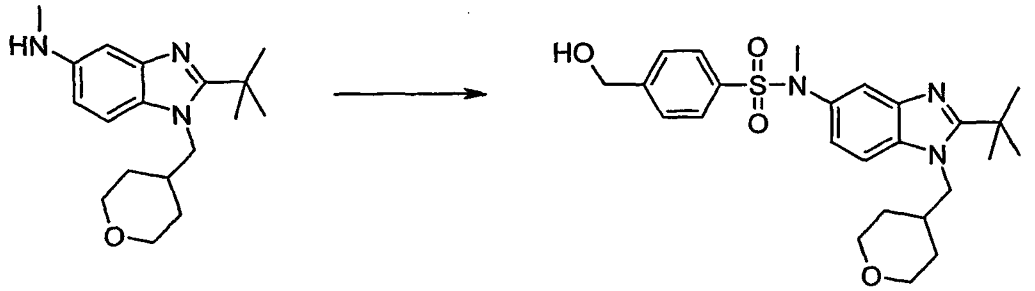

2-tert-Butyl-N-methyl-1-(tetrahydro-2H-pyran-4-ylmethyl)-1H-benzimidazol-5-amine (35 mg, 0.116 mmol) and 4-formylbenzenesulfonyl chloride (29 mg, 0.139 mmol) were stirred in 3 mL of DCM containing a catalytic amount of DMAP at rt for 2h. The solution was washed with aqueous $NaHCO_3$ solution, brine and dried over anhydrous $MgSO_4$. The solvent was evaporated. The residue was dissolved in MeOH (5 mL) and $NaCNBH_3$ (20 mg, 0.298 mmol) was added. The solution was stirred overnight at rt. The solvent was evaporated. The residue was dissolved in EtOAc and washed with aqueous $NaHCO_3$ solution, brine and dried over anhydrous $MgSO_4$. The solvent was evaporated. The crude product was purified by silica gel MHz, METHANOL-$D_4$) (TFA salt): δ 1.50 - 1.56 (m, 2 H), 1.57 -1.65 (m, 2 H), 1.68 (s, 9 H), 2.31 - 2.41 (m, 1 H), 3.26 (s, 3 H), 3.35 (td, J=11.57, 2.64 Hz, 2 H), 3.93 (d, J=3.32 Hz, 1 H), 3.96 (d, J=3.71 Hz, 1 H), 4.52 (d, J=7.42 Hz, 2 H), 4.68 (s, 2 H), 7.30 (dd, J=8.98, 2.15 Hz, 1 H), 7.50 (s, 4 H), 7.54 (d, J=1.56 Hz, 1 H), 7.87 (d, J=8.98 Hz, 1 H); MS (ESI) $(M+H)^+$ 472.0; Anal. Calcd for $C_{25}H_{33}N_3O_4S$ + 1.5 TFA + 0.3 $H_2O$: C, 51.89; H, 5.46; N, 6.48. Found: C, 51.94; H, 5.48; N, 6.31.

**Example 33**

***N*-[2-*tert*-Butyl-1-(tetrahydro-2*H*-pyran-4-ylmethyl)-1*H*-benzimidazol-5-yl]-*N*-methyl-4-(1*H*-1,2,3-triazol-1-ylmethyl)benzenesulfonamide**

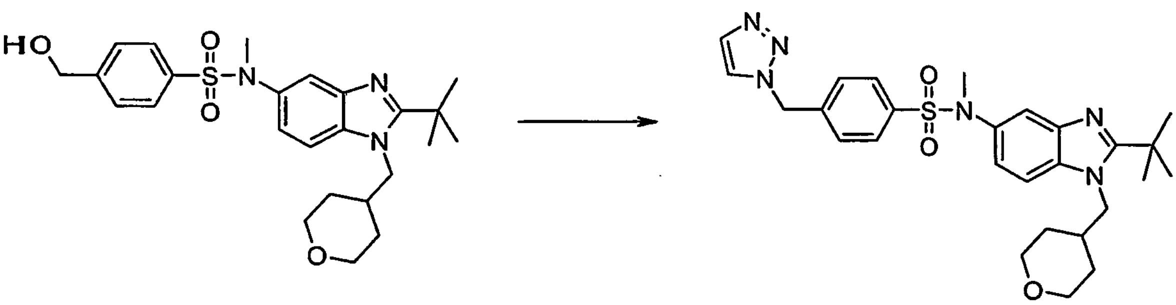

*N*-[2-*tert*-Butyl-1-(tetrahydro-2*H*-pyran-4-ylmethyl)-1*H*-benzimidazol-5-yl]-4-(hydroxymethyl)-N-methylbenzenesulfonamide (55mg, 0.117 mmol) and TEA (0.025 mL, 0.176 mmol) were dissolved in 5 mL of DCM at 0°C. Methanesulfonyl chloride (0.01 mL, 0.140 mmol) was added dropwise and the solution was stirred at rt for 3h. The solution was washed with aqueous $NaHCO_3$ solution, brine and dried over anhydrous $MgSO_4$. The solvent was evaporated. The residue was dissolved in 2 mL of DMF and KI (19 mg, 0.117 mmol) followed by 1H-1,2,3-triazole (0.135 mL, 2.34 mmol) were added. The solution was stirred at 80°C for 1h. The solvent was evaporated. The product was purified by reversed-phase HPLC using 10-70% $CH_3CN/H_2O$ and lyophilized affording the title compound as the corresponding TFA salt. Yield: 35 mg (47%). $^1H$ NMR (400 MHz, METHANOL-$D_4$): δ 1.50 - 1.56 (m, 2 H), 1.56 - 1.65 (m, 2 H), 1.68 (s, 9 H), 2.32 - 2.40 (m, 1 H), 3.26 (s, 3 H), 3.35 (td, J=11.57, 2.64 Hz, 2 H), 3.93 (d, J=3.32 Hz, 1 H), 3.96 (d, J=3.51 Hz, 1 H), 4.52 (d, J=7.42 Hz, 2 H), 5.74 (s, 2 H), 7.31 (dd, J=8.98, 1.95 Hz, 1 H), 7.41 (d, J=8.59 Hz, 2 H), 7.54 (s, 1 H), 7.55 - 7.57 (m, 2 H), 7.79 (s, 1 H), 7.88 (d, J=8.98 Hz, 1 H), 8.09 (s, 1 H); MS (ESI) $(M+H)^+$ 523.0; Anal. Calcd for $C_{27}H_{34}N_6O_3S$ + 2.4 TFA: C, 47.96; H, 4.61; N, 10.55. Found: C, 48.02; H, 4.72; N, 10.22.

**Example 34**

***N*-[2-*tert*-Butyl-1-(tetrahydro-2*H*-pyran-4-ylmethyl)-1*H*-benzimidazol-5-yl]-4-{[(2-hydroxyethyl)amino] methyl}-*N*-methylbenzenesulfonamide**

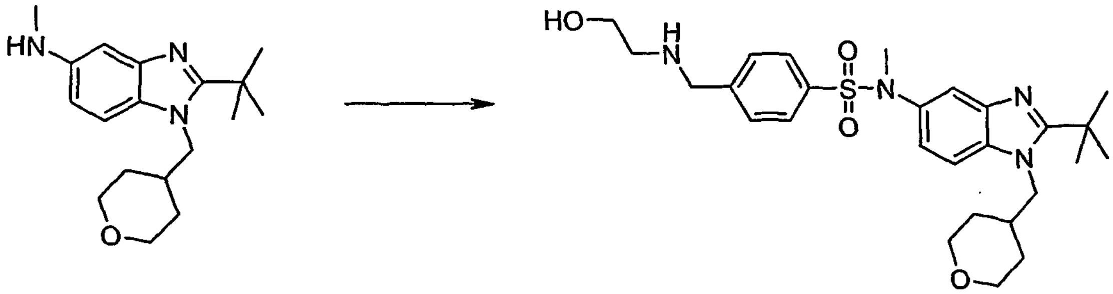

2-tert-Butyl-N-methyl-1-(tetrahydro-2H-pyran-4-ylmethyl)-1H-benzimidazol-5-amine (58 mg, 0.192 mmol) and 4-formylbenzenesulfonyl chloride (47 mg, 0.230 mmol) were stirred in 5 mL of DCM containing a catalytic amount of DMAP at rt for 3h. The solution was washed with aqueous $NaHCO_3$ solution, brine and dried over anhydrous $MgSO_4$ The solvent was evaporated. The residue was dissolved in MeOH (5 mL) containing a few drops of AcOH and 3Å molecular sieves. Ethanolamine (0.057 mL, 0.960 mmol) was added and the solution was stirred at rt for 30 min. $NaCNBH_3$ (36 mg, 0.576 mmol) was added and the solution was stirred at rt for 3h. The solvent was evaporated. The residue was dissolved in EtOAc and washed with aqueous $NaHCO_3$ solution, brine and dried over anhydrous $MgSO_4$. The product was purified by reversed-phase HPLC using 10-60% $CH_3CN/H_2O$ and lyophilized affording the title compound as the corresponding TFA salt. Yield: 48 mg (40%). $^1$H NMR (400 MHz, METHANOL-$D_4$): δ 1.49 - 1.55 (m, 2 H), 1.55 - 1.61 (m, 2 H), 1.67 (s, 9 H), 2.32 - 2.39 (m, 1 H), 3.15 - 3.18 (m, 2 H), 3.27 (s, 3 H), 3.34 (dt, J=11.47, 2.64 Hz, 2 H), 3.81 (dd, J=5.96, 4.39 Hz, 2 H), 3.92 (d, J=3.12 Hz, 1 H), 3.95 (d, J=3.71 Hz, 1 H), 4.33 (s, 2 H), 4.51 (d, J=7.62 Hz, 2 H), 7.28 (dd, J=9.08, 2.05 Hz, 1 H), 7.56 (d, J=1.95 Hz, 1 H), 7.61 - 7.68 (m, 4 H), 7.85 (d, J=8.98 Hz, 1 H); MS (ESI) $(M+H)^+$ 515.2; Anal. Calcd for $C_{27}H_{38}N_4O_4S$ + 2.7 TFA + 0.9 $H_2O$: C, 46.40; H, 5.11; N, 6.68. Found: C, 46.41; H, 5.05; N, 6.75.

**Example 35**

***N*-[2-*tert*-Butyl-1-(cyclopentylmethyl)-1*H*-benzimidazol-5-yl]-*N*-methylbenzenesulfonamide**

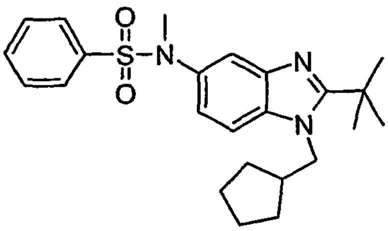

**Step A: *N*-[2-*tert*-Butyl-1-(cyclopentylmethyl)-1*H*-benzimidazol-5-yl]-*N*-methylbenzenesulfonamide**

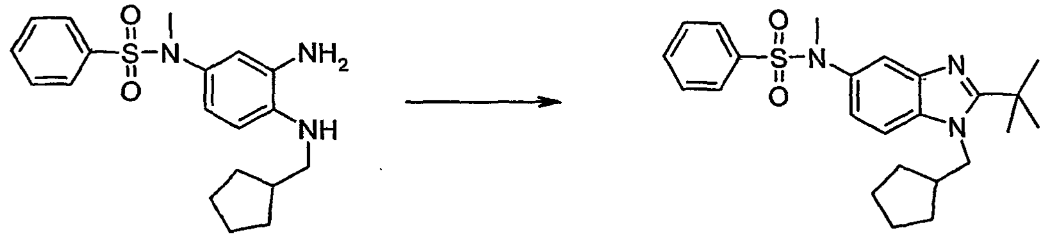

*N*-{3-Amino-4-[(cyclopentylmethyl)amino]phenyl}-N-methylbenzenesulfonamide (see following Steps B and C for preparation) (50 mg, 0.139 mmol) and trimethylacetyl chloride (0.019 mL, 0.153 mmol) were stirred in 2 mL of DCM containing a catalytic amount of DMAP at rt for 1h. The solvent was evaporated. The product was dissolved in 2 mL of AcOH and was stirred at 150°C for 40 min using a Personal Chemistry microwaves instrument. The solvent was evaporated. The residue was dissolved in EtOAc and washed with aqueous $NaHCO_3$ solution, brine and dried over anhydrous $MgSO_4$. The product was purified by reversed-phase HPLC using 20-80% $CH_3CN/H_2O$ and lyophilized affording the title compound as the corresponding TFA salt. Yield: 26 mg (35%). $^1H$ NMR (400 MHz, METHANOL-$D_4$): δ 1.47 (m, 2 H), 1.62 (m, 2 H), 1.68 (s, 9 H), 1.78 (m, 4 H), 2.51 (m, 1 H), 3.28 (s, 3 H), 4.61 (d, J=7.42 Hz, 2 H), 7.30 (dd, J=9.08, 2.05 Hz, 1 H), 7.54 (m, 5 H), 7.68 (m, 1 H), 7.87 (d, J=9.18 Hz, 1 H); MS (ESI) $(M+H)^+$ 426.2; Anal. Calcd for $C_{24}H_{31}N_3O_2S$ + 1.5 TFA + 0.9 $H_2O$: C, 52.92; H, 5.64; N, 6.96. Found: C, 52.80; H, 5.51; N, 7.35.

**Step B: *N*-{4-[(Cyclopentylmethyl)amino]-3-nitrophenyl}-*N*-methylbenzenesulfonamide**

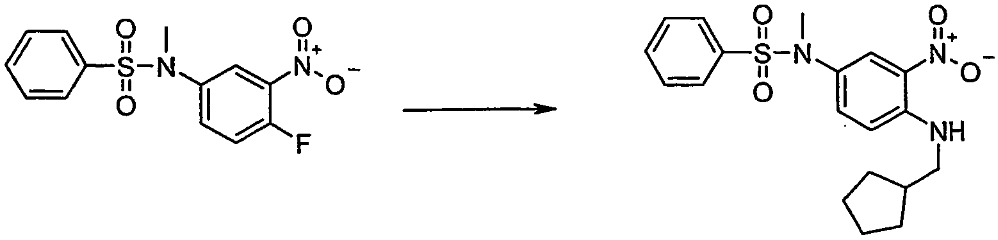

*N*-(4-Fluoro-3-nitrophenyl)-*N*-methylbenzenesulfonamide (for preparation see Example 3, Steps B and C) (50 mg, 0.161 mmol) and cyclopentylmethylamine (0.062 mL) of a 1g/3 mL solution, 0.209 mmol) were stirred in 2 mL of EtOH containing TEA (0.025 mL, 0.241 mmol) at 75°C for 5h. The solvent was evaporated. The residue was dissolved in EtOAc and washed with 5% aqueous $KHSO_4$ solution, aqueous $NaHCO_3$ solution, brine and dried over anhydrous $MgSO_4$. The crude product was purified by silica gel flash chromatography using 3:1 /hexanes:EtOAc as eluent. Yield: 57 mg (91%). $^1H$ NMR (400 MHz, CHLOROFORM-D): δ 1.31 (m, 3 H), 1.66 (m, 4 H), 1.91 (m, 2 H), 2.28 (m, 1 H), 3.13 (s, 3 H), 3.24 (dd, J=7.23, 5.08 Hz, 2 H), 6.84 (d, J=9.37 Hz, 1 H), 7.45 (dd, J=9.28, 2.64 Hz, 1 H), 7.50 (m, 2 H), 7.60 (m, 3 H), 8.17 (m, 1 H).

**Step C: *N*-{3-Amino-4-[(cyclopentylmethyl)amino]phenyl}-*N*-methylbenzenesulfonamide**

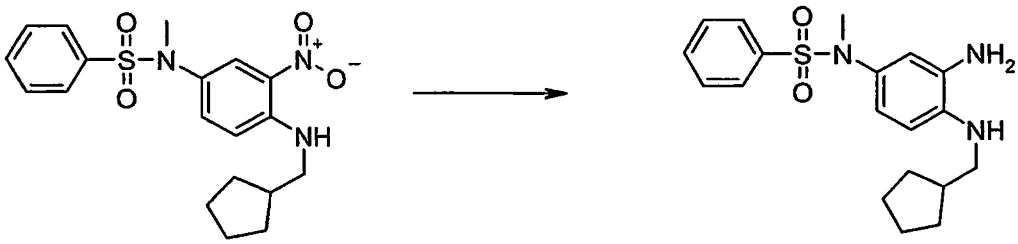

*N*-{4-[(Cyclopentylmethyl)amino]-3-nitrophenyl}-*N*-methylbenzenesulfonamide (55 mg, 0.141 mmol) was dissolved in 15 mL of EtOAc containing a catalytic amount of 10% Pd/C. The solution was shaken under $H_2$ atmosphere (40 psi) at rt for 3h. The solution was filtered through celite and the solvent was evaporated. Yield: 51 mg (99%). MS (ESI) $(M+H)^+$ 360.26.

**Example 36**

***N*-[2-*tert*-Butyl-1-(cyclobutylmethyl)-1*H*-benzimidazol-5-yl]-*N*-methylbenzenesulfonamide**

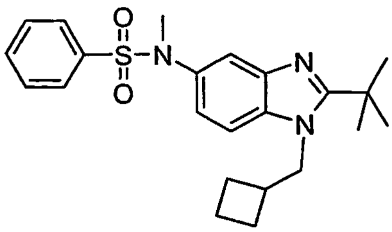

**Step A: *N*-[2-*tert*-Butyl-1-(cyclobutylmethyl)-1H-benzimidazol-5-yl]-*N*-methylbenzenesulfonamide**

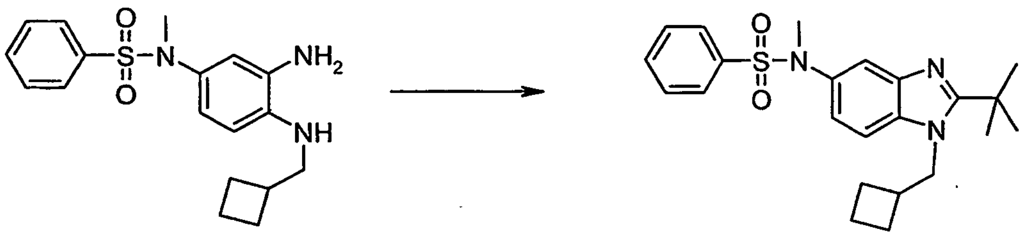

Following the same procedure used as in Step A in Example 35 using *N*-{3-amino-4-[(cyclobutylmethyl)amino]phenyl}-*N*-methylbenzenesulfonamide (for preparation see following Steps B and C) (53 mg, 0.153 mmol) and trimethylacetyl chloride (0.021 mL, 0.168 mmol) in 2 mL of DCM containing a catalytic amount ofDMAP. The product was purified by reversed-phase HPLC using 20-80% $CH_3CN/H_2O$ and lyophilized affording the title compound as the corresponding TFA salt. Yield: 43 mg (53%). $^1H$ NMR (400 MHz, METHANOL-$D_4$): δ 1.66 (s, 9 H), 1.92 (m, 2 H), 2.10 (m, 4 H), 2.87 (m, 1 H), 3.27 (s, 3 H), 4.65 (d, J=6.44 Hz, 2 H), 7.30 (dd, J=8.98, 1.95 Hz, 1 H), 7.53 (m, 5 H), 7.68 (m, 1 H), 7.81 (d, J=8.98 Hz, 1 H); MS (ESI) $(M+H)^+$ 412.3; Anal. Calcd for $C_{23}H_{29}N_3O_2S$ + 1.4 TFA + 0.8 $H_2O$: C, 52.92; H, 5.51; N, 7.18. Found: C, 52.91; H, 5.46; N, 7.11.

**Step B: *N*-{4-[(Cyclobutylmethyl)amino]-3-nitrophenyl}-*N*-methylbenzenesulfonamide**

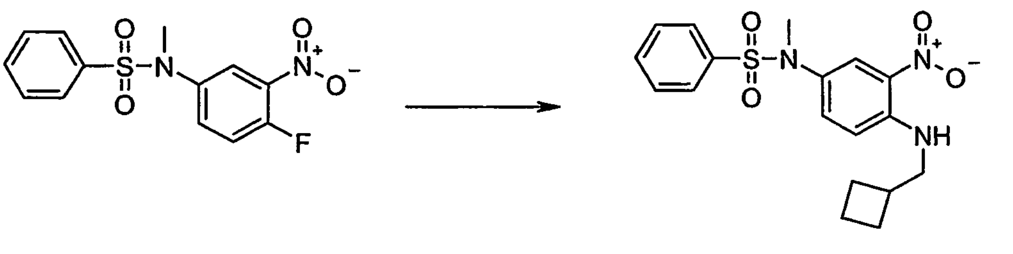

Following the same procedure used as in Example 35, Step B using *N*-(4-fluoro-3-nitrophenyl)-*N*-methylbenzenesulfonamide (for preparation see Example 3, Steps B and C) (50 mg, 0.161 mmol), cyclobutylmethylamine (0.040 mL of a 5.3M solution/MeOH, 0.209 mmol) in 2 mL of EtOH containing TEA (0.025 mL, 0.242 mmol). The crude product was purified by silica gel flash chromatography using 3:1 /hexanes:EtOAc as eluent. Yield: 61 mg (99%). $^1$H NMR (400 MHz, CHLOROFORM-D): δ 1.56 (s, 2 H), 1.81 (m, 2 H), 1.95 (m, 1 H), 2.19 (m, 2 H), 3.13 (s, 3 H), 3.33 (dd, J=7.23, 5.08 Hz, 2 H), 6.83 (d, J=9.18 Hz, 1 H), 7.45 (dd, J=9.18, 2.54 Hz, 1 H), 7.51 (m, 2 H), 7.61 (m, 3 H), 8.04 (m, 1 H).

**Step C: *N*-{3-Amino-4-[(cyclobutylmethyl)amino]phenyl}-*N*-methylbenzenesulfonamide**

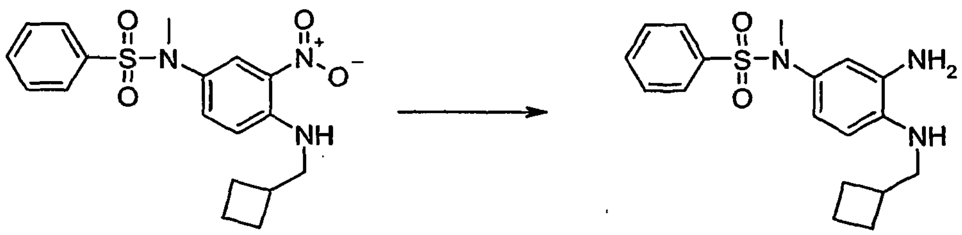

Following the same procedure used as in Example 35 Step C using *N*-{4-[(cyclobutylmethyl)amino]-3-nitrophenyl}-*N*-methylbenzenesulfonamide (58 mg, 0.154 mmol) in 15 mL of EtOAC containing a catalytic amount of 10% Pd/C. Yield: 53 mg (99%). MS (ESI) $(M+H)^+$ 346.20.

**Example 37**

***N*-[(2-tert-Butyl-1-(2-cyclohexylethyl)-1*H*-benzimidazol-5-yl)-*N* methylbenzenesulfonamide**

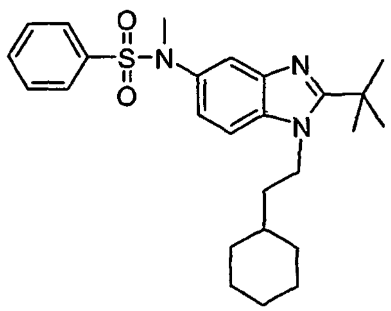

**Step A: *N*-[2-*tert*-Butyl-1-(2-cyclohexylethyl)-1*H*-benzimidazol-5-yl]-*N* methylbenzenesulfonamide**

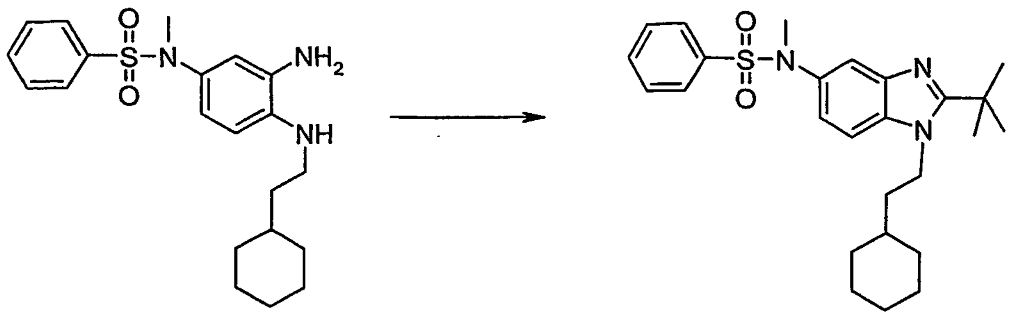

Following the same procedure used as in Step A of Example 35 using *N*-{3-amino-4-[(2-cyclohexylethyl)amino]phenyl}-*N*-methylbenzenesulfonamide (for preparation see following Steps B and C) (56 mg, 0.145 mmol) and trimethylacetyl chloride (0.020 mL, 0.160 mmol) in 2 mL of DCM containing a catalytic amount of DMAP. The product was purified by reversed-phase HPLC using 20-80% $CH_3CN/H_2O$ and lyophilized affording the title compound as the corresponding TFA salt. Yield: 37 mg (45%). $^1H$ NMR (400 MHz, METHANOL-$D_4$): δ 1.11 (ddd, J=23.92, 12.11, 2.83 Hz, 2 H), 1.34 (m, 3 H), 1.60 (m, 1 H), 1.66 (s, 9 H), 1.72 (m, 1 H), 1.77 (m, 1 H), 1.81 (m, 2 H), 1.85 (m, 2 H), 1.90 (m, 1 H), 3.27 (s, 3 H), 4.60 (m, 2 H), 7.33 (dd, J=8.98, 1.95 Hz, 1 H), 7.54 (m, 5 H), 7.68 (m, 1 H), 7.74 (d, J=8.98 Hz, 1 H); MS (ESI) $(M+H)^+$454.2; Anal. Calcd for $C_{26}H_{35}N_3O_2S$ + 1.4 TFA: C, 56.40; H, 5.98; N, 6.85. Found: C, 56.48; H, 5.98; N, 6.99.

**Step B: *N*-{4-[(2-Cyclohexylethyl)amino]-3-nitrophenyl}-*N*-methylbenzenesulfonamide**

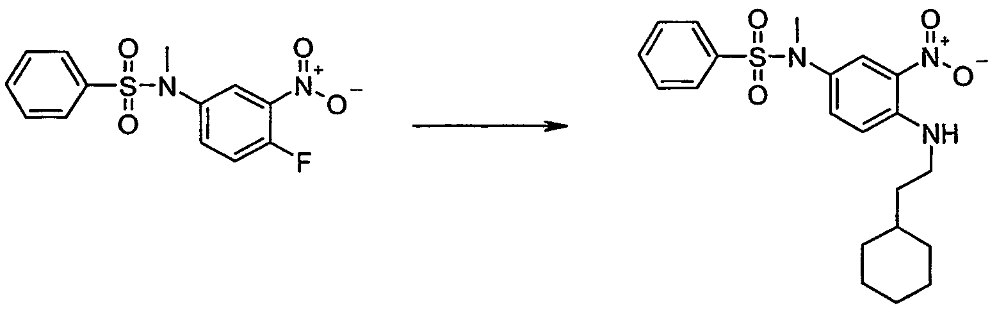

Following the same procedure used as in Example 35 Step B using *N*-(4-fluoro-3-nitrophenyl)-*N*-methylbenzenesulfonamide (for preparation see Example 3, Steps B and C) (50 mg, 0.161 mmol), (2-cyclohexylethyl)amine hydrochloride (32 mg, 0.193 mmol) in 2 mL of EtOH containing TEA (0.060 mL, 0.402 mmol). The crude product was purified by silica gel flash chromatography using 4:1 / hexanes:EtOAc as eluent. Yield: 65 mg (97%). $^1H$ NMR (400 MHz, CHLOROFORM-D): δ 0.99 (m, 2 H), 1.25 (m, 4 H), 1.43 (m, 1 H), 1.64 (m, 3 H), 1.72 (m, 1 H), 1.75 (m, 2 H), 1.78 (m, 1 H), 3.13 (s, 3 H), 3.33 (m, 2 H), 6.83 (d, J=9.37 Hz, 1 H), 7.46 (m, 1 H), 7.51 (m, 2 H), 7.58 (m, 2 H), 7.61 (m, 1 H), 8.08 (m, 1 H).

**Step C: *N*-{3-Amino-4-[(2-cyclohexylethyl)amino]phenyl}-*N*-methylbenzenesulfonamide**

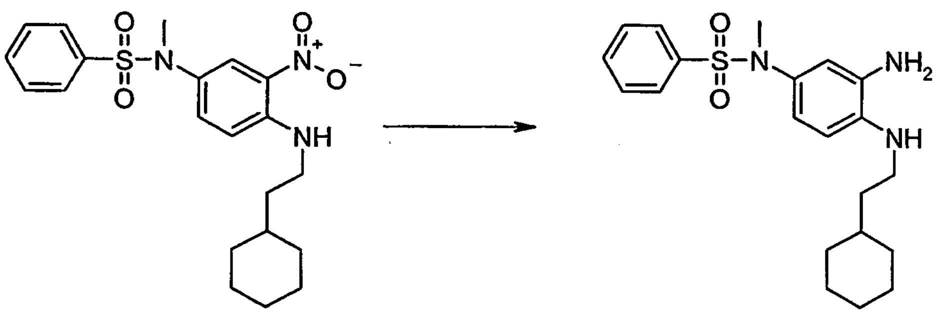

Following the same procedure used as in Example 35 Step C using *N*-{4-[(2-cyclohexylethyl)amino]-3-nitrophenyl}-*N*-methylbenzenesulfonamide (60 mg, 0.144 mmol) in 15 mL of EtOAc containing a catalytic amount of 10% Pd/C. Yield: 56 mg (99%). MS (ESI) $(M+H)^+$ 388.26.

**Example 38**

***N*-[1-(1-Benzylpyrrolidin-3-yl)-2-*tert*-butyl-1*H*-benzimidazol-5-yl]-*N*-methylbenzenesulfonamide**

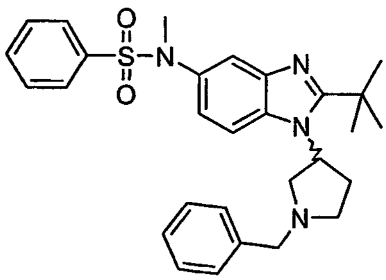

**Step A: *N*-[1-(1-Benzylpyrrolidin-3-yl)-2-*tert*-butyl-1*H*-benzimidazol-5-yl]-*N*-methylbenzenesulfonamide**

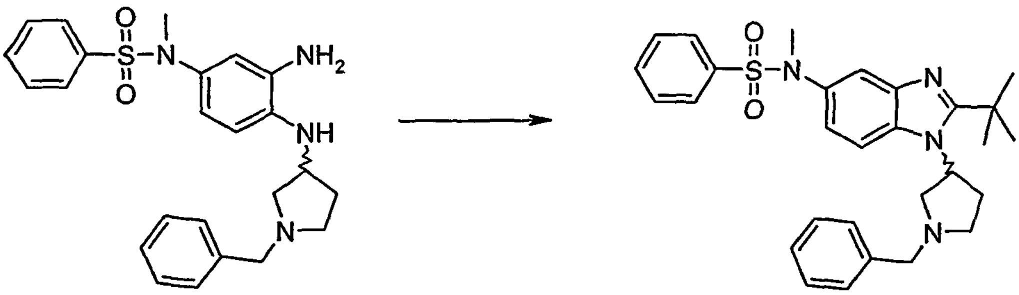

Following the same procedure used as in Step A in Example 35 using *N*-{3-amino-4-[(1-benzylpyrrolidin-3-yl)amino] phenyl}-*N*-methylbenzenesulfonamide (for preparation see following Steps B and C) (75 mg, 0.172 mmol) and trimethylacetyl chloride (0.025 mL, 0.189 mmol) in 5 mL of DCM containing a catalytic amount of DMAP. The product was purified by reversed-phase HPLC using 10-60% $CH_3CN/H_2O$ and lyophilized affording the title compound as the corresponding TFA salt. Yield: 48 mg (45%). $^1$HNMR (400 MHz, METHANOL-$D_4$): δ 1.59 (s, 9 H), 2.61 (m, 1 H), 2.72 (m, 1 H), 3.23 (m, 3 H), 3.44 (m, 1 H), 3.66 (m, 1 H), 3.75 (m, 1 H), 3.81 (m, 1 H), 4.44 (m, 2 H), 5.85 (m, 1 H), 7.23 (dd, J=8.89, 2.05 Hz, 1 H), 7.43 (m, 4 H), 7.51 (m, 6 H), 7.65 (m, 1 H), 7.94 (d, J=7.81 Hz, 1 H); MS (ESI) $(M+H)^+$ 503.2; Anal. Calcd for $C_{29}H_{34}N_4O_2S$ + 2.3 TFA + 0.3 $H_2O$: C, 52.39; H, 4.83; N, 7.27. Found: C, 52.44; H, 4.87; N, 7.28.

**Step B: *N*-{4-[(1-Benzylpyrrolidin-3-yl)amino]-3-nitrophenyl}-*N*-methylbenzenesulfonamide**

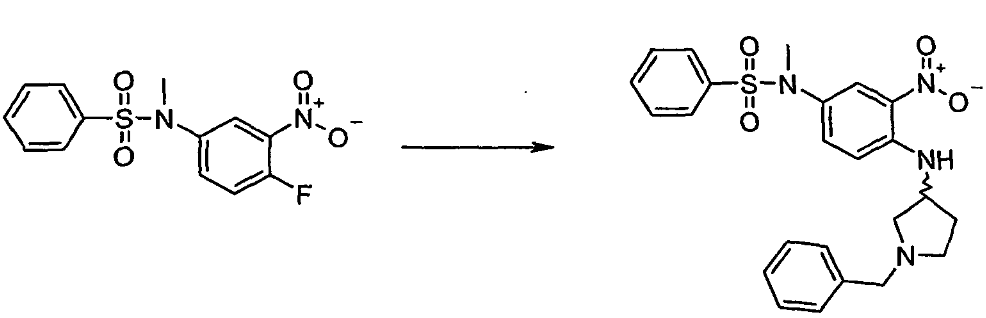

Following the same procedure used as in Example 35 Step B using *N*-(4-fluoro-3-nitrophenyl)-*N*-methylbenzenesulfonamide (for preparation see Example 3, Steps B and C) (70 mg, 0.226 mmol), 1-benzylpyrrolidin-3-amine (50 mg, 0.271 mmol) in 3 mL of EtOH containing TEA (0.050 mL, 0.339 mmol). The crude product was purified by silica gel flash chromatography using 50 to 80% EtOAc in hexanes as eluent. Yield: 95 mg (90%). MS (ESI) $(M+H)^+$ 466.99.

**Step C: *N*-{3-Amino-4-[(1-benzylpyrrolidin-3-yl)amino]phenyl}-*N*-methylbenzenesulfonamide**

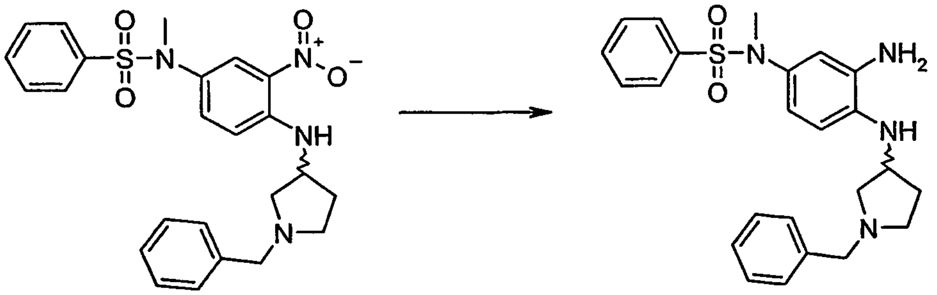

Following the same procedure used as in Example 35 Step C using *N*-{4-[(1-benzylpyrrolidin-3-yl)amino]-3-nitrophenyl}-*N*-methylbenzenesulfonamide (95 mg, 0.204 mmol) in 15 mL of EtOAc containing a catalytic amount of 10% Pd/C. Yield: 76 mg (85%). MS (ESI) $(M+H)^+$ 437.02.

**Example 39**

***N*-{2-*tert*-Butyl-1-[(4,4-difluorocyclohexyl)methyl]-1*H*-benzimidazol-5-yl}-*N*-methylbenzenesulfonamide**

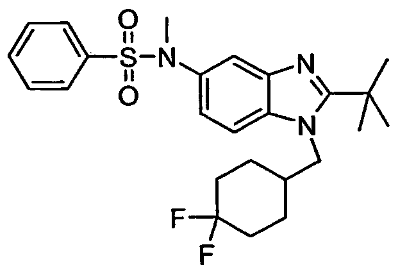

**Step A: N-{2-*tert*-Butyl-1-[(4,4-difluorocyclohexyl)methyl]-1*H*-benzimidazol-5-yl}-*N*-methylbenzenesulfonamide**

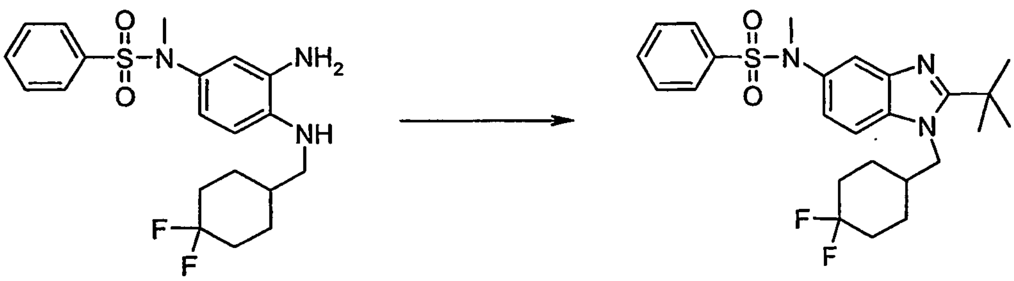

Following the same procedure used as in Step A of Example 35 using *N*-(3-amino-4-{[(4,4-difluorocyclohexyl)methyl] amino}phenyl)-*N*-methylbenzenesulfonamide (for preparation see following Steps B, C, D and E) (61 mg, 0.149 mmol) and trimethylacetyl chloride (0.020 mL, 0.160 mmol) in 5 mL of DCM containing a catalytic amount of DMAP. The product was purified by reversed-phase HPLC using 20-80% $CH_3CN/H_2O$ and lyophilized affording the title compound as the corresponding TFA salt. Yield: 25 mg (28%). $^1H$ NMR (400 MHz, METHANOL-$D_4$): δ 1.57 (s, 2 H), 1.67 (s, 9 H), 1.72

(s, 2 H), 1.76 (m, 1 H), 1.80 (m, 1 H), 2.07 (m, 2 H), 2.24 (m, 1 H), 3.27 (s, 3 H), 4.54 (d, J=7.62 Hz, 2 H), 7.31 (dd, J=8.98, 1.95 Hz, 1 H), 7.54 (m, 5 H), 7.68 (m, 1 H), 7.86 (d, J=8.98 Hz, 1 H); MS (ESI) $(M+H)^+$ 476.0; Anal. Calcd for $C_{25}H_{31}N_3O_2SF_2$ + 1.6 TFA: C, 51.47; H, 4.99; N, 6.39. Found: C, 51.46; H, 5.00; N, 6.53.

**Step B: *tert*-Butyl [(4,4-difluorocyclohexyl)methyl]carbamate**

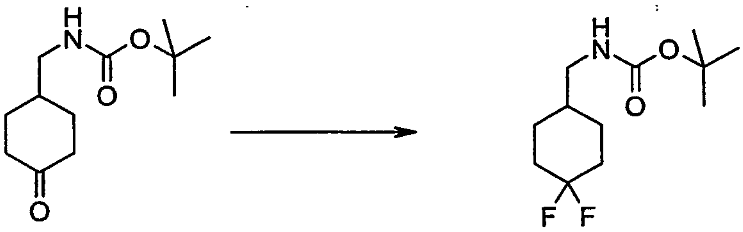

4-N-Boc-aminomethyl cyclohexanone (1.00 g, 4.4 mmol) was dissolved in 30 mL of DCM at 0°C. DAST (1.45 mL, 11.0 mmol) was added dropwise and the solution was stirred at rt overnight. The solution was washed with aqueous 5% $KHSO_4$ solution, saturated aqueous $NaHCO_3$ solution, brine and dried over anhydrous $MgSO_4$. The crude product was purified by silica gel flash chromatography using 3:1 / hexanes : EtOAc as eluent. Yield: 508mg (46%). $^1$H NMR (400 MHz, CHLOROFORM-D): δ 1.19 - 1.36 (m, 2 H), 1.44 (s, 9 H), 1.51 - 1.56 (m, 1 H), 1.59 - 1.75 (m, 2 H), 1.75 - 1.84 (m, 2 H), 2.01 - 2.16 (m, 2 H), 3.03 (t, *J*=6.54 Hz, 2 H), 4.62 (br.s, 1 H).

**Step C: [(4,4-Difluorocyclohexyl)methyl]amine hydrochloride**

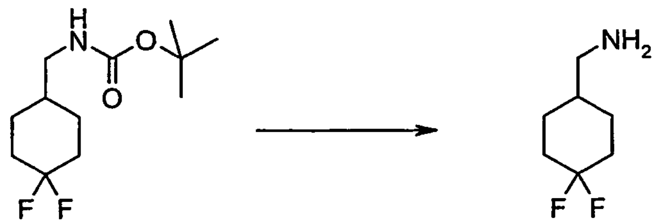

*tert*-Butyl [(4,4-difluorocyclohexyl)methyl]carbamate (505 mg, 2.03 mmol) was stirred in 5 mL of 1M HCl/AcOH at rt for 2h. The solvent was evaporated. The residue was washed with ether, filtered and dried. Yield: 330 mg (88%). $^1$H NMR (400 MHz, METHANOL-$D_4$): δ 1.28 - 1.40 (m, 2 H), 1.71 - 1.82 (m, 2 H), 1.84 (d, *J*=3.12 Hz, 2 H), 1.86 - 1.89 (m, 1 H), 2.03 - 2.15 (m, 2 H), 2.85 (d, *J*=7.03 Hz, 2 H).

**Step D: *N*-(4-{[(4,4-Difluorocyclohexyl)methyl]amino}-3-nitrophenyl)-*N*-methylbenzenesulfonamide**

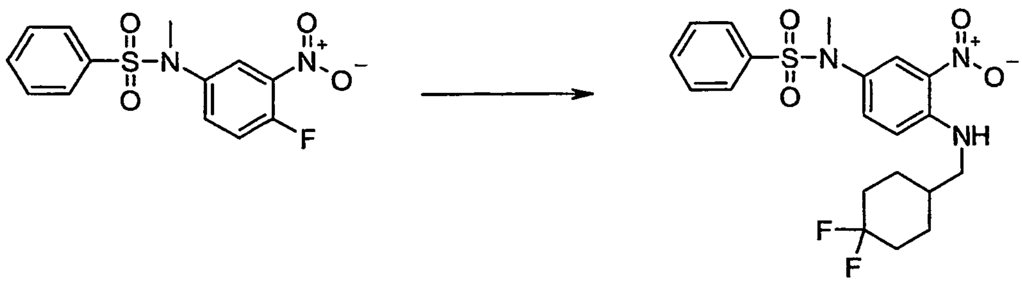

Following the same procedure used as in Example 35 Step B using *N*-(4-fluoro-3-nitrophenyl)-*N*-methylbenzenesulfonamide (for preparation see Example 3, Steps B and C) (50 mg, 0.161 mmol), [(4,4-difluorocyclohexyl)methyl]amine hydrochloride (35 mg, 0.193 mmol) in 3 mL of EtOH containing TEA (0.056 mL, 0.403 mmol). The crude product was purified by silica gel flash chromatography using 40% EtOAc in hexanes as eluent. Yield: 71 mg (99%). $^1$H NMR (400 MHz, CHLOROFORM-D): δ 1.42 (m, 2 H), 1.71 (m, 1 H), 1.80 (m, 2 H), 1.92 (m, 1 H), 1.96 (m, 1 H), 1.96 (m, 1 H), 2.17 (m, 2 H), 3.13 (s, 3 H), 3.24 (dd, J=6.64, 5.66 Hz, 2 H), 6.82 (d, J=9.18 Hz, 1 H), 7.48 (m, 2 H), 7.51 (m ,2 H), 7.61 (m, 3 H), 8.20 (t, J=5.27 Hz, 1 H).

**Step E: *N*-(3-Amino-4-{[(4,4-difluorocyclohexyl)methyl]amino}phenyl)-*N*-methylbenzenesulfonamide**

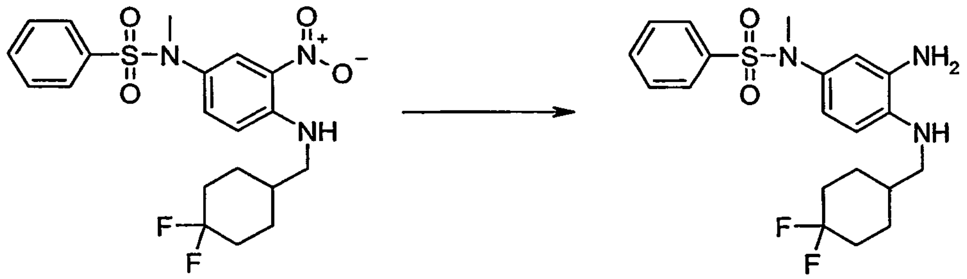

Following the same procedure used as in Example 35 Step C using *N*-(4-{[(4,4-difluorocyclohexyl)methyl]amino}-3-nitrophenyl)-*N*-methylbenzenesulfonamide (65 mg, 0.148 mmol) in 20 mL of EtOAC containing a catalytic amount of 10% Pd/C. Yield: 61mg (99%). MS (ESI) $(M+H)^+$ 410.24.

**Example 40**

***N*-[2-*tert*-Butyl-1-(pyridin-4-ylmethyl)-1*H*-benzimidazol-5-yl]-*N*-methylbenzenesulfonamide**

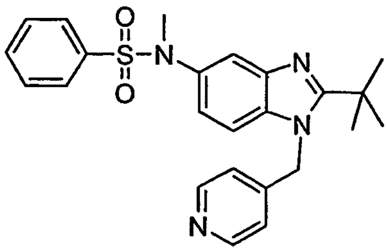

**Step A: *N*-[2-*tert*-Butyl-1-(pyridin-4-ylmethyl)-1*H*-benzimidazol-5-yl]-*N*-methylbenzenesulfonamide**

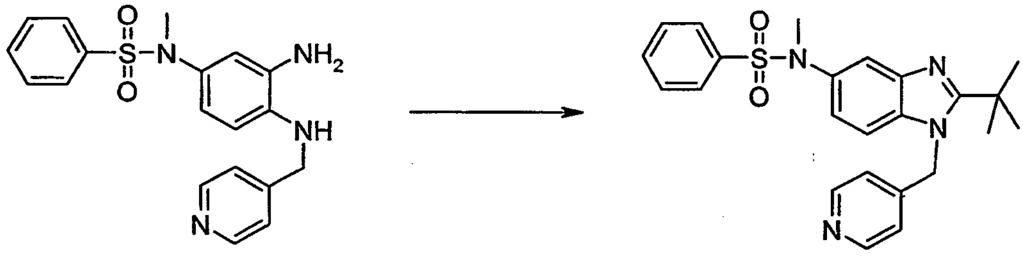

Following the same procedure used as in Step A of Example 35 using *N*-{3-amino-4-[(pyridin-4-ylmethyl)amino]phenyl}-*N*-methylbenzenesulfonamide (for preparation see following Steps B and C) (63 mg, 0.171 mmol) and trimethylacetyl

chloride (0.025 mL, 0.188 mmol) in 5 mL of DCM containing a catalytic amount of DMAP. The product was purified by reversed-phase HPLC using 10-60% $CH_3CN/H_2O$ and lyophilized affording the title compound as the corresponding TFA salt. Yield: 48 mg (51 %). $^1$H NMR (400 MHz, METHANOL-$D_4$): δ 1.57 (s, 9 H), 3.22 (s, 3 H), 6.08 (s, 2 H), 7.13 (dd, J=8.88, 2.05 Hz, 1 H), 7.29 (d, J=8.40 Hz, 1 H), 7.38 (d, J=4.88 Hz, 2 H), 7.49 (m, 4 H), 7.53 (m, 1 H), 7.63 (m, 1 H), 8.62 (m, 2 H); MS (ESI) $(M+H)^+$ 435.0; Anal. Calcd for $C_{24}H_{26}N_4O_2S$ + 2.3 TFA + 0.1 $H_2O$: C, 49.17; H, 4.11; N, 8.02. Found: C, 49.15; H, 4.10; N, 8.08.

**Step B: *N*-Methyl-*N*-{3-nitro-4-[(pyridin-4-ylmethyl)amino]phenyl}benzenesulfonamide**

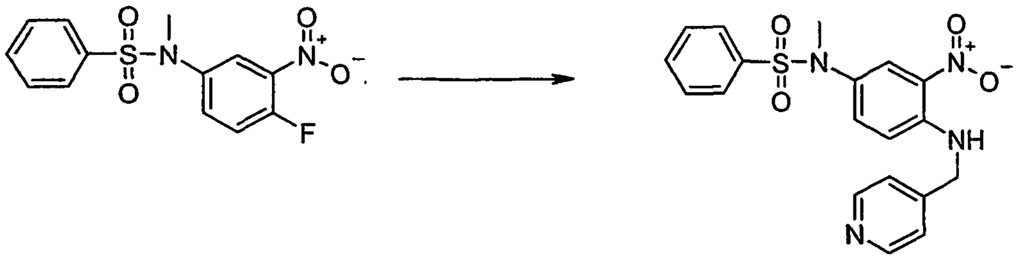

*N*-(4-Fluoro-3-nitrophenyl)-*N*-methylbenzenesulfonamide (for preparation see Example 3, Steps B and C) (105 mg, 0.338 mmol) and 4-(aminomethyl)pyridine (0.070 mL, 0.676 mmol) were stirred in 3 mL of $CH_3CN$ at rt for 24h. The solvent was evaporated. The residue was dissolved in EtOAc and washed with aqueous $NaHCO_3$ solution, brine and dried over anhydrous $MgSO_4$. The crude product was purified by silica gel flash chromatography using EtOAc as eluent. Yield: 102 mg (76%). $^1$H NMR (400 MHz, CHLOROFORM-D): δ 3.13 (s, 3 H), 4.61 (d, *J*=5.08 Hz, 2 H), 6.65 (dd, *J*=9.08, 1.07 Hz, 1 H), 7.25 - 7.29 (m, 2 H), 7.37 (dd, *J*=9.18, 2.73 Hz, 1 H), 7.47 - 7.53 (m, 2 H), 7.56 - 7.59 (m, 2 H), 7.60 - 7.65 (m, 1 H), 7.70 (d, *J*=2.54 Hz, 1 H), 8.50 - 8.54 (m, 1 H), 8.62 (d, *J*=5.47 Hz, 2 H).

**Step C: *N*-{3-Amino-4-[(pyridin-4-ylmethyl)amino]phenyl}-*N*-methylbenzenesulfonamide**

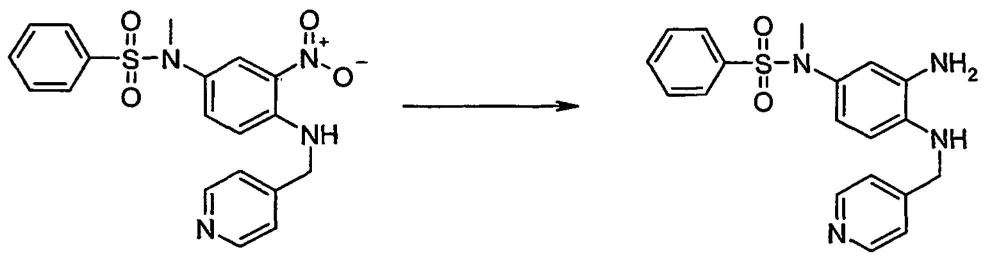

Following the same procedure used as in Example 35 Step C using *N*-methyl-*N*-{3-nitro-4-[(pyridin-4-ylmethyl)amino] phenyl}benzenesulfonamide (96 mg, 0.241 mmol) in 15 mL of EtOAC containing a catalytic amount of 10% Pd/C. Yield: 63 mg (71%). MS (ESI) $(M+H)^+$ 368.94.

**Example 41**

***N*-methyl-*N*-[1-(tetrahydro-2*H*-pyran-4-ylmethyl)-2-(trifluoromethyl)-1*H*-benzimidazol-5-yl]benzenesulfonamide**

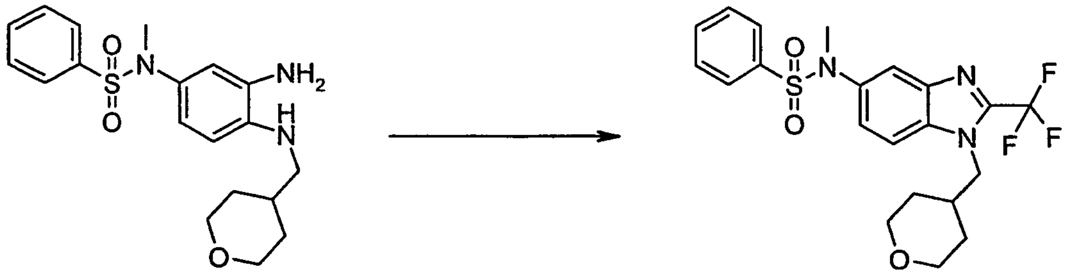

A solution of *N*-{3-amino-4-[(tetrahydro-2*H*-pyran-4-ylmethyl)amino]phenyl}-*N*- methylbenzenesulfonamide (61.2 mg, 0.163 mmol) (for preparation, see the following steps B and C in Example 21) in trifluoroacetic acid (3 mL) was heated for 20 h at reflux. Upon evaporation, the residue was purified by reversed-phase HPLC using 20-70% CH3CN/$H_2O$ and then lyophilized affording the title compound as the corresponding TFA salt. Yield: 38.3 mg (52%). $^1$HNMR (400 MHz, $CD_3OD$): δ 1.40 - 1.50 (m, 4 H), 2.16 - 2.33 (m, 1 H), 3.25 (s, 3 H), 3.31 - 3.41 (m, 2 H), 3.86-4.00 (m, 2 H), 4.32 (d, J=7.62 Hz, 2 H), 7.32 (dd, J=8.88, 2.05 Hz, 1 H), 7.38 (d, J=1.76 Hz, 1 H), 7.48 - 7.58 (m, 4 H), 7.62 - 7.69 (m, 1 H), 7.72 (d, J=8.79 Hz, 1 H). MS (ESI) $(M+H)^+$ = 454.0. Anal. Calcd for $C_{21}H_{22}F_3N_3O_3S$+ 0.20 TFA (476.29): C, 53.97; H, 4.70; N, 8.82; Found: C, 54.01; H, 4.73; N, 9.00.

**Example 42**

***N*-[2-(1,1-dyfluoroethyl)-1-(tetrahydro-2*H*-pyran-4-ylmethyl)-1*H*-benzimidazol-5-yl]-*N*-methylbenzenesulfonamide**

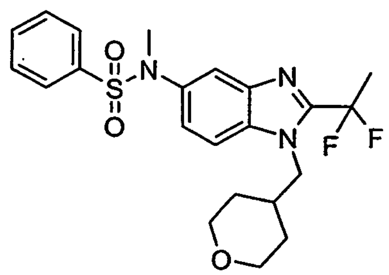

Diisopropylethylamine (56.9 mg, 0.44 mmol) was added into a solution of *N*-{3-amino-4-[(tetrahydro-2*H*-pyran-4-ylmethyl) amino]phenyl}-*N*-methylbenzenesulfonamide (75.3 mg, 0.20 mmol) (for preparation, see the steps B, C, D and E in Example 41) and 2,2-difluoropropionic acid (23.1 mg, 0.21 mmol) in DMF (5 mL) at 0 °C. Stirring for 20 min, HATU (91.3 mg, 0.24 mmol) was added. The reaction mixture was stirred overnight at room temperature. After evaporation of the solvent, the residue was dissolved in acetic acid (10 mL) and heated for 12 h at 90 °C. Upon evaporation of the solvent, the residue was diluted with EtOAc (100 mL), washed with 2*N* NaOH(10 mL), saturated NaCl (2x10 mL) and dried over anhydrous sodium sulphate. After filtration and concentration, the crude product was purified by MPLC using Hex/EtOAc (1:1) on silica gel to give 71.4 mg (79%) of a white solid as the title compound. $^1$HNMR (400 MHz, $CD_3OD$): δ 1.28 - 1.45 (m, 4 H), 2.08 - 2.15 (m, 1 H), 2.21 (t, J=19.53 Hz, 3 H), 3.13 - 3.19 (m, 2 H), 3.19 (s, 3 H), 3.80 (m, 2 H), 4.28 (d, J=7.42 Hz, 2 H), 7.13 (dd, J=8.79, 1.95 Hz, 1 H), 7.36 (d, J=1.95 Hz, 1H), 7.47 7.52 (m, 2 H) 7.53 - 7.63 (m, 2 H) 7.67 - 7.73 (m, 1 H), 7.75 (d, J=8.79 Hz, 1 H). MS (ESI) $(M+H)^+$ = 450.0. Anal. Calcd for $C_{22}H_{25}F_2N_3O_3S$ (449.52): C, 58.78; H, 5.61; N, 9.35; Found: C, 58.94; H, 5.51; N, 8.94.

**Example 43**

***N*-methyl-*N*-[1-(tetrahydro-2*H*-pyran-4-ylmethyl)-2-(2,2,2-trifluoroethyl)-1*H*-benzimidazol-5-yl]benzenesulfonamide**

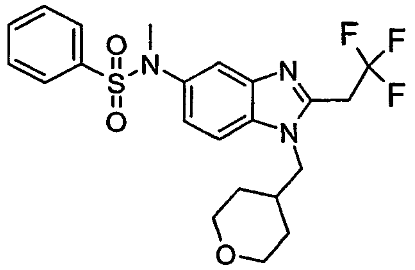

Following the procedure for Example 42, using *N*-{3-Amino-4-[(tetrahydro-2*H* pyran-4-ylmethyl)amino]phenyl}-*N*-methylbenzenesulfonamide (150.6 mg, 0.40 mmol) (for preparation, see the steps B, C D and E in Example 41), diisopropylethylamine (113.8 mg, 0.88 mmol) and 3,3,3-trifluoropropionic acid (56.3 mg, 0.44 mmol) and HATU (182.6 mg, 0.48 mmol) in DMF (10 mL) and then in acetic acid (5 mL), the crude product was purified by MPLC using EtOAc on silica gel to give 46.4 mg (32%) of a light yellow solid as the title compound. $^1$HNMR (400 MHz, $CD_3OD$) δ 1.39 - 1.56 (m, 4 H), 2.12 - 2.30 (m, 1 H), 3.25 (s, 3 H), 3.32 - 3.38 (m, 2 H), 3.88 - 3.96 (m, 2 H), 4.22 (q, J=9.96 Hz, 2 H), 4.31 (d, J=7.62 Hz, 2 H), 7.22 (dd, J=8.89, 2.05 Hz, 1 H), 7.39 (d, J=1.56 Hz, 1 H), 7.49 - 7.59 (m, 4 H) ,7.63 7.68 (m, 1 H), 7.70 (d, J=8.98 Hz, 1 H). MS (ESI) $(M+H)^+$ = 468.0. Anal. Calcd for $C_{22}H_{24}F_3N_3O_3S$+ 1.10 TFA+0.20 $H_2O$ +0.20 $CH_3OH$ (602.95): C, 48.61; H, 4.40; N, 6.97; Found: C, 48.59; H, 4.31; N, 6.85.

**Example 44**

***N*-[1-(cyclohexylmethyl)-2-(1-ethylpropyl)-1*H*-benzimidazol-5-yl]benzenesulfonamide**

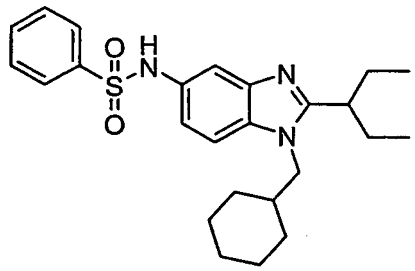

Following the procedure for Step A in Example 7, using *N*-{3-amino-4-[(cyclohexylmethyl)amino]phenyl}benzene sulfonamide (184.0mg, 0.51 mmol), DMAP (15.0 mg, 0.12 mmol) and 2-ethylbutyryl chloride (74.0 mg, 0.55 mmol) in $CH_2Cl_2$ (15 mL) and then in acetic acid (15 mL), the crude product was purified by MPLC using Hex/EtOAc (1:1) on silica gel. Yield: 164.4 mg (73%). $^1$HNMR(400 MHz, $CD_3OD$): δ 0.92 (t, J=7.42 Hz, 6 H), 1.18 (m, 5 H), 1.57 (m, 2 H), 1.73 (m, 3 H), 1.83 (m, 2 H), 1.94 (m, 3 H), 3.33 (m, 1 H), 4.26 (d, J=7.62 Hz, 2 H), 7.25 (m, 1 H), 7.48 (m, 2 H) ,7.57 (m, 2 H), 7.73 (d, J=8.98 Hz, 1 H), 7.81 (m, 2 H). MS (ESI) $(M+H)^+$ = 440.0. Anal. Calcd for $C_{25}H_{33}N_3O_2S$+1.10 TFA+0.10 $H_2O$ (566.85): C, 57.63; H, 6.10; N, 7.41; Found: C, 57.56; H, 6.11; N, 7.45.

**Example 45**

***N*-[1-(cyclohexylmethyl)-2-(1-ethylpropyl)-1*H*-benzimidazol-5-yl]-*N*-methylbenzenesulfonamide**

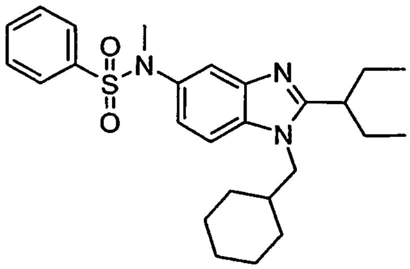

Following the procedure for Example 13, using *N*-[1-(cyclohexylmethyl)-2-(1-ethylpropyl)-1*H*-benzimidazol-5-yl]benzenesulfonamide (85.4 mg, 0.194 mmol) (for preparation, see Example 7), sodium hydride (23.3 mg, 60%, 0.583 mmol) and iodomethane (82.6 mg, 0.582 mmol) in THF(10 mL). Yield: 69.9 mg (79%); white solid for TFA salt. $^1$HNMR (400 MHz, $CD_3OD$) δ 0.95 (t, J=7.42 Hz, 6 H), 1.21 (m, 5 H), 1.61 (m, 2 H), 1.71 (m, 1 H,) 1.76 (m, 2 H), 1.86 (m, 2 H), 1.99 (m, 3 H), 3.27 (s, 3 H), 3.38 (m, 1 H), 4.33 (d, J=7.81 Hz, 2 H), 7.29 (m, 1 H), 7.53 (m, 2 H), 7.57 (m, 3 H), 7.68 (m, 1 H), 7.83 (d, J=8.98 Hz, 1 H). MS (ESI) $(M+H)^+$ = 454.2. Anal. Calcd for $C_{26}H_{35}N_3O_2S$+1.00 TFA+0.20 $H_2O$ (571.28): C, 58.87; H, 6.42; N, 7.36; Found: C, 58.85; H, 6.54; N, 7.24.

## Example 46

**N-[2-*tert*-butyl-1-(cyclohexylmethyl)-1*H*-benzimidazol-5-yl]-*N*-ethylbenzenesulfonamide**

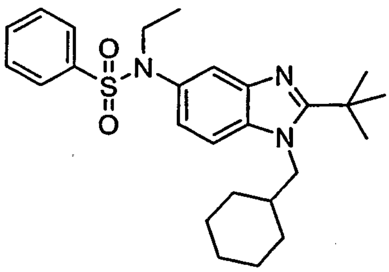

Following the procedure for Example 13, using *N*-[2-*tert*-butyl-1-(cyclohexylmethyl)-1*H*-benzimidazol-5-yl]benzene sulfonamide (52.0 mg, 0.122 mmol) (for preparation, see Example 7), sodium hydride (20.3 mg, 60%, 0.508 mmol) and iodoethane (57.2 mg, 0.367 mmol) in THF (8 mL). Yield: 43.8 mg (79%), white solid for TFA salt. $^1$HNMR (400 MHz, $CD_3OD$): δ 1.08 (t, J=7.13 Hz, 3 H), 1.25 (m, 5 H), 1.62 (m, 2 H), 1.67 (s, 9 H), 1.71 (m, 1 H,) 1.78 (m, 2 H), 2.10 (m, 1 H), 3.74 (q, J=7.03 Hz, 2 H), 4.45 (d, J=7.62 Hz, 2 H), 7.22 (dd, J=8.98, 1.95 Hz, 1 H), 7.49 (d, J=1.56 Hz, 1 H), 7.54 (m, 2 H), 7.60 (m, 2 H), 7.66 (m, 1 H), 7.85 (d, J=8.98 Hz, 1 H). MS (ESI) $(M+H)^+$ = 454.0. Anal. Calcd for $C_{26}H_{35}N_3O_2S$+1.10 TFA+0.20 $H_2O$ (582.68): C, 58.13; H, 6.31; N, 7.21; Found: C, 58.15; H, 6.41; N, 6.99.

## Example 47

**N-methyl-*N*-[2-(1-methyl-1-pyridin-2-ylethyl)-1-(tetrahydro-2*H*-pyran-4-ylmethyl)-1*H*-benzimidazol-5-yl]benzenesulfonamide**

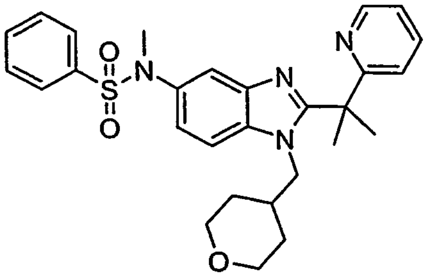

**Step A. *N*-methyl-*N*-[2-(1-methyl-1-pyridin-2-ylethyl)-1-(tetrahydro-2*H*-pyran-4-ylmethyl)-1*H*-benzimidazol-5-yl] benzenesulfonamide**

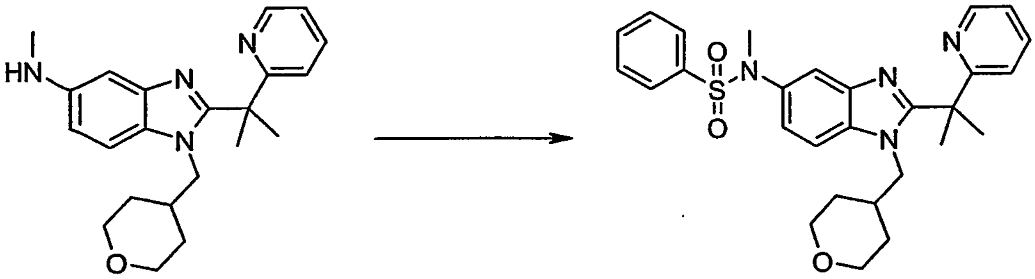

*N*-methyl-2-(1-methyl-1-pyridin-2-ylethyl)-1-(tetrahydro-2*H*-pyran-4-ylmethyl)-1*H*-benzimidazol-5-amine hydrochloride (85.0 mg, 0.13 mmol) (for preparation, see the following steps B, C, D, E, F, G and H), DMAP (64.0 mg, 0.53 mmol) and benzenesulfonyl chloride (46.0 mg, 0.26 mmol) in MeCN (5 mL) were stirred for 8 h at room temperature. The reaction mixture was quenched with $H_2O$ (3 mL). Upon evaporation, the residue was purified by reversed-phase HPLC using 15-75% $CH_3CN/H_2O$ and then lyophilized affording the title compound as the corresponding TFA salt. Yield: 55.0 mg (68%). $^1$HNMR (400 MHz, $CD_3OD$) δ 1.02 - 1.22 (m, 5 H), 2.01 (s, 6 H), 2.96 - 3.10 (m, 2 H), 3.28 (s, 3 H), 3.70 - 3.81 (m, 2 H), 3.93 (d, J=6.83 Hz, 2 H), 7.25 (dd, J=8.98, 2.15 Hz, 1 H), 7.39 - 7.47 (m, 1 H), 7.50 - 7.60 (m, 5 H), 7.64 - 7.76 (m, 4 H), 7.91 - 7.99 (m, 1 H). MS (ESI) $(M+H)^+$ = 505.0. Anal. Calcd for $C_{28}H_{32}N_4O_3S$+ 1.0 TFA+0.2 $H_2O$+0.4 $CH_3OH$ (635.10): C, 57.49; H, 5.55; N, 8.82; Found: C, 57.52; H, 5.46; N, 8.72.

**Step B. *N*-(4-fluoro-3-nitrophenyl)acetamide**

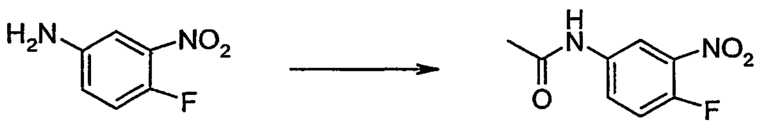

4-Fluoro-3-nitro-aniline (45.0 g, 0.288 mol) was added in portions to acetic anhydride (150 mL) at room temperature. The reaction mixture was stirred at room temperature for 2 h. The white solid was collected and dried *in vacuo* to give the title compound (42.0 g, 70%). $^1$H NMR (400 MHz, $CDCl_3$): δ 2.23 (s, 3 H), 7.26 (m, 1 H), 7.50 (s broad, 1 H), 7.87 (m, 1 H), 8.23 (dd, *J*=6.44, 2.73 Hz, 1 H).

**Step C. *N*-(4-fluoro-3-nitrophenyl)-*N*-methylacetamide**

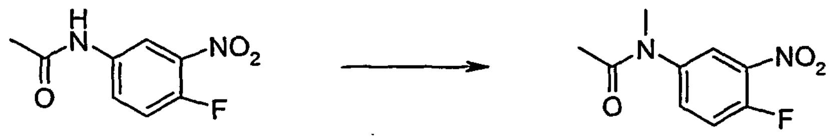

Sodium hydride (2.40g, 60%, 60 mmol) was added in portions to a solution of *N*-(4-fluoro-3-nitrophenyl)acetamide(7.93 g, 40 mmol) in THF (120 mL) at 0 °C. Stirring for 20 min, iodomethane (17.0 g, 120 mmol) was added. The reaction mixture was stirred at room temperature for 2 h, quenched with saturaed $NaHCO_3$ (30 mL) and extracted with EtOAc (3x100 mL). The combined organic phases were washed with saturated NaCl (2x30 mL). After filtration and concentration, 8.73 g (100%) of the title compound was obtained as a brown solid. $^1$H NMR (400 MHz, $CDCl_3$): δ 1.92 (s, 3 H), 3.30 (s, 3 H), 7.38 (s, 1 H), 7.52 (s, 1 H), 7.95 (s, 1 H).

**Step D. *N*-methyl-*N*-{3-nitro-4-[(tetrahydro-2*H*-pyran-4-ylmethyl)amino]phenyl}acetamide**

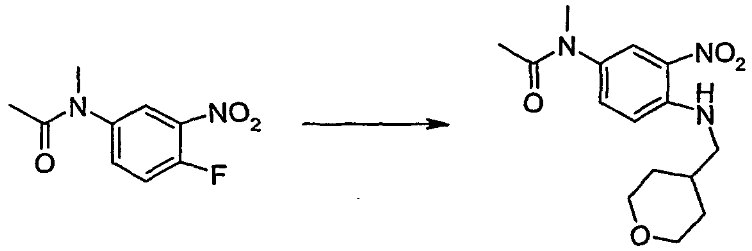

4-Aminomethylpyran (2.50 g, 21.7 mmol) was added to a mixture of *N*-(4-fluoro-3-nitrophenyl)-*N*-methylacetamide (4.61 g, 21.27 mmol) and sodium carbonate (5.10 g, 47.7 mmol) in EtOH (120 mL) at room temperature. The reaction mixture was heated for 3 days at 60 °C. Upon evaporation of ethanol, the residue was dissolved in EtOAc (400 mL), washed with $H_2O$ (3x50 mL), saturated NaCl (3x50 mL), and dried over $Na_2SO_4$. After filtation and concentration, 6.62 g (100%) of the title compound was obtained as an orange-red solid. $^1$H NMR (400 MHz, $CDCl_3$): δ 1.38 - 1.52 (m, 2 H), 1.72 - 1.81 (m, 2 H), 1.90 (s, 3 H), 1.93 - 2.02 (m, 1 H), 3.23 (s, 3 H), 3.23 - 3.27 (m, 2 H), 3.36 - 3.49 (m, 2 H), 4.01 - 4.07 (m, 2 H), 6.91 (d, *J*=9.18 Hz, 1 H), 7.29 (dd, *J*=9.08, 2.64 Hz, 1 H), 8.05 (d, *J*=2.34 Hz, 1 H), 8.22 (t, *J*=5.37 Hz, 1 H). MS (ESI) $(M+H)^+$ = 309.12.

**Step E. *N*-{3-amino-4-[(tetrahydro-2*H*-pyran-4-ylmethyl)amino]phenyl}-*N*-methylacetamide**

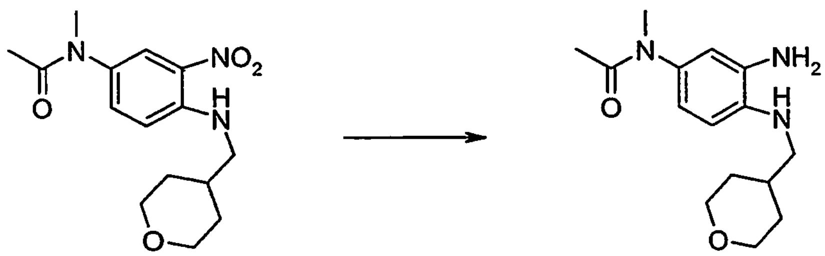

*N*-methyl-*N*-{3-nitro-4-[(tetrahydro-2*H*-pyran-4-ylmethyl)amino]phenyl} acetamide (5.39 g,16.7 mmol) was hydrogenated in ethyl acetate (200 mL) catalyzed by 10% Pd/C (0.2 g) at 30-40 psi $H_2$ in Parr shaker for 18 h at room temperature. After filtration through celite and concentration, 6.0 g (100%) of a purple solid was obtained as HCl salt, which was used in the next step without purification. $^1$H NMR (400 MHz, $CD_3OD$): δ 1.32 - 1.46 (m, 2 H), 1.78 - 1.84 (m, 2 H), 1.85 (s, 3 H), 1.91 - 2.06 (m, 1 H), 3.16 (d, *J*=6.83 Hz, 2 H), 3.20 (s, 3 H), 3.39 - 3.51 (m, 2 H), 3.94 - 4.03 (m, 2 H), 7.01 (d, *J*=8.59 Hz, 1 H), 7.12 (d, *J*=2.15 Hz, 1 H), 7.17 (dd, *J*=8.49,4.39 Hz, 1 H), MS (ESI) $(M+H)^+$: 278.7

**Step F. *N*-methyl-*N*-[2-(pyridin-2-ylmethyl)-1-(tetrahydro-2*H*-pyran-4-ylmethyl)-1*H*-benzimidazol-5-yl]acetamide**

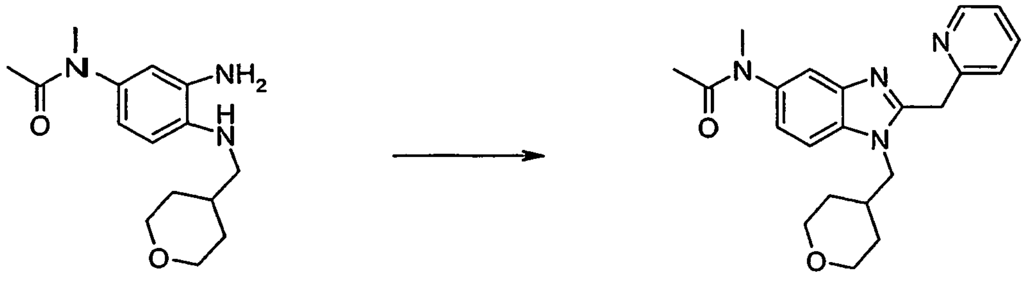

Following the procedure for Example 10, using *N*-{3-amino-4-[(tetrahydro-2*H*-pyran-4-ylmethyl)amino]phenyl}-*N*-methylacetamide hydrochoride (416.1 mg, 1.33 mmol), 2-pyridylacetic acid hydrochloride (286.4 mg, 1.65 mmol), diisopropylethylamine (970 mg, 7.5 mmol) and HATU (680.0 mg, 1.80 mmol) in DMF (15 mL) and then in acetic acid (10 mL), the crude product was purified by MPLC using EtOAc/MeOH (20:1) on silica gel to give 308.1 mg (61%) of a yellow solid as the title compound. MS (ESI) $(M+H)^+$ = 379.0.

**Step G. *N*-methyl-*N*-[2-(1-methyl-1-pyridin-2-ylethyl)-1-(tetrahydro-2*H*-pyran-4-ylmethyl)-1*H*-benzimidazol-5-yl]acetamide**

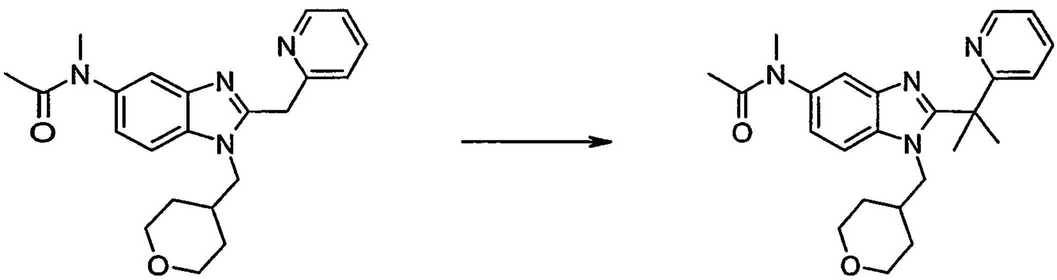

KHMDS (1.6 mL, 0.5 *M*, 0.8 mmol) was added to a solution of *N*-methyl-*N*-[2-(pyridin-2-ylmethyl)-1-(tetrahydro-2*H*-pyran-4-ylmethyl)-1*H*-benzimidazol-5-yl]acetamide (248.4 mg, 0.656 mmol) in THF (25 mL) at -78 °C. Stirring for 10 min, iodomethane (113.6 mg, 50 uL, 0.80 mmol) was added. The mixture was stirred for 30 min at -78 °C and 30 min at room temperature, then cooled down to -78 °C again. Another 1.2 equivalent KHMDS and iodomethane were added. The resulting mixture was stirred for 30 min at -78 °C and 45 min at room temperature, quenched with saturated $NaHCO_3$ (5 mL), and extracted with EtOAc (3x20 mL). The combined organic phases were washed with saturated $NaHCO_3$ (20 mL), saturated NaCl (20 mL) and dried over $Na_2SO_4$. After filtration and concentration, the residue was purified by MPLC using EtOAc/MeOH (20:1) on silica gel to give 218.1 mg (90%) of the title compound as a white solid. $^1H$ NMR (400 MHz, $CDCl_3$): δ 1.02 - 1.12 (m, 2 H), 1.13 - 1.19 (m, 2 H), 1.19 - 1.27 (m, 1 H), 1.90 (s, 3 H), 1.97 (s, 6 H), 2.90 - 3.11 (m, 2 H), 3.31 (s, 3 H), 3.68 (d, *J*=7.22 Hz, 2 H), 3.81 (m, 2 H), 7.04 (dd, *J*=8.49, 2.05 Hz, 1 H), 7.18 - 7.32 (m, 3 H), 7.57 - 7.70 (m, 2 H), 8.53 - 8.70 (m, 1 H). MS (ESI) $(M+H)^+$=407.03.

**Step H. *N*-methyl-2-(1-methyl-1-pyridin-2-ylethyl)-1-(tetrahydro-2*H*-pyran-4-ylmethyl)-1*H*-benzimidazol-5-amine**

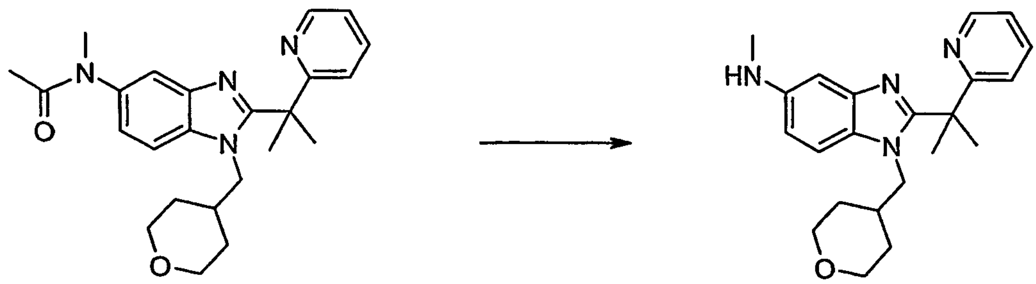

*N*-methyl- *N*-[2-(1- methyl- 1- pyridin- 2- ylethyl)- 1-(tetrahydro- 2*H*-pyran- 4- ylmethyl)- 1H- benzimidazol- 5- yl] acetamide (214.0 mg, 0.526 mmol) was dissolved in 5 mL of EtOH-2*N* HCl (3:2), and then heated at 120°C in a Personal Chemistry SmithSynthesizer microwave instrument for 1h. After concentration and dried in *vacuo*, 331 mg (100%) of a grey white solid was obtained as the title product. $^1$H NMR (400 MHz, DMSO-$D_6$): δ 0.86 - 1.08 (m, 4 H), 1.94 (s, 6 H), 1.96 - 2.03 (m, 1 H), 2.71 - 2.92 (m, 5 H), 3.55 - 3.70 (m, 2 H), 3.86 (d, *J*=5.47 Hz, 2 H), 7.31 - 7.48 (m, 2 H), 7.69 (d, *J*=7.42 Hz, 1 H), 7.74 - 7.84 (m, 1 H), 7.93 (t, *J*=8.30 Hz, 1 H), 8.48 (d, *J*=4.10 Hz, 2 H). MS (ESI) $(M+H)^+$ = 365.04.

**Example 48**

***N*-[2-(1-cyano-1-methylethyl)-1-(tetrahydro-2*H*-pyran-4-ylmethyl)-1*H*-benzimidazol-5-yl]-*N*-methylbenzenesulfonamide**

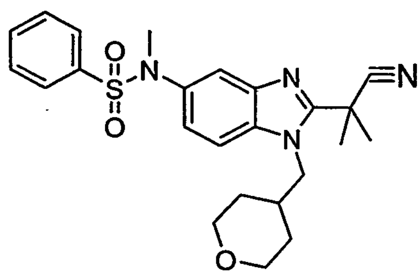

Following the procedure for Example 10, using *N*-{3-amino-4-[(tetrahydro-2*H*-pyran-4-ylmethyl)amino]phenyl}-*N*-methylbenzenesulfonamide (1.0 g, 2.66 mmol) (for preparation, see the step C in Example 21), 2-cyano-2-methylpropanoic acid (0.33 g, 2.93 mmol), diisopropylethylamine (0.76 g, 5.85 mmol) and HATU (1.21 g, 3.19 mmol) in DMF (30 mL) and then in acetic acid (50 mL), the crude product was purified by MPLC using Hex/EtOAc (1:1) on silica gel to give 0.41 g (34%) of a white solid as the title compound. $^1$HNMR (400 MHz, $CD_3OD$): δ 1.43 - 1.53 (m, 2 H), 1.54 - 1.67 (m, 2 H), 1.93 (s, 6 H), 2.38 - 2.55 (m, 1 H), 3.24 (s, 3 H), 3.32 - 3.40 (m, 2 H), 3.93 (m, 2 H), 4.44 (d, J=7.62 Hz, 2 H), 7.13 (dd, J=8.79, 2.15 Hz, 1 H), 7.32 (d, J=1.56 Hz, 1 H), 7.47 - 7.57 (m, 4 H), 7.62 (d, J=8.98 Hz, 1 H), 7.64 - 7.69 (m, 1 H). MS (ESI) $(M+H)^+$ = 453.0. Anal. Calcd for $C_{24}H_{28}N_4O_3S$+ 0.8 TFA+0.2 $H_2O$ (547.40): C, 56.17; H, 5.38; N, 10.24; Found: C, 56.05; H, 5.29; N, 10.44.

**Example 49**

***N*-methyl-*N*-[2-propyl-1-(tetrahydro-2*H*-pyran-4-ylmethyl)-1*H*-benzimidazol-5-yl]benzenesulfonamide**

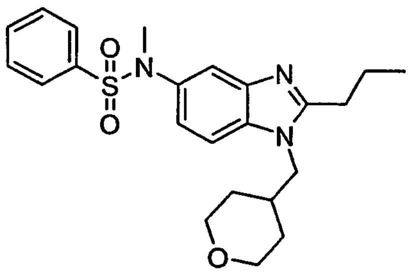

Following the procedure for Example 10, using *N*-{3-amino-4-[(tetrahydro-2*H*-pyran-4-ylmethyl)amino]phenyl}-*N*-methylbenzenesulfonamide (75.3 mg, 0.20 mmol) (for preparation, see the step C in Example 21), butyric acid (18.5 mg, 0.21 mmol), diisopropylethylamine (56.9 mg, 0.44 mmol) and HATU (91.3 mg, 0.24 mmol) in DMF (5 mL) and then in acetic acid (5 mL), the crude product was purified by reversed-phase HPLC using 20-50% $CH_3CN/H_2O$ and then lyophilized affording the title compound as the corresponding TFA salt. Yield: 60.5 mg (71%). $^1$HNMR (400 MHz, $CD_3OD$) δ 1.14 (t, J=7.42 Hz, 3 H), 1.43 - 1.65 (m, 4 H), 1.88 - 2.04 (m, 2 H), 2.15 - 2.35 (m, 1 H), 3.16 - 3.24 (m, 2 H), 3.27 (s, 3 H), 3.31 - 3.42 (m, 2 H), 3.88 - 3.99 (m, 2 H), 4.36 (d, J=7.62 Hz, 2 H), 7.31 (dd, J=8.98, 1.95 Hz, 1 H), 7.47 - 7.59 (m, 5 H,) 7.63 - 7.73 (m, 1 H), 7.86 (d, J=8.98 Hz, 1 H). MS (ESI) $(M+H)^+$ = 428.0. Anal. Calcd for $C_{23}H_{29}N_3O_3$ 1.80 TFA+2.3 $H_2O$ +0.60 $CH_3CN$ (698.88): C, 47.78; H, 5.3 7; N, 7.21; Found: C, 47.76 ; H, 5.38; N, 7.20.

**Example 50**

***N*-[2-butyl-1-(tetrahydro-2*H*-pyran-4-ylmethyl)-1*H*-benzimidazol-5-yl]-*N*-methylbenzenesulfonamide**

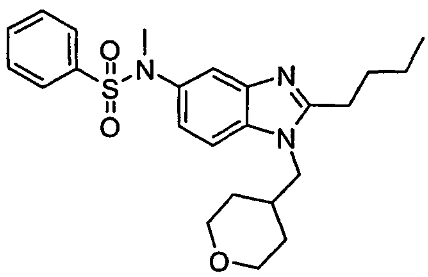

Following the procedure for Example 10, using *N*-{3-amino-4-[(tetrahydro-2*H*-pyran-4-ylmethyl)amino]phenyl}-*N*-methylbenzenesulfonamide (75.3 mg, 0.20 mmol) (for preparation, see the step C in Example 21), pentanoic acid (21.5 mg, 0.21 mmol), diisopropylethylamine (56.9 mg, 0.44 mmol) and HATU (91.3 mg, 0.24 mmol) in DMF (5 mL) and then in acetic acid (5 mL), the crude product was purified by MPLC using Hex/EtOAc (1:1) on silica gel to give 56.4 mg (64%) of a white solid as the title compound. $^1$HNMR (400 MHz, $CD_3OD$) δ 1.05 (t, J=7.32 Hz, 3 H), 1.45 - 1.63 (m, 6 H), 1.85 - 1.99 (m, 2 H), 2.15 - 2.32 (m, 1 H,) 3.20 - 3.26 (m, 2 H), 3.27 (s, 3 H), 3.31 - 3.41 (m, 2 H), 3.85 - 4.05 (m, 2 H), 4.37 (d, J=7.62 Hz, 2 H), 7.32 (dd, J=8.98, 1.95 Hz, 1 H), 7.47 - 7.60 (m, 5 H), 7.63 - 7.73 (m, 1 H), 7.87 (d, J=8.79 Hz, 1 H). MS (ESI) $(M+H)^+$ = 442.0. Anal. Calcd for $C_{24}H_{31}N_3O_3S$+ 1.00 HCl+1.00 $H_2O$ (496.07): C, 58.11; H, 6.91; N, 8.47; Found: C, 58.14 ; H, 6.92; N, 8.30.

**Example 51**

***N*-[2-*tert*-butyl-1-(tetrahydro-2*H*-pyran-4-ylmethyl)-1*H*-benzimidazol-5-yl]-*N*-ethylbenzenesulfonamide**

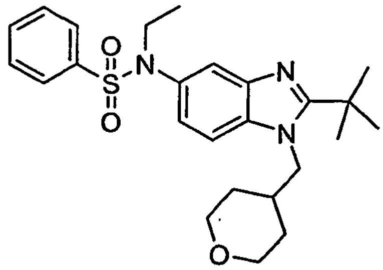

**Step A. *N*-[2-*tert*-butyl-1-(tetrahydro-2*H*-pyran-4-ylmethyl)-1*H*-benzimidazol-5-yl]-*N*-ethylbenzenesulfonamide**

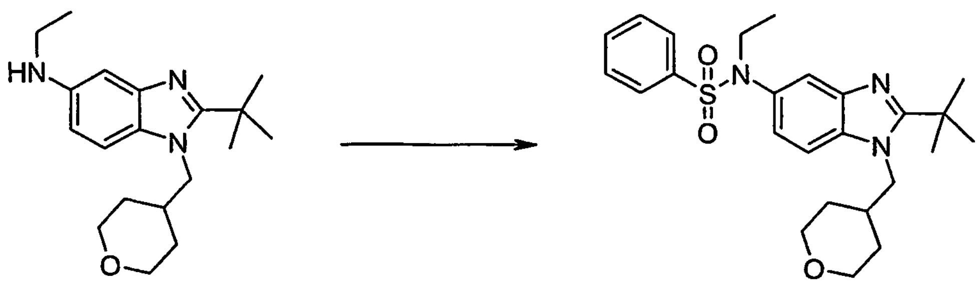

2-*tert*-butyl-*N*-ethyl-1-(tetrahydro-2*H*-pyran-4-ylmethyl)-1*H*-benzimidazol-5-amine hydrochloride (52.8 mg, 0.15 mmol) (for preparation, see the following steps B, C, D, E and F), DMAP (73.3 mg, 0.60 mmol) and benzenesulfonyl chloride (53.0 mg, 0.30 mmol) in MeCN (5 mL) were stirred overnight at room temperature. The reaction mixture was diluted with EtOAc (100 mL), washed with saturated $NaHCO_3$ (10 mL) and saturated NaCl (10 mL) and dried over $Na_2SO_4$. Upon evaporation, the residue was purified by MPLC using Hex/EtOAc (1:1) on silica gel to give 53.0 mg (77%) of a white solid as the title compound. $^1$HNMR (400 MHz, $CD_3OD$) δ 0.95 (t, J=7.03 Hz, 3 H), 1.36 - 1.50 (m, 4 H), 1.53 (s, 9 H), 2.08 - 2.29 (m, 1 H), 3.12 - 3.27 (m, 2 H), 3.65 (q, J=7.09 Hz, 2 H), 3.76 - 3.88 (m, 2 H), 4.37 (d, J=7.03 Hz, 2 H), 7.03 (d, J=8.79 Hz, 1 H), 7.32 (s, 1 H), 7.51 - 7.63 (m, 4 H), 7.64 - 7.76 (m, 1 H), 7.83 (d, J=8.59 Hz, 1 H). MS (ESI) $(M+H)^+$ = 456.0 . Anal. Calcd for $C_{25}H_{33}N_3O_3S$+ 1.20 TFA+0.3 $CH_3CN$ (604.77): C, 55.61; H, 5.85; N, 7.64; Found: C, 55.57; H, 5.79; N, 7.61.

**Step B. *N*-ethyl-*N*-(4-fluoro-3-nitrophenyl)acetamide**

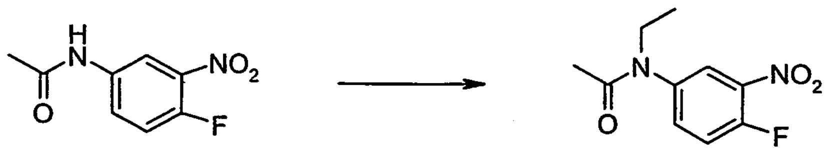

Sodium hydride (1.20g, 60%, 30 mmol) was added in portions to a solution of *N*-(4-fluoro-3-nitrophenyl)acetamide(3.96 g, 20 mmol) (for preparation see the step B in Example 47) in THF (100 mL) at 0 °C. Stirring for 20 min, iodoethane (9.32 g, 60 mmol) was added. The reaction mixture was stirred overnight at room temperature, quenched with saturated $NaHCO_3$ (30 mL) and extracted with EtOAc (3x100 mL). The combined organic phases were washed with saturated NaCl (2x30 mL). After filtration and concentration, the residue was purified by MPLC using Hex/EtOAc (1:1) on silica gel to give 2.36 g (52%) of a yellow solid as the title compound. $^1$H NMR (400 MHz, $CDCl_3$): δ 1.14 (t, J=6.93 Hz, 3 H), 1.88 (s, 3 H), 3.70 - 3.84 (q, J=7.0 Hz, 2 H), 7.34 - 7.43 (m, 1 H), 7.48 (s, 1 H), 7.87 - 7.98 (m, 1 H).

**Step C. *N*-ethyl-*N*-{3-nitro-4-[(tetrahydro-2*H*-pyran-4-ylmethyl)amino]phenyl}acetamide**

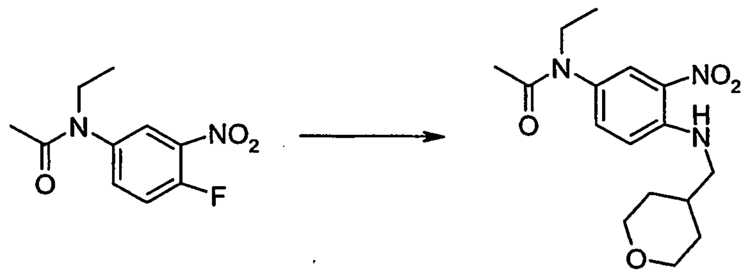

4-Aminomethylpyran (1.32 g, 11.4 mmol) was added to a mixture of *N*-ethyl-*N*-(4-fluoro-3-nitrophenyl)acetamide (2.36 g, 10.4 mmol) and sodium carbonate (2.43 g, 22.9 mmol) in EtOH (70 mL) at room temperature. The reaction mixture was heated for a weekend at 60°C. Upon evaporation of ethanol, the residue was diluted with $H_2O$ (50 mL), and extracted with EtOAc (3x100 mL). The combined organic phases weer washed saturated NaCl (2x50 mL) and dried over $Na_2SO_4$. After filtation and concentration, the residue was purified by MPLC using Hex/EtOAc (1:1) on silica gel to give 2.83 g (85%) of an orange-red solid as the title compound. $^1H$ NMR (400 MHz, $CDCl_3$): δ 1.11 (t, *J*=7.13 Hz, 3 H), 1.38 - 1.52 (m, 2 H), 1.78 (m, 2 H), 1.86 (s, 3 H), 1.92 - 2.04 (m, 1 H), 3.20 - 3.29 (m, 2 H), 3.39 - 3.49 (m, 2 H), 3.71 (q, *J*=7.09 Hz, 2 H), 4.00 - 4.08 (m, 2 H), 6.91 (d, *J*=8.98 Hz, 1 H), 7.24 (d, *J*=2.54 Hz, 1 H), 8.01 (d, *J*=2.54 Hz, 1 H), 8.22 (t, *J*=4.98 Hz, 1 H).

**Step D. *N*-{3-amino-4-[(tetrahydro-2*H*-pyran-4-ylmethyl)amino]phenyl}-*N*-ethylacetamide**

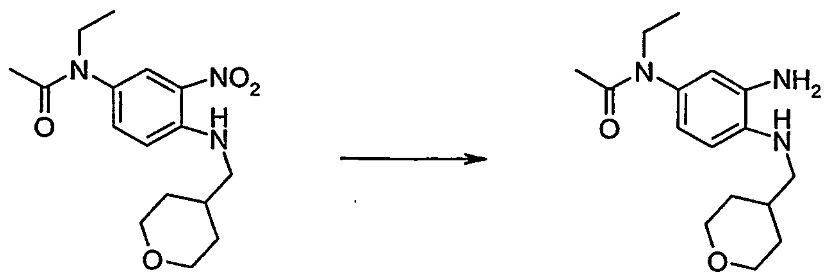

*N*-ethyl-*N*-{3-nitro-4-[(tetrahydro-2*H*-pyran-4-ylmethyl)amino]phenyl}acetamide (2.83 g, 8.79 mmol) was hydrogenated in ethyl acetate (200 mL) catalyzed by 10% Pd/C (0.2 g) at 30-40 psi $H_2$ in Parr shaker for 16 h at room temperature. After filtration through celite and concentration, 2.45 g (95%) of a light yellow solid was obtained, which was used in the next step without purification. MS (ESI) $(M+H)^+$ = 292.3

**Step E. *N*-[2-*tert*-butyl-1-(tetrahydro-2*H*-pyran-4-ylmethyl)-1*H*-benzimidazol-5-yl]-*N*-ethylacetamide**

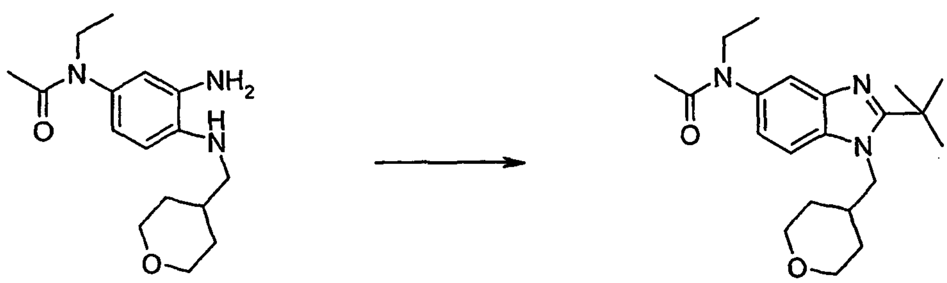

Following the procedure for Step A in Example 7, using *N*-{3-amino-4-[(tetrahydro-2*H*-pyran-4-ylmethyl)amino]phenyl}-*N*-ethylacetamide (803.1 mg, 2.75 mmol), DMAP (671.9 mg, 5.50 mmol) and trimethylacetyl chloride (380.9 mg, 3.16 mmol) in $CH_2Cl_2$ (60 mL) and then in DCE (30 mL), the crude product was purified by MPLC using EtOAc/MeOH (20:1) on silica gel. Yield: 694.1 mg (71%). $^1$H NMR (400 MHz, $CDCl_3$): δ 1.12 (t, *J*=7.13 Hz, 3 H), 1.51 1.57 (m, 4 H), 1.58 (s, 9 H), 1.83 (s, 3 H), 2.21 - 2.40 (m, 1 H), 3.26 - 3.43 (m, 2 H), 3.78 (q, *J*=7.23 Hz, 2 H), 3.94-4.07 (m, 2 H), 4.22 (d, *J*=7.42 Hz, 2 H), 7.02 (dd, *J*=8.59,1.95 Hz, 1 H), 7.34 (d, *J*=8.59 Hz, 1 H), 7.54 (d, *J*=0.98 Hz, 1 H). MS (ESI) $(M+H)^+$ = 358.07.

**Step F. 2-*tert*-butyl-*N*-ethyl-1-(tetrahydro-2*H*-pyran-4-ylmethyl)-1*H*-benzimidazol-5-amine**

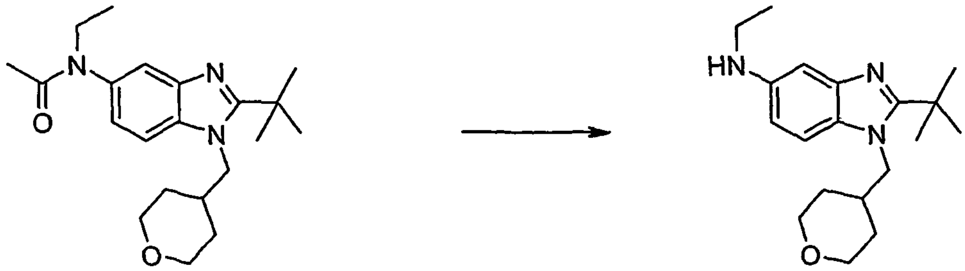

*N*-[2-*tert*-butyl-1-(tetrahydro-2*H*-pyran-4-ylmethyl)-1*H*-benzimidazol-5-yl]-*N*-ethylacetamide (648.3 mg, 2.06 mmol) was dissolved in 15 mL of EtOH-2N HCl (3:2), and then heated at 120°C in a Personal Chemistry SmithSynthesizer microwave instrument for 3h. After concentration and dried *in vacuo,* 754.71 mg (100%) of a grey white solid was obtained as the title product. MS (ESI) $(M+H)^+$ = 316.3.

**Example 52**

***N*-ethyl-*N*-[2-(1-methoxy-1-methylethyl)-1-(tetrahydro-2*H*-pyran-4-ylmethyl)-1*H*-benzimidazol-5-yl]benzenesulfonamide**

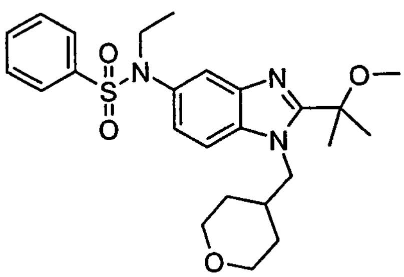

**Step A *N*-ethyl-*N*-[2-(1-methoxy-1-methylethyl)-1-(tetrahydro-2*H*-pyran-4-ylmethyl)-1*H*-benzimidazol-5-yl]benzenesulfonamide**

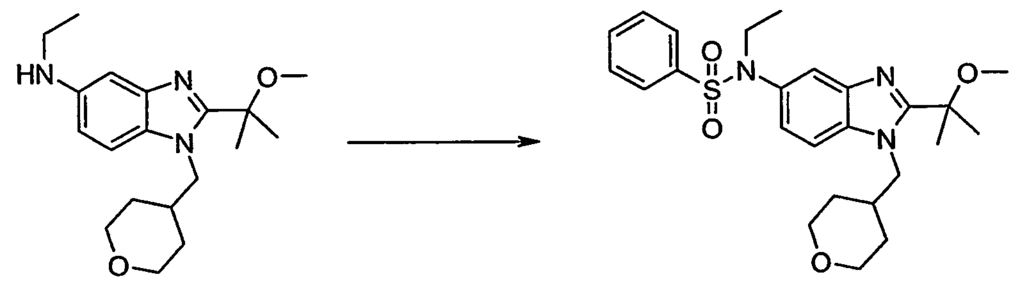

Following the procedure for the step A in Example 8), using *N*-ethyl-2-(1-methoxy-1-methylethyl)-1-(tetrahydro-2*H*-pyran-4-ylmethyl)-1*H*-benzimidazol-5-amine hydrochloride (50.0 mg, 0.136 mmol) (for preparation, see the following steps B, C and D), DMAP (64.5 mg, 0.50 mmol) and benzenesulfonyl chloride (45.9.0 mg, 0.26 mmol) in MeCN (5 mL), the crude product was purified by reversed-phase HPLC using 20-50% $CH_3CN/H_2O$ and then lyophilized affording the title compound as the corresponding TFA salt. Yield: 12.1 mg (19%). $^1$HNMR (400 MHz, $CD_3OD$): δ 1.07 (t, J=7.13 Hz, 3 H), 1.48 - 1.58 (m, 4 H), 1.78 (s, 6 H), 2.27 - 2.51 (m, 1 H), 3.32 (s, 3 H), 3.33 - 3.40 (m, 2 H), 3.72 (q, J=7.23 Hz, 2 H), 3.88 - 4.00 (m, 2 H), 4.51 (d, J=7.42 Hz, 2 H), 7.15 (dd, J=8.88, 1.86 Hz, 1 H), 7.40 (d, J=1.76 Hz, 1 H), 7.49 - 7.58 (m, 2 H), 7.59 - 7.64 (m, 2 H), 7.63 - 7.70 (m, 1 H), 7.76 (d, J=8.98 Hz, 1 H). MS (ESI) $(M+H)^+$ = 472.0. Anal. Calcd for $C_{25}H_{33}N_3O_4S$+ 0.90 TFA+0.20 $H_2O$ +0.40 $CH_3OH$ (590.66): C, 55.31; H, 6.13; N, 7.11; Found: C, 55.29; H, 6.06; N, 7.10.

**Step B. *N*-ethyl-*N*-[2-(1-hydroxy-1-methylethyl)-1-(tetrahydro-2*H*-pyran-4-ylmethyl)-1*H*-benzimidazol-5-yl] acetamide**

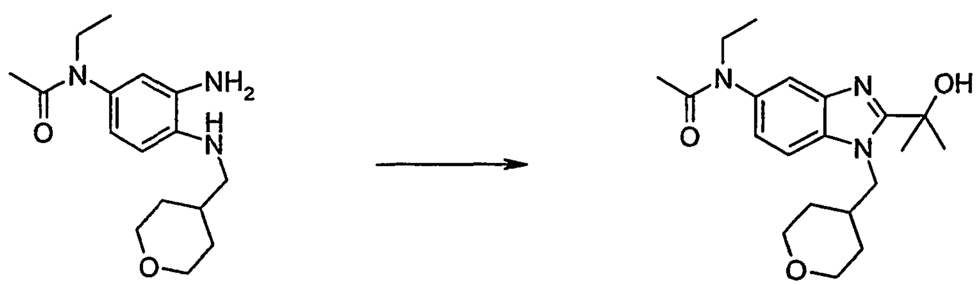

Following the procedure for Example 10, using *N*-{3-amino-4-[(tetrahydro-2*H*-pyran-4-ylmethyl)amino]phenyl}-*N*-ethylacetamide (0.841 g, 2.88 mmol) (for preparation, see the steps B, C and D in Example 51), 2-hydroxy-2-methylpropanoic acid (0.330 g, 3.17 mmol), diisopropylethylamine (0.558 g, 4.32 mmol) and HATU (1.31 g, 3.46 mmol) in DMF (40 mL) and then in acetic acid (50 mL), the crude product (1.78 g, purity >43%) was used directly for next step without purification. MS (ESI) $(M+H)^+$ = 360.04.

**Step C. *N*-ethyl-*N*-[2-(1-methoxy-1-methylethyl)-1-(tetrahydro-2*H*-pyran-4-ylmethyl)-1*H*-benzimidazol-5-yl] acetamide**

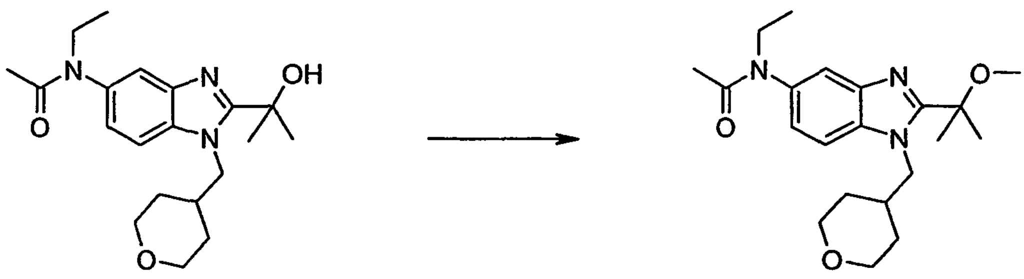

Sodium hydride (0.35 g, 60%, 8.64 mmol) was added in portions to a solution of *N*-ethyl-*N*-[2-(1-hydroxy-1-methylethyl)-1-(tetrahydro-2*H*-pyran-4-ylmethyl)-1*H*-benzimidazol-5-yl]acetamide (1.78 g of the above crude product, 2.88 mmol) in THF (100 mL) at 0 °C. Stirring for 20 min, iodoethane (1.23 g, 8.64 mmol) was added. The reaction mixture was stirred overnight at room temperature, quenched with saturated $NH_4Cl$ (20 mL) and diluted with EtOAc (100 mL), washed with saturated NaCl (2x20 mL). After filtration and concentration, the residue was purified by MPLC using EtOAc/MeOH (20:1) on silica gel to give 0.423 g (39%) of a grey white solid as the title compound. MS (ESI) $(M+H)^+$ = 374.03.

**Step D. *N*-ethyl-2-(1-methoxy-1-methylethyl)-1-(tetrahydro-2*H*-pyran-4-ylmethyl)-1*H*-benzimidazol-5-amine**

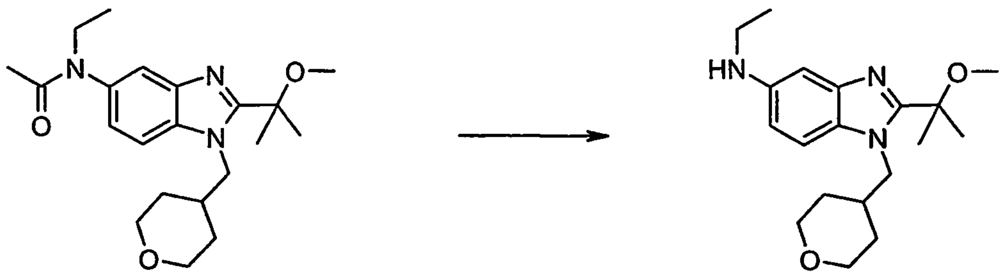

*N*-ethyl-*N*-[2-(1-methoxy-1-methylethyl)-1-(tetrahydro-2*H*-pyran-4-ylmethyl)-1*H*-benzimidazol-5-yl]acetamide (422.5 mg, 1.13 mmol) was dissolved in 15 mL of EtOH-2*N* HCl (3:2), and then heated at 120°C in a Personal Chemistry SmithSynthesizer microwave instrument for 3.5 h. After concentration and dried *in vacuo,* 441.9 mg (100%) of a light brown solid was obtained as the title product. MS (ESI) $(M+H)^+$ = 332.04.

## Claims

**1.** A compound of Formula I or a pharmaceutically acceptable salt thereof:

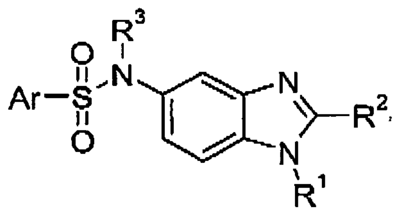

**I**

wherein

$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_3$-$_{10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy and amino;

$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$akyl, and $C_{4-8}$cycloalkeny-$C_{1-4}$alkyl used in defining $R^2$ is optionally substituted by one or more groups selected from halogen, methoxy, ethoxy, methyl, ethyl, hydroxy, and amino;

$R^3$ is selected from -H, $C_{1-6}$alkyl $C_{2-6}$alkenyl, $C_{1-6}$cycloalkyl, and $C_{3-6}$cycloalkyl-$C_{1-4}$alkyl; and

Ar is selected from $C_{6-10}$aryl and $C_{3-6}$heteroaryl, wherein said $C_{6-10}$aryl and $C_3$-$_6$heteroaryl are optionally substituted with one or more groups selected from $C_1$-$_3$alkyl, $C_{1-6}$alkoxy, $C_{1-6}$alkylaminocarbonyl and halogen; with the proviso that the compound is not *N*-(1-Ethoxymethyl-2-trifluoromethyl-1*H*-benzoimidazol-5-yl)-benzenesulfonamide, 2-Chloro-*N*-(1-ethoxymethyl-2-trifluoromethyl-1*H*-benzoimidazol-5-yl)-benzenesulfonamide, or *N*-{1,2-Dimethyl-1*H*-benzoimidazol-5-yl)-4-methyl-benzenesulfonamide.

**2.** A compound as claimed in claim 1, wherein
$R^1$ is selected from $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl used in defining $R^1$ is optionally substituted by one or more groups selected from halogen, methoxy, ethoxy, methyl, hydroxy and amino;
$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$-cycloalkenyl-$C_{1-4}$alkyl, wherein said $C_{1-6}$alkyl, $C_{2-6}$alkeny, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, methoxy, ethoxy and hydroxy;
$R^3$ is selected from -H and $C_{1-3}$alkyl; and
Ar is selected from phenyl and $C_{3-6}$heteroaryl, wherein said phenyl and $C_{3-6}$heteroaryl are optionally substituted with one or more groups selected from methyl, methoxy, fluoro, chloro, bromo and iodo.

**3.** A compound as claimed claim 1,
$R^1$ is selected from cyclopentyl-methyl, cyclohexyl-methyl, cyclobutylmethyl, cyclopropylmethyl, 4,4-difluorocyclohexanemethyl, tetrahydropyranylmethyl, tetrahydrofuranyl-methyl, morpholinyl-methyl, piperdinylethyl, N-methyl-piperdinyl-methyl and piperdinyl-methyl;
$R^2$ is selected from t-butyl, n-butyl, 2-methyl-2-butyl, isopentyl, 2-hydroxy-propyl, 2-methoxy-2-propyl, 1-methylpropyl, 1,1-dimethyl-propyl, 1,1-dimethyl-3-buten-1-yl, trifluoromethyl, 1,1-difluoroethyl, 2,2,2-trifluoroethyl, ethyl, and 2-propyl;
$R^3$ is selected from -H and methyl; and
Ar is selected from phenyl, pyridyl, pyrimidyl, thiazolyl, thienyl, isoxazolyl, imidazolyl, and pyrazolyl, wherein said phenyl, pyridyl, pyrimidyl, thiazolyl, thienyl, isoxazolyl, imidazolyl, and pyrazolyl are optionally substituted with one or more groups selected from methyl, methoxy, fluoro and chloro.

**4.** A compound as claimed in claim 1, wherein
$R^1$ is cyclohexyl-methyl, tetrahydropyranylmethyl and 4,4-difluorocyclohexanemethyl;
$R^2$ is t-butyl and 1,1-difluoroethyl;
$R^3$ is selected from -H and methyl; and
Ar is selected from phenyl, pyridyl, thiazolyl, thienyl, isoxazolyl, imidazolyl, and pyrazolyl, wherein said phenyl, pyridyl, thiazolyl, thienyl, isoxazolyl, imidazolyl, and pyrazolyl are optionally substituted with one or more methyl groups.

**5.** A compound selected from:
*N*-[2-*tert*-Butyl-1-(cyclohexylmethyl)-1*H*-benzimidazol-5-yl]thiophene-2-sulfonamide;
*N*-[2-*tert*-Butyl-1-(cyclohexylmethyl)-*1H*-benzimidazol-5-yl]-*N*-methylthiophene-2-sulfonamide;
*N*-(1-Benzyl-2-*tert*-butyl-1*H*-benzimidazol-5-yl)-*N*-methylbenzenesulfonamide;
*N*-[2-*tert*-Butyl-1-(cyclohexylmethyl)-1*H*-benzimidazol-5-yl]-*N*,3,5-trimethylisoxazole-4-sulfonamide;
*N*-[2-*tert*-Butyl-1-(cyclohexylmethyl)-1*H*-benzimidazol-5-yl]-*N*,1,2-trimethyl-1*H*-imidazole-4-sulfonamide;
*N*-[2-*tert*-Butyl-1-(cyclohexylmethyl)-1*H*-benzimidazol-5-yl]-*N*,1,3,5-tetramethyl-1*H*-pyrazole-4-sulfonamide;
*N*-[2-*tert*-butyl-1-(cyclohexylmethyl)-1*H*-benzimidazol-5-yl]benzene sulphonamide;
*N*-[1-(cyclohexylmethyl)-2-ethyl-1*H*-benzimidazol-5-yl]benzenesulfonamide;
*N*-[1-(cyclohexylmethyl)-2-isopropyl-1*H*-benzimidazol-5-yl]benzene sulphonamide;
*N*-[1-(cyclohexylmethyl)-2-(1-methylcyclopropyl)-1*H*-benzimidazol-5-yl]benzenesulfonamide;
*N*-[1-(cyclohexylmethyl)-2-(1,1-dimethylpropyl)-1*H*-benzimidazol-5-yl]-benzenesulfonamide;
*N*-[1-(cyclohexylmethyl)-2-(1,1-dimethyl-3-butenyl)-1*H*-benzimidazol-5-yl]-benzenesulfonamide;
*N*-[1-(cyclohexylmethyl)-2-(1,1-dimethylethyl)-1*H*-benzimidazol-5-yl]-*N*-methylbenzenesulfonamide;
*N*-[1-(cyclohexylmethyl)-2-ethyl-1*H*-benzimidazol-5-yl]-*N*-methyl-benzene sulphonamide;
*N*-[1-(cyclohexylmethyl)-2-isopropyl-1*H*-benzimidazol-5-yl]-*N*-methyl-benzene sulphonamide;
*N*-[1-(cyclohexylmethyl)-2-(1-methylcyclopropyl)-1*H*-benzimidazol-5-yl]-*N*-methyl-benzenesulfonamide;
*N*-[2-(1,1-dimethylethyl)-1-[(tetrahydro-2*H*-pyran-4-yl)methyl]-1*H*-benzimidazol-5-yl]-benzenesulfonamide;
*N*-[2-(1,1-dimethylethyl)-1-[(tetrahydro-2-furanyl)methyl]-1*H*-benzimidazol-5-yl]-benzenesulfonamide;
*N*-[1-(cyclobutylmethyl)-2-(1,1-dimethylethyl)-1*H*-benzimidazol-5-yl]-benzenesulfonamide;
*N*-[1-(cyclopropylmethyl)-2-(1,1-dimethylethyl)-1*H*-benzimidazol-5-yl]-benzenesulfonamide;
*N*-[2-*tert*-Butyl-1-(tetrahydro-2*H*-pyran-4-ylmethyl)-1*H*-benzimidazol-5-yl]-*N*-methylbenzenesulfonamide;
*N*-[2-*tert*-Butyl-1-(tetrahydro-2*H*-pyran-2-ylmethyl)-1*H*-benzimidazol-5-yl]-*N*-methylbenzenesulfonamide;
*N*-[1-(cyclohexylmethyl)-2-(1-hydroxy-1-methylethyl)-1*H*-benzimidazol-5-yl]-benzenesulfonamide;

*N*-[1-(cyclohexylmethyl)-2-(1-methoxy-1-methylethyl)-1*H*-benzimidazol-5-yl]-*N*-methyl-benzenesulfonamide;
*N*-[1-(cyclohexylmethyl)2-(1-methoxy-1-methylethyl)-1*H*-benzimidazol-5-yl]-benzenesulfonamide;
*N*-[2-*tert*-butyl-1-(cyclohexylmethyl)-1*H*-benzimidazol-5-yl]-*N*,1-dimethyl-1*H*-imidazole-4-sulfonamide;
*N*-(5-{[[2-*tert*-Butyl-1-(cyclohexylmethyl)-1*H*-benzimidazol-5-yl](methyl)amino]sulfonyl}-4-methyl-1,3-thiazol-2-yl) acetamide;
*N*-[2-*tert*-Butyl-1-(cyclohexylmethyl)-*1H*-benzimidazol-5-yl]-*N*-methylpyridine-3-sulfonamide;
*N*-[2-*tert*-Butyl-1-(cyclohexylmethyl)-1*H*-benzimidazol-5-yl]-*N*,1,2-trimethyl-1*H*-imidazole-5-sulfonamide;
*N*-[2-*tert*-Butyl-1-(tetrahydro-2*H*-pyran-4-ylmethyl)-1*H*-benzimidazol-5-yl]-*N*,1,2-trimethyl-1*H*-imidazole-5-sulfonamide; Ethyl 4-{[[2-*tert*-butyl-1-(tetrahydro-2*H*-pyran-4-ylmethyl)-1*H*-benzimidazol-5-yl](methyl)amino]sulfonyl}-3,5-dimethyl-1*H*-pyrrole-2-carboxylate;
*N*-[2-*tert*-Butyl- 1-(tetrahydro- 2*H*-pyran- 4- ylmethyl)- 1*H*-benzimidazol- 5- yl]- 4-(hydroxymethyl)-*N*-methylbenzenesulfonamide;
*N*-[2-*tert*-Butyl-1-(tetrahydro-2*H*-pyran-4-ylmethyl)-1*H*-benzimidazol-5-yl]-*N*-methyl-4-(1*H*-1,2,3-triazol-1-ylmethyl) benzenesulfonamide;
*N*-[2-*tert*-Butyl- 1-(tetrahydro- 2*H*-pyran- 4- ylmethyl)- 1*H*-benzimidazol- 5- yl]- 4- { [ (2- hydroxyethyl) amino] methyl}-*N*-methylbenzenesulfonamide;
*N*-[2-*tert*-Butyl-1-(cyclopentylmethyl)-1*H*-benzimidazol-5-yl]-*N*-methylbenzenesulfonamide;
*N*-[2-*tert*-Butyl-1-(2-cyclohexylethyl)-1*H*-benzimidazol-5-yl]-*N*-methylbenzenesulfonamide;
*N*-[1-(1-Benzylpyrrolidin-3-yl)-2-*tert*-butyl-1*H*-benzimidazol-5-yl]-*N*-methylbenzenesulfonamide;
*N*-{2-*tert*-Butyl-1-[(4,4-difluorocyclohexyl)methyl]-1*H*-benzimidazol-5-yl}-*N*-methylbenzenesulfonamide;
*N*-[2-*tert*-Butyl-1-(pyridin-4-ylmethyl)-1*H*-benzimidazol-5-yl]-*N*-methylbenzenesulfonamide;
*N*-methyl-*N*-[1-(tetrahydro-2*H*-pyran-4-ylmethyl)-2-(trifluoromethyl)-1*H*-benzimidazol-5-yl]benzenesulfonamide;
*N*-[2-(1,1-difluoroethyl)-1-(tetrahydro-2*H*-pyran-4-ylmethyl)-1*H*-benzimidazol-5-yl]-*N*-methylbenzenesulfonamide;
*N*-methyl- *N*-[1-(tetrahydro- 2*H*-pyran- 4- ylmethyl)- 2-(2,2,2- trifluoroethyl)- 1*H*- benzimidazol- 5- yl] benzenesulfonamide;
*N*-[1-(cyclohexylmethyl)-2-(1-ethylpropyl)-1*H*-benzimidazol-5-yl]benzenesulfonamide;
*N*-[1-(cyclohexylmethyl)-2-(1-ethylpropyl)-1*H*-benzimidazol-5-yl]-*N*-methylbenzenesulfonamide;
*N*-[2-*tert*-butyl-1-(cyclohexylmethyl)-1*H*-benzimidazol-5-yl]-*N*-ethylbenzenesulfonamide;
*N*-methyl- *N*-[2-(1- methyl- 1- pyridin- 2- ylethyl)- 1-(tetrahydro- 2*H*-pyran- 4- ylmethyl)- 1*H*-benzimidazol- 5- yl] benzenesulfonamide;
*N*-[2-(1-cyano-1-methylethyl)-1-(tetrahydro-2*H*-pyran-4-ylmethyl)-1*H*-benzimidazol-5-yl]-*N*-methylbenzenesulfonamide;
*N*-methyl-*N*-[2-propyl-1-(tetrahydro-2*H*-pyran-4-ylmethyl)-1*H*-benzimidazol-5-yl]benzenesulfonamide;
*N*-[2-butyl-1-(tetrahydro-2*H*-pyran-4-ylmethyl)-1*H*-benzimidazol-5-yl]-*N*-methylbenzenesulfonamide;
*N*-[2-*tert*-butyl-1-(tetrahydro-2*H*-pyran-4-ylmethyl)-1*H*-benzimidazol-5-yl]-*N*-ethylbenzenesulfonamide;
*N*-ethyl-*N*-[2-(1-methoxy-1-methylethyl)-1-(tetrahydro-2*H*-pyran-4-ylmethyl)-1*H*-benzimidazol-5-yl]benzenesulfonamide;
and pharmaceutically acceptable salts thereof

**6.** A compound according to any one of claims 1-5 for use as a medicament.

**7.** The use of a compound according to any one of claims 1-5 in the manufacture of a medicament for the therapy of pain.

**8.** The use of a compound according to any one of claims 1-5 in the manufacture of a medicament for the treatment of anxiety disorders.

**9.** The use of a compound according to any one of claims 1-5 in the manufacture of a medicament for the treatment of cancer, multiple sclerosis, Parkinson's disease, cancer, Huntington's chorea, Alzheimer's disease, gastrointestinal disorders and cardiovascular disorders.

**10.** A pharmaceutical composition comprising a compound according to any one of claims 1-5 and a pharmaceutically acceptable carrier.

**11.** A method of preparing a compound of Formula I,

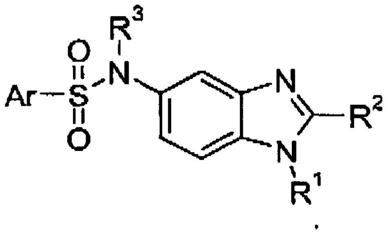

**I**

comprising:

reacting a compound of Formula II,

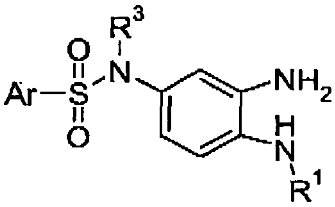

**II**

with a compound of $R^2COX$, in the presence of a base, such as an alkylamine, and optionally a coupling reagent, such as HATU, EDC;

wherein
X is selected from Cl, Br, F and OH;
$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$-alkyl, $C_{3-10}$cycloalkyl, $C_{4-8}$cycloalkenyl, and $C_{3-6}$heterocycloakyl, wherein said $C_{1-1}$alkyl, $C_{2-10}$alkenyl, $C_3$-$_{10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-1}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy and amino;
$R^2$ is selected from $C_1$-$_{10}$alkyl, $C_{2-10}$alkenyl, $C_{3-10}$cyloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_3$. $_{10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, ethoxy, methyl, ethyl, hydroxy, and amino;
$R^3$ is selected from -H, $C_{1-6}$alkyl, $C_{2-6}$alkenyl, $C_{3-6}$cycloalkyl, and $C_{3-6}$cycloalkyl-$C_{1-4}$alkyl and
Ar is selected from $C_{6-10}$aryl and $C_{3-6}$heteroaryl, wherein said $C_{6-10}$aryl and $C_3$-$_6$heteroaryl are optionally substituted with one or more groups selected from $C_{1-3}$alkyl, $C_{1-6}$alkoxy, $C_{1-6}$alkylaminocarbonyl and halogen.

## Patentansprüche

**1.** Verbindungen der Formel I oder deren pharmazeutisch annehmbare Salze:

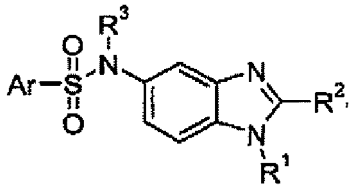

I

wobei

$R^1$ ausgewählt ist aus $C_{1-10}$-Alkyl, $C_{2-10}$-Alkenyl, $C_{3-10}$-Cycloalkyl-$C_{1-4}$-alkyl, $C_{4-8}$-Cycloalkenyl-$C_{1-4}$-alkyl, $C_{3-6}$-Heterocycloalkyl-$C_{1-4}$-alkyl, $C_{3-10}$-Cycloalkyl, $C_{4-8}$-Cycloalkenyl und $C_{3-6}$-Heterocycloalkyl, wobei die bei der Definition von $R^1$ verwendeten $C_{1-10}$-Alkyl-, $C_{2-10}$-Alkenyl- $C_{3-10}$-Cycloalkyl-$C_{1-4}$-alkyl-, $C_{4-8}$-Cycloalkenyl-$C_{1-4}$-alkyl-, $C_{3-6}$-Heterocycloalkyl-$C_{1-4}$-alkyl-, $C_{3-10}$-Cycloalkyl-, $C_{4-8}$-Cycloalkenyl- und $C_{3-6}$-Heterocycloalkylreste gegebenenfalls durch eine oder mehrere Gruppen ausgewählt aus Halogen, Cyano, Nitro, Methoxy, Ethoxy, Methyl, Ethyl, Hydroxy und Amino substituiert sind;

$R^2$ ausgewählt ist aus $C_{1-10}$-Alkyl, $C_{2-10}$-Alkenyl, $C_{3-10}$-Cycloalkyl, $C_{3-10}$-Cycloalkyl-$C_{1-4}$-alkyl und $C_{4-8}$-Cycloalkenyl-$C_{1-4}$-alkyl, wobei die bei der Definition von $R^2$ verwendeten $C_{1-10}$-Alkyl-, $C_{2-10}$-Alkenyl-, $C_{3-10}$-Cycloalkyl-, $C_{3-10}$-Cycloalkyl-$C_{1-4}$-alkyl- und $C_{4-8}$-Cycloalkenyl-$C_{1-4}$-alkylreste gegebenenfalls durch eine oder mehrere Gruppen ausgewählt aus Halogen, Methoxy, Ethoxy, Methyl, Ethyl, Hydroxy und Amino substituiert sind;

$R^3$ ausgewählt ist aus -H, $C_{1-6}$-Alkyl, $C_{2-6}$-Alkenyl, $C_{3-6}$-Cycloalkyl und $C_{3-6}$-Cycloalkyl-$C_{1-4}$-alkyl; und

Ar ausgewählt ist aus $C_{6-10}$-Aryl und $C_{3-6}$-Heteroaryl, wobei die $C_{6-10}$-Aryl- und $C_{3-6}$-Heteroarylreste gegebenenfalls durch eine oder mehrere Gruppen ausgewählt aus $C_{1-3}$-Alkyl, $C_{1-6}$-Alkoxy, $C_{1-6}$-Alkylaminocarbonyl und Halogen substituiert sind;

mit der Maßgabe, daß es sich bei der Verbindung nicht um

*N*-(1-Ethoxymethyl-2-trifluormethyl-1*H*-benzimidazol-5-yl)benzolsulfonsäureamid,

2-Chlor-*N*-(1-ethoxymethyl-2-trifluormethyl-1*H*-benzimidazol-5-yl)benzolsulfonsäureamid oder

*N*-(1,2-Dimethyl-1*H*-benzimidazol-5-yl)-4-methylbenzolsulfonsäureamid handelt.

**2.** Verbindungen nach Anspruch 1, wobei

$R^1$ ausgewählt ist aus $C_{1-6}$-Alkyl, $C_{2-6}$-Alkenyl, $C_{3-6}$-Cycloalkyl-$C_{1-4}$-alkyl, $C_{4-6}$-Cycloalkenyl-$C_{1-4}$-alkyl und $C_{3-6}$-Heterocycloalkyl-$C_{1-4}$-alkyl, wobei die bei der Definition von $R^1$ verwendeten $C_{1-6}$-Alkyl-, $C_{2-6}$-Alkenyl-, $C_{3-6}$-Cycloalkyl-$C_{1-4}$-alkyl-, $C_{4-6}$-Cycloalkenyl-$C_{1-4}$-alkyl- und $C_{3-6}$-Heterocycloalkyl-$C_{1-4}$-alkylreste gegebenenfalls durch eine oder mehrere Gruppen ausgewählt aus Halogen, Methoxy, Ethoxy, Methyl, Hydroxy und Amino substituiert sind;

$R^2$ ausgewählt ist aus $C_{1-6}$-Alkyl, $C_{2-6}$-Alkenyl, $C_{3-6}$-Cycloalkyl, $C_{3-6}$-Cycloalkyl-$C_{1-4}$-alkyl und $C_{4-6}$-Cycloalkenyl-$C_{1-4}$-alkyl, wobei die bei der Definition von $R^2$ verwendeten $C_{1-6}$-Alkyl-, $C_{2-6}$-Alkenyl-, $C_{3-6}$-Cycloalkyl-, $C_{3-6}$-Cycloalkyl-$C_{1-4}$-alkyl- und $C_{4-6}$-Cycloalkenyl-$C_{1-4}$-alkylreste gegebenenfalls durch eine oder mehrere Gruppen ausgewählt aus Halogen, Methoxy, Ethoxy und Hydroxy substituiert sind;

$R^3$ ausgewählt ist aus -H und $C_{1-3}$-Alkyl; und

Ar ausgewählt ist aus Phenyl und $C_{3-6}$-Heteroaryl, wobei diese Phenyl- und $C_{3-6}$-Heteroarylreste gegebenenfalls durch eine oder mehrere Gruppen ausgewählt aus Methyl, Methoxy, Fluor, Chlor, Brom und Iod substituiert sind.

**3.** Verbindungen nach Anspruch 1, wobei

$R^1$ ausgewählt ist aus Cyclopentylmethyl, Cyclohexylmethyl, Cyclobutylmethyl, Cyclopropylmethyl, 4,4-Difluorcyclohexanmethyl, Tetrahydropyranylmethyl, Tetrahydrofuranylmethyl, Morpholinylmethyl, Piperidinylethyl, N-Methylpiperidinylmethyl und Piperidinylmethyl;

$R^2$ ausgewählt ist aus t-Butyl, n-Butyl, 2-Methyl-2-butyl, Isopentyl, 2-Hydroxypropyl, 2-Methoxy-2-propyl, 1-Methylpropyl, 1,1-Dimethylpropyl, 1,1-Dimethyl-3-buten-1-yl, Trifluormethyl, 1,1-Difluorethyl, 2,2,2-Trifluorethyl, Ethyl und 2-Propyl;

$R^3$ ausgewählt ist aus -H und Methyl; und

Ar ausgewählt ist aus Phenyl, Pyridyl, Pyrimidyl, Thiazolyl, Thienyl, Isoxazolyl, Imidazolyl und Pyrazolyl, wobei diese Phenyl-, Pyridyl-, Pyrimidyl-, Thiazolyl-, Thienyl-, Isoxazolyl-, Imidazolyl- und Pyrazolylreste gegebenenfalls durch eine oder mehrere Gruppen ausgewählt aus Methyl, Methoxy, Fluor und Chlor substituiert sind.

**4.** Verbindungen nach Anspruch 1, wobei

$R^1$ für Cyclohexylmethyl, Tetrahydropyranylmethyl oder 4,4-Difluorcyclohexanmethyl steht;

$R^2$ für t-Butyl oder 1,1-Difluorethyl steht;
$R^3$ ausgewählt ist aus -H und Methyl; und
Ar ausgewählt ist aus Phenyl, Pyridyl, Thiazolyl, Thienyl, Isoxazolyl, Imidazolyl und Pyrazolyl, wobei diese Phenyl-, Pyridyl-, Thiazolyl-, Thienyl-, Isoxazolyl-, Imidazolyl- und Pyrazolylreste gegebenenfalls durch eine oder mehrere Methylgruppen substituiert sind.

**5.** Verbindungen, ausgewählt aus:
*N*-[2-*tert*.-Butyl-1-(Cyclohexylmethyl)-1*H*-benzimidazol-5-yl]thiophen-2-sulfonsäureamid;
*N*-[2-*tert*.-Butyl-1-(Cyclohexylmethyl)-1*H*-benzimidazol-5-yl]-*N*-methylthiophen-2-sulfonsäureamid;
*N*-(1-Benzyl-2-*tert*.-butyl-1*H*-benzimidazol-5-yl)-*N*-methylbenzolsulfonsäureamid;
*N*-[2-*tert*.-Butyl-1-(Cyclohexylmethyl)-1*H*-benzimidazol-5-yl]-N,3,5-trimethylisoxazol-4-sulfonsäureamid;
*N*-[2-*tert*.-Butyl-1-(cyclohexylmethyl)-1*H*-benzimidazol-5-yl]-*N*,1,2-trimethyl-*1H*-imidazol-4-sulfonsäureamid;
*N*-[2-*tert*.-Butyl-1-(cyclohexylmethyl)-1*H*-benzimidazol-5-yl]-*N*,1,3,5-tetramethyl-1*H*-pyrazol-4-sulfonsäureamid;
*N*-[2-*tert*.-Butyl-1-(cyclohexylmethyl)-1*H*-benzimidazol-5-yl]benzolsulfonsäureamid;
*N*-[1-(cyclohexylmethyl)-2-ethyl-1*H*-benzimidazol-5-yl]benzolsulfonsäureamid;
*N*-[1-(cyclohexylmethyl)-2-isopropyl-1*H*-benzimidazol-5-yl]benzolsulfonsäureamid;
*N*-[1-(cyclohexylmethyl)-2-(1-methylcyclopropyl)-1*H*-benzimidazol-5-yl]benzolsulfonsäureamid;
*N*-[1-(cyclohexylmethyl)-2-(1,1-dimethylpropyl)-1*H*-benzimidazol-5-yl]benzolsulfonsäureamid;
*N*-[1-(cyclohexylmethyl)-2-(1,1-dimethyl-3-butenyl)-1*H*-benzimidazol-5-yl]benzolsulfonsäureamid;
*N*-[1-(cyclohexylmethyl)-2-(1,1-dimethylethyl)-1*H*-benzimidazol-5-yl]-*N*-methylbenzolsulfonsäureamid;
*N*-[1-(cyclohexylmethyl)-2-ethyl-1*H*-benzimidazol-5-yl]-*N*-methylbenzolsulfonsäureamid;
*N*-[1-(cyclohexylmethyl)-2-isopropyl-1*H*-benzimidazol-5-yl]-*N*-methylbenzolsulfonsäureamid;
*N*-[1-(cyclohexylmethyl)-2-(1-methylcyclopropyl)-1*H*-benzimidazol-5-yl]-*N*-methylbenzolsulfonsäureamid;
*N*-[2-(1,1-Dimethylethyl)-1-[(tetrahydro-2*H*-pyran-4-yl)methyl]-1*H*-benzimidazol-5-yl]benzolsulfonsäureamid;
*N*-[2-(1,1-Dimethylethyl)-1-[(tetrahydro-2-furanyl)methyl]-1*H*-benzimidazol-5-yl]benzolsulfonsäureamid;
*N*-[1-(cyclobutylmethyl)-2-(1,1-dimethylethyl)-1*H*-benzimidazol-5-yl]benzolsulfonsäureamid;
*N*-[1-(cyclopropylmethyl)-2-(1,1-dimethylethyl)-1*H*-benzimidazol-5-yl]benzolsulfonsäureamid;
*N*-[2-*tert*.-Butyl-1-(tetrahydro-2*H*-pyran-4-ylmethyl)-1*H*-benzimidazol-5-yl]-*N*-methylbenzolsulfonsäureamid;
*N*-[2-*tert*.-Butyl-1-(tetrahydro-2*H*-pyran-2-ylmethyl)-1*H*-benzimidazol-5-yl]-*N*-methylbenzolsulfonsäureamid;
*N*-[1-(cyclohexylmethyl)-2-(1-hydroxy-1-methylethyl)-1*H*-benzimidazol-5-yl]benzolsulfonsäureamid;
*N*-[1-(cyclohexylmethyl)-2-(1-methoxy-1-methylethyl)-1*H*-benzimidazol-5-yl]-*N*-methylbenzolsulfonsäureamid;
*N*-[1-(Cyclohexylmethyl)-2-(1-methoxy-1-methylethyl)-1*H*-benzimidazol-5-yl]benzolsulfonsäureamid;
*N*-[2-*tert*.-Butyl-1-(cyclohexylmethyl)-1*H*-benzimidazol-5-yl]-*N*,1-dimethyl-1*H*-imidazol-4-sulfonsäureamid;
*N*-(5-{[[2-*tert*.-Butyl-1-(cyclohexylmethyl)-1*H*-benzimidazol-5-yl](methyl)amino]sulfonyl}-4-methyl-1,3-thiazol-2-yl) acetamid;
*N*-[2-*tert*.-Butyl-1-(cyclohexylmethyl)-1*H*-benzimidazol-5-yl]-*N*-methylpyridin-3-sulfonsäureamid;
*N*-[2-*tert*.-Butyl-1-(cyclohexylmethyl)-1*H*-benzimidazol-5-yl]-*N*,1,2-trimethyl-1*H*-imidazol-5-sulfonsäureamid;
*N*-[2-*tert*.-Butyl-1-(tetrahydro-2*H*-pyran-2-ylmethyl)-1*H*-benzimidazol-5-yl]-*N*,1,2-trimethyl-1*H*-imidazol-5-sulfonsäureamid;
4-{[[2-*tert*.-Butyl-1-(tetrahydro-2*H*-pyran-4-ylmethyl)-1*H*-benzimidazol-5-yl] (methyl) amino] sulfonyl}-3, 5-dimethyl-1*H*-pyrrol-2-carbonsäureethylester;
*N*-[2-tert.-Butyl-1-(tetrahydro-2*H*-pyran-4-ylmethyl)-1*H*-benzimidazol-5-yl]-4-(hydroxymethyl)-*N*-methylbenzolsulfonsäureamid;
*N*-[2-*tert*.-Butyl-1-(tetrahydro-2*H*-pyran-4-ylmethyl)-1*H*-benzimidazol-5-yl]-*N*-methyl-4-(1*H*-1,2,3-triazol-1-ylmethyl)benzolsulfonsäureamid;
*N*-[2-*tert*.-Butyl-1-(tetrahydro-2*H*-pyran-4-ylmethyl)-1*H*-benzimidazol-5-yl]-4-{[(2-hydroxyethyl)amino]methyl}-*N*-methylbenzolsulfonsäureamid;
*N*-[2-*tert*.-Butyl-1-(cyclopentylmethyl)-1*H*-benzimidazol-5-yl]-*N*-methylbenzolsulfonsäureamid;
*N*-[2-*tert*.-Butyl-1-(2-cyclohexylethyl)-1*H*-benzimidazol-5-yl]-*N*-methylbenzolsulfonsäureamid;
*N*-[1-(1-Benzylpyrrolidin-3-yl)-2-*tert*.-butyl-1*H*-benzimidazol-5-yl]-*N*-methylbenzolsulfonsäureamid;
*N*-{2-tert.-Butyl-1- [(4,4-difluorcyclohexyl) methyl] -1*H*-benzimidazol-5-yl}-*N*-methylbenzolsulfonsäureamid;
*N*-[2-*tert*.-Butyl-1-(pyridin-4-ylmethyl)-1*H*-benzimidazol-5-yl]-*N*-methylbenzolsulfonsäureamid;
*N*-Methyl-*N*-[1-(tetrahydro-2*H*-pyran-4-ylmethyl)-2-(trifluormethyl)-1*H*-benzimidazol-5-yl]benzolsulfonsäureamid;
*N*-[2-(1,1-Difluorethyl)-1-(tetrahydro-2*H*-pyran-4-ylmethyl)-1*H*-benzimidazol-5-yl]-*N*-methylbenzolsulfonsäureamid;
*N*-Methyl-*N*-[1-(tetrahydro-2*H*-pyran-4-ylmethyl)-2-(2,2,2-trifluorethyl)-1*H*-benzimidazol-5-yl]benzolsulfonsäureamid;
*N*-[1-(Cyclohexylmethyl)-2-(1-ethylpropyl)-1*H*-benzimidazol-5-yl]benzolsulfonsäureamid;

*N*-[1-(Cyclohexylmethyl)-2-(1-ethylpropyl)-1*H*-benzimidazol-5-yl]-*N*-methylbenzolsulfonsäureamid;
*N*-[2-*tert*.-Butyl-1-(CyClohexylmethyl)-1*H*-benzimidazol-5-yl]-*N*-ethylbenzolsulfonsäureamid;
*N*-Methyl-*N*-[2-(1-methyl-1-pyridin-2-ylethyl)-1-(tetrahydro-2*H*-pyran-4-ylmethyl)-1*H*-benzimidazol-5-yl]benzolsulfonsäureamid;
*N*-[2-(1-Cyano-1-methylethyl)-1-(tetrahydro-2H-pyran-4-ylmethyl)-1*H*-benzimidazol-5-yl]-*N*-methylbenzolsulfonsäureamid;
*N*-Methyl-*N*-[2-propyl-1-(tetrahydro-2*H*-pyran-4-ylmethyl)-1*H*-benzimidazol-5-yl]benzolsulfonsäureamid;
*N*-[2-Butyl-1-(tetrahydro-2*H*-pyran-4-ylmethyl)-1*H*-benzimidazol-5-yl]-*N*-methylbenzolsulfonsäureamid;
*N*-[2-*tert*.-Butyl-1-(tetrahydro-2H-pyran-4-ylmethyl)-1*H*-benzimidazol-5-yl]-*N*-ethylbenzolsulfonsäureamid;
*N*-Ethyl-*N*-[2-(1-methoxy-1-methylethyl)-1-(tetrahydro-2*H*-pyran-4-ylmethyl)-1*H*-benzimidazol-5-yl]benzolsulfonsäureamid;
und deren pharmazeutisch annehmbare Salze.

**6.** Verbindungen nach einem der Ansprüche 1 bis 5 zur Verwendung als Medikament.

**7.** Verwendung einer Verbindung nach einem der Ansprüche 1 bis 5 bei der Herstellung eines Medikaments zur Behandlung von Schmerzen.

**8.** Verwendung einer Verbindung nach einem der Ansprüche 1 bis 5 bei der Herstellung eines Medikaments zur Behandlung von Angststörungen.

**9.** Verwendung einer Verbindung nach einem der Ansprüche 1 bis 5 bei der Herstellung eines Medikaments zur Behandlung von Krebs, multipler Sklerose, Parkinson-Krankheit, Krebs, Chorea Huntington, Alzheimer-Krankheit, Erkrankungen des Magen-DarmTraktes und Herzkreislaufkrankheiten.

**10.** Pharmazeutische Zusammensetzung, enthaltend eine Verbindung nach einem der Ansprüche 1 bis 5 und einen pharmazeutisch annehmbaren Träger.

**11.** Verfahren zur Herstellung einer Verbindung der Formel I

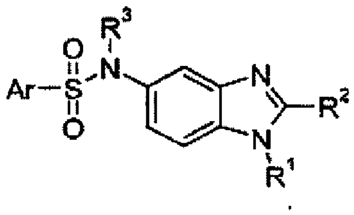

I

bei dem man:

eine Verbindung der Formel II

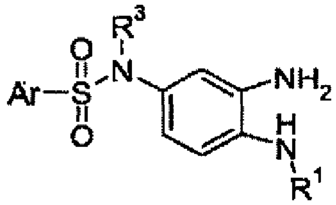

II

in Gegenwart einer Base wie einem Alkylamin und
gegebenenfalls einem Kupplungsmittel wie HATU oder EDC mit einer Verbindung $R^2COX$ umsetzt;

wobei
X ausgewählt ist aus Cl, Br, F und OH;
$R^1$ ausgewählt ist aus $C_{1-10}$-Alkyl, $C_{2-10}$-Alkenyl, $C_{3-10}$-Cycloalkyl-$C_{1-4}$-alkyl, $C_{4-8}$-Cycloalkenyl-$C_{1-4}$-alkyl, $C_{3-6}$-Heterocycloalkyl-$C_{1-4}$-alkyl, $C_{3-10}$-Cycloalkyl, $C_{4-8}$-Cycloalkenyl und $C_{3-6}$-Heterocycloalkyl, wobei die bei der Definition von $R^1$ verwendeten $C_{1-10}$-Alkyl-, $C_{2-10}$-Alkenyl-, $C_{3-10}$-Cycloalkyl-$C_{1-4}$-alkyl-, $C_{4-8}$-Cycloalkenyl-$C_{1-4}$-alkyl-, $C_{3-6}$-Heterocycloalkyl-$C_{1-4}$-alkyl-, $C_{3-10}$-Cycloalkyl-, $C_{4-8}$-Cycloalkenyl- und $C_{3-6}$-Heterocycloalkylreste gegebenenfalls durch eine oder mehrere Gruppen ausgewählt aus Halogen, Cyano, Nitro, Methoxy, Ethoxy, Methyl, Ethyl, Hydroxy und Amino substituiert sind;
$R^2$ ausgewählt ist aus $C_{1-10}$-Alkyl, $C_{2-10}$-Alkenyl, $C_{3-10}$-Cycloalkyl, $C_{3-10}$-Cycloalkyl-$C_{1-4}$-alkyl und $C_{4-8}$-Cycloalkenyl-$C_{1-4}$-alkyl, wobei die bei der Definition von $R^2$ verwendeten $C_{1-10}$-Alkyl-, $C_{2-10}$-Alkenyl-, $C_{3-10}$-Cycloalkyl-, $C_{3-10}$-Cycloalkyl-$C_{1-4}$-alkyl- und $C_{4-8}$-Cycloalkenyl-$C_{i}$-$_{4}$-alkylreste gegebenenfalls durch eine oder mehrere Gruppen ausgewählt aus Halogen, Methoxy, Ethoxy, Methyl, Ethyl, Hydroxy und Amino substituiert sind;
$R^3$ ausgewählt ist aus -H, $C_{1-6}$-Alkyl, $C_{2-6}$-Alkenyl, $C_{3-6}$-Cycloalkyl und $C_{3-6}$-Cycloalkyl-$C_{1-4}$-alkyl; und
Ar ausgewählt ist aus $C_{6-10}$-Aryl und $C_{3-6}$-Heteroaryl, wobei die $C_{6-10}$-Aryl- und $C_{3-6}$-Heteroarylreste gegebenenfalls durch eine oder mehrere Gruppen ausgewählt aus $C_{1-3}$-Alkyl, $C_{1-6}$-Alkoxy, $C_{1-6}$-Alkylaminocarbonyl und Halogen substituiert sind.

## Revendications

**1.** Composé de formule I ou un sel pharmaceutiquement acceptable de celui-ci :

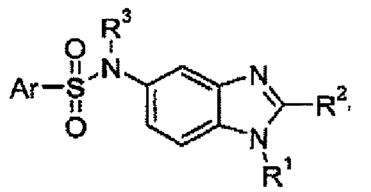

I

dans laquelle
$R^1$ est choisi parmi $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle, et $C_{3-6}$hétérocycloalkyle, où lesdits $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle, et $C_{3-6}$hétérocycloalkyle utilisés pour définir $R^1$ sont éventuellement substitués par un ou plusieurs groupements choisis parmi halogène, cyano, nitro, méthoxy, éthoxy, méthyle, éthyle, hydroxy et amino ;
$R^2$ est choisi parmi $C_{1-10}$alkyle, $C_{2-10}$alcényle, $C_{3-10}$cycloalkyle, $C_{3-10}$cycloalkyl-$C_{1-4}$alkyle, et $C_{4-8}$cycloalcényl-$C_{1-4}$alkyle, où lesdits $C_{1-10}$alkyle, $C_{2-10}$alcényle, $C_{3-10}$cycloalkyl-$C_{1-4}$alkyle, et $C_{4-8}$cycloalcényl-$C_{1-4}$alkyle utilisés pour définir $R^2$ sont éventuellement substitués par un ou plusieurs groupements choisis parmi halogène, méthoxy, éthoxy, méthyle, éthyle, hydroxy, et amino ;
$R^3$ est choisi parmi -H, $C_{1-6}$alkyle, $C_{2-6}$alcényle, $C_{3-6}$cycloalkyle, et $C_{3-6}$cycloalkyl-$C_{1-4}$alkyle ; et
Ar est choisi parmi $C_{6-10}$aryle et $C_{3-6}$hétéroaryle, où lesdits $C_{6-10}$aryle et $C_{3-6}$hétéroaryle sont éventuellement substitués par un ou plusieurs groupements choisis parmi $C_{1-3}$alkyle, $C_{1-6}$alcoxy, $C_{1-6}$alkylaminocarbonyle et halogène ;
à condition que le composé ne soit pas
le *N*-(1-éthoxyméthyl-2-trifluorométhyl-1*H*-benzoimidazol-5-yl)-benzènesulfonamide,
le 2-chloro-*N*-(1-éthoxyméthyl-2-trifluorométhyl-1*H*-benzoimidazol-5-yl)-benzènesulfonamide, ou
le *N*-(1,2-diméthyl-1*H*-benzoimidazol-5-yl)-4-méthyl-benzènesulfonamide.

**2.** Composé selon la revendication 1, **caractérisé en ce que**
$R^1$ est choisi parmi $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle, où lesdits $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle utilisés pour définir $R^1$ sont éventuellement substitués par un ou plusieurs groupements choisis parmi halogène, méthoxy, éthoxy, méthyle, hydroxy et amino ;
$R^2$ est choisi parmi $C_{1-6}$alkyle, $C_{2-6}$alcényle, $C_{3-6}$cycloalkyle, $C_{3-6}$cycloalkyl-$C_{1-4}$alkyle, et $C_{4-6}$cycloalcényl-

$C_{1-4}$alkyle, où lesdits $C_{1-6}$alkyle, $C_{2-6}$alcényle, $C_{3-6}$cycloalkyle, $C_{3-6}$cycloalkyl-$C_{1-4}$alkyle, et $C_{4-6}$cycloalcényl-$C_{1-4}$alkyle utilisés pour définir $R^2$ sont éventuellement substitués par un ou plusieurs groupements choisis parmi halogène, méthoxy, éthoxy et hydroxy ;
$R^3$ est choisi parmi -H et $C_{1-3}$alkyle ; et
Ar est choisi parmi phényle et $C_{3-6}$hétéroaryle, où lesdits phényle et $C_{3-6}$hétéroaryle sont éventuellement substitués par un ou plusieurs groupements choisis parmi méthyle, méthoxy, fluoro, chloro, bromo et iodo.

**3.** Composé selon la revendication 1,
$R^1$ est choisi parmi cyclopentylméthyle, cyclohexylméthyle, cyclobutylméthyle, cyclopropylméthyle, 4,4-difluorocyclohexaneméthyle, tétrahydropyranylméthyle, tétrahydrofuranylméthyle, morpholinylméthyle, pipéridinyléthyle, N-méthylpipéridinylméthyle et pipéridinylméthyle ;
$R^2$ est choisi parmi t-butyle, n-butyle, 2-méthyl-2-butyle, isopentyle, 2-hydroxypropyle, 2-méthoxy-2-propyle, 1-méthylpropyle, 1,1-diméthylpropyle, 1,1-diméthyl-3-butén-1-yle, trifluorométhyle, 1,1-difluoroéthyle, 2,2,2-trifluoroéthyle, éthyle, et 2-propyle ;
$R^3$ est choisi parmi -H et méthyle ; et
Ar est choisi parmi phényle, pyridyle, pyrimidyle, thiazolyle, thiényle, isoxazolyle, imidazolyle et pyrazolyle, où lesdits phényle, pyridyle, pyrimidyle, thiazolyle, thiényle, isoxazolyle, imidazolyle, et pyrazolyle sont éventuellement substitués par un ou plusieurs groupements choisis parmi méthyle, méthoxy, fluoro et chloro.

**4.** Composé selon la revendication 1, **caractérisé en ce que**
$R^1$ est cyclohexylméthyle, tétrahydropyranylméthyle et 4,4-difluorocyclohexaneméthyle ;
$R^2$ est t-butyle et 1,1-difluoroéthyle ; et
$R^3$ est choisi parmi -H et méthyle ; et
Ar est choisi parmi phényle, pyridyle, thiazolyle, thiényle, isoxazolyle, imidazolyle, et pyrazolyle, où lesdits phényle, pyridyle, thiazolyle, thiényle, isoxazolyle, imidazolyle, et pyrazolyle sont éventuellement substitué par un ou plusieurs groupements méthyle.

**5.** Composé choisi parmi :

le *N*-[2-tert-butyl-1-(cyclohexylméthyl)-1*H*-benzimidazol-5-yl]thiophène-2-sulfonamide,
le *N*-[2-tert-butyl-1-(cyclohexylméthyl)-1*H*-benzimidazol-5-yl]-*N*-méthylthiophène-2-sulfonamide ;
le *N*-(1-benzyl-2-*tert*-butyl-1*H*-benzimidazol-5-yl)-*N*-méthylbenzènesulfonamide ;
le *N*-[2-*tert*-butyl-1-(cyclohexylméthyl)-1*H*-benzimidazol-5-yl]-*N*,3,5-triméthylisoxazole-4-sulfonamide ;
le *N*-[2-*tert*-butyl-1-(cyclohexylméthyl)-1*H*-benzimidazol-5-yl]-*N*,1,2-triméthyl-1*H*-imidazole-4-sulfonamide ;
le *N*-[2-*tert*-butyl-1-(cyclohexylméthyl)-1*H*-benzimidazol-5-yl]-N,1,3,5-tétraméthyl-1*H*-pyrazole-4-sulfonamide ;
le *N*-[2-*tert*-butyl-1-(cyclohexylméthyl)-1*H*-benzimidazol-5-yl]benzènesulfonamide ;
le *N*-[1-(cyclohexylméthyl)-2-éthyl-1*H*-benzimidazol-5-yl]benzènesulfonamide ;
le *N*-[1-(cyclohexylméthyl)-2-isopropyl-1*H*-benzimidazol-5-yl]benzènesulfonamide ;
le *N*- [1- (cyclohexylméthyl) -2- (1-méthylcyclopropyl)-1*H*-benzimidazol-5-yl]benzènesulfonamide ;
le *N*-[1-(cyclohexylméthyl)-2-(1,1-diméthylpropyl) - 1*H*-benzimidazol-5-yl]benzènesulfonamide ;
le *N*-[1-(cyclohexylméthyl)-2-(1,1-diméthyl-3-butényl)-1*H*-benzimidazol-5-yl]benzènesulfonamide ;
le *N*- [1- (cyclohexylméthyl) -2- (1,1-diméthyléthyl) - 1*H*-benzimidazol-5-yl]-*N*-méthylbenzènesulfonamide ;
le *N*-[1-(cyclohexylméthyl)-2-éthyl-1*H*-benzimidazol-5-yl]-*N*-méthylbenzènesulfonamide ;
le *N*-[1-(cyclohexylméthyl)-2-isopropyl-1*H*-benzimidazol-5-yl]-*N*-méthylbenzènesulfonamide ;
le *N*- [1- (cyclohexylméthyl) -2- (1-méthylcyclopropyl)-1*H*-benzimidazol-5-yl]-*N*-méthylbenzènesulfonamide ;
le *N*-[2-(1,1-diméthyléthyl)-1-[(tétrahydro-2*H*-pyran-4-yl)méthyl]-1*H*-benzimidazol-5-yl]benzènesulfonamide ;
le *N*-[2-(1,1-diméthyléthyl)-1-[(tétrahydro-2-furanyl)méthyl]-1*H*-benzimidazol-5-yl]benzènesulfonamide ;
le *N*-[1-(cyclobutylméthyl)-2-(1,1-diméthyléthyl)-1*H*-benzimidazol-5-yl]benzènesulfonamide ;
le *N*-[1-(cyclopropylméthyl)-2-(1,1-diméthyléthyl)-1*H*-benzimidazol-5-yl]benzènesulfonamide ;
le *N*-[2-*tert*-butyl-1-(tétrahydro-2*H*-pyran-4-ylméthyl)-1*H*-benzimidazol-5-yl]-*N*-méthylbenzènesulfonamide ;
le *N*-[2-*tert*-butyl-1-(tétrahydro-2*H*-pyran-2-ylméthyl)-1*H*-benzimidazol-5-yl]-*N*-méthylbenzènesulfonamide ;
le *N*-[1-(cyclohexylméthyl)-2-(1-hydroxy-1-méthyléthyl)-1*H*-benzimidazol-5-yl]benzènesulfonamide ;
le *N*-[1-(cyclohexylméthyl)-2-(1-méthoxy-1-méthyléthyl)-1*H*-benzimidazol-5-yl]-*N*-méthylbenzènesulfonamide ;
le *N*-[1-(cyclohexylméthyl)-2-(1-méthoxy-1-méthyléthyl)-1*H*-benzimidazol-5-yl]benzènesulfonamide ;
le *N*-[2-*tert*-butyl-1-(cyclohexylméthyl)-1*H*-benzimidazol-5-yl]-N,1-diméthyl-1H-imidazole-4-sulfonamide ;
le *N*-(5-{[[2-*tert*-butyl-1-(cyclohexylméthyl) -1*H*-benzimidazol-5-yl](méthyl)amino]sulfonyl}-4-méthyl-1,3-thiazol-2-yl)acétamide ;
le *N*-[2-*tert*-butyl-1-(cyclohexylméthyl)-1*H*-benzimidazol-5-yl]-*N*-méthylpyridine-3-sulfonamide ;

le *N*-[2-*tert*-butyl-1-(cyclohexylméthyl)-1*H*-benzimidazol-5-yl]-*N*,1,2-triméthyl-1*H*-imidazole-5-sulfonamide ;
le *N*-[2-*tert*-butyl-1-(tétrahydro-2*H*-pyran-4-ylméthyl)-1*H*-benzimidazol-5-yl]-*N*,1,2-triméthyl-1*H*-imidazole-5-sulfonamide ;
le 4-{[[2-*tert*-butyl-1-(tétrahydro-2*H*-pyran-4-ylméthyl)-1*H*-benzimidazol-5-yl](méthyl)amino]sulfonyl}-3,5-diméthyl-1*H*-pyrrole-2-carboxylate d'éthyle ;
le *N*-[2-*tert*-butyl-1-(tétrahydro-2*H*-pyran-4-ylméthyl)-1*H*-benzimidazol-5-yl]-4-(hydroxyméthyl)-*N*-méthylbenzènesulfonamide ;
le *N*-[2-*tert*-butyl-1-(tétrahydro-2*H*-pyran-4-ylméthyl)-1*H*-benzimidazol-5-yl]-*N*-méthyl-4-(1*H*-1,2,3-triazol-1-ylméthyl)benzènesulfonamide ;
le *N*-[2-*tert*-butyl-1-(tétrahydro-2*H*-pyran-4-ylméthyl)-1*H*-benzimidazol-5-yl]-4-{[(2-hydroxyéthyl)amino]méthyl}-*N*-méthylbenzènesulfonamide ;
le *N*-[2-*tert*-butyl-1-(cyclopentylméthyl)-1*H*-benzimidazol-5-yl]-*N*-méthylbenzènesulfonamide ;
le *N*-[2-*tert*-butyl-1-(2-cyclohexyléthyl)-1*H*-benzimidazol-5-yl]-*N*-méthylbenzènesulfonamide ;
le *N*-[1-(1-benzylpyrrolidin-3-yl)-2-tert-butyl-1*H*-benzimidazol-5-yl]-*N*-méthylbenzènesulfonamide ;
le *N*-{2-*tert*-butyl-1-[(4,4-difluorocyclohexyl)méthyl]-1*H*-benzimidazol-5-yl}-*N*-méthylbenzènesulfonamide ;
le *N*-[2-*tert*-butyl-1-(pyridin-4-ylméthyl)-1*H*-benzimidazol-5-yl]-*N*-méthylbenzènesulfonamide ;
le *N*-méthyl-*N*-[1-(tétrahydro-2*H*-pyran-4-ylméthyl)-2-(trifluorométhyl)-1*H*-benzimidazol-5-yl]benzènesulfonamide ;
le *N*-[2-(1,1-difluoroéthyl)-1-(tétrahydro-2*H*-pyran-4-ylméthyl)-1*H*-benzimidazol-5-yl]-*N*-méthylbenzènesulfonamide ;
le *N*-méthyl-*N*-[1-(tétrahydro-2H-pyran-4-ylméthyl)-2-(2,2,2-trifluoroéthyl)-1*H*-benzimidazol-5-yl]benzènesulfonamide ;
le *N*-[1-(cyclohexylméthyl)-2-(1-éthylpropyl)-1*H*-benzimidazol-5-yl]benzènesulfonamide ;
le *N*-[1-(cyclohexylméthyl)-2-(1-éthylpropyl)-1*H*-benzimidazol-5-yl]-*N*-méthylbenzènesulfonamide ;
le *N*-[2-*tert*-butyl-1-(cyclohexylméthyl)-1*H*-benzimidazol-5-yl]-*N*-éthylbenzènesulfonamide ;
le *N*-méthyl-*N*-[2-(1-méthyl-1-pyridin-2-yléthyl)-1-(tétrahydro-2*H*-pyran-4-ylméthyl)-1*H*-benzimidazol-5-yl] benzènesulfonamide ;
le *N*-[2-(1-cyano-1-méthyléthyl)-1-(tétrahydro-2*H*-pyran-4-ylméthyl)-1*H*-benzimidazol-5-yl]-*N*-méthylbenzènesulfonamide ;
le *N*-méthyl-*N*-[2-propyl-1-(tétrahydro-2*H*-pyran-4-ylméthyl)-1*H*-benzimidazol-5-yl]benzènesulfonamide ;
le *N*-[2-butyl-1-(tétrahydro-2*H*-pyran-4-ylméthyl)-1H-benzimidazol-5-yl]-*N*-méthylbenzènesulfonamide ;
le *N*-[2-*tert*-butyl-1-(tétrahydro-2*H*-pyran-4-ylméthyl)-1*H*-benzimidazol-5-yl]-*N*-éthylbenzènesulfonamide ;
le *N*-éthyl-*N*-[2-(1-méthoxy-1-méthyléthyl)-1-(tétrahydro-2*H*-pyran-4-ylméthyl)-1*H*-benzimidazol- 5- yl] benzènesulfonamide ;

et les sels pharmaceutiquement acceptables de ceux-ci.

**6.** Composé selon l'une quelconque des revendications 1 à 5, destiné à être utilisé comme médicament.

**7.** Utilisation d'un composé selon l'une quelconque des revendications 1 à 5, dans la fabrication d'un médicament destiné à la thérapie des douleurs.

**8.** Utilisation d'un composé selon l'une quelconque des revendications 1 à 5, dans la fabrication d'un médicament destiné au traitement des troubles de l'anxiété.

**9.** Utilisation d'un composé selon l'une quelconque des revendications 1 à 5, dans la fabrication d'un médicament destiné au traitement du cancer, de la sclérose en plaques, de la maladie de Parkinson, du cancer, de la chorée de Huntington, de la maladie d'Alzheimer, des troubles gastro-intestinaux et des troubles cardiovasculaires.

**10.** Composition pharmaceutique comprenant un composé selon l'une quelconque des revendications 1 à 5, et un support pharmaceutiquement acceptable.

**11.** Procédé de préparation d'un composé de formule I,

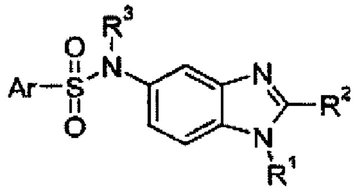

I

comprenant :

la réaction d'un composé de formule II,

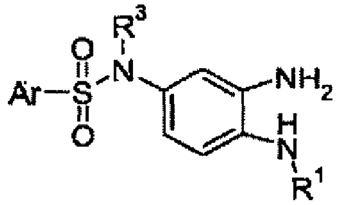

II

avec un composé de formule $R^2COX$, en présence d'une base, telle qu'une alkylamine, et éventuellement d'un réactif de couplage tel que HATU, EDC ;

dans lesquelles
X est choisi parmi Cl, Br, F et OH ;
$R^1$ est choisi parmi $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle, et $C_{3-6}$hétérocycloalkyle, où lesdits $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle, et $C_{3-6}$hétérocycloalkyle utilisés pour définir $R^1$ sont éventuellement substitués par un ou plusieurs groupements choisis parmi halogène, cyano, nitro, méthoxy, éthoxy, méthyle, éthyle, hydroxy et amino ;
$R^2$ est choisi parmi $C_{1-10}$alkyle, $C_{2-10}$alcényle, $C_{3-10}$cycloalkyle, $C_{3-10}$cycloalkyl-$C_{1-4}$alkyle, et $C_{4-8}$gcycloalcényl-$C_{1-4}$alkyle, où lesdits $C_{1-10}$alkyle, $C_{2-10}$alcényle, $C_{3-10}$cycloalkyle, $C_{3-10}$cycloalkyl-$C_{1-4}$alkyle, et $C_{4-8}$cycloalcényl-$C_{1-4}$alkyle utilisés pour définir $R^2$ sont éventuellement substitués par un ou plusieurs groupements choisis parmi halogène, méthoxy, éthoxy, méthyle, éthyle, hydroxy, et amino ;
$R^3$ est choisi parmi -H, $C_{1-6}$alkyle, $C_{2-6}$alcényle, $C_{3-6}$cycloalkyle, et $C_{3-6}$cycloalkyl-$C_{1-4}$alkyle ; et
Ar est choisi parmi $C_{6-10}$aryle et $C_{3-6}$hétéroaryle, où lesdits $C_{6-10}$aryle et $C_{3-6}$hétéroaryle sont éventuellement substitués par un ou plusieurs groupements choisis parmi $C_{1-3}$alkyle, $C_{1-6}$alcoxy, $C_{1-6}$alkylaminocarbonyle et halogène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Nomenclature of Organic Chemistry, Sections A, B, C, D, E, F, and H. Pergamon Press, 1979